(19) Europäisches Patentamt
European Patent Office
Office européen des brevets

(11) Publication number: 0 396 065
A1

(12) # EUROPEAN PATENT APPLICATION

(21) Application number: 90108163.8

(22) Date of filing: 28.04.90

(51) Int. Cl.$^5$: C07K 5/06, C07K 5/02, C07K 5/08, C07D 233/64, A61K 37/64, A61K 31/415

(30) Priority: 02.05.89 JP 112245/89
27.10.89 JP 278490/89

(43) Date of publication of application:
07.11.90 Bulletin 90/45

(84) Designated Contracting States:
AT BE CH DE DK ES FR GB IT LI NL SE

(71) Applicant: Japan Tobacco Inc.
2-1 Toranomon, 2-Chome
Minato-Ku Tokyo 105(JP)

Applicant: YOSHITOMI PHARMACEUTICAL INDUSTRIES, LTD.
6-9, Hiranomachi 2-chome Chuo-ku
Osaka-shi Osaka 541(JP)

(72) Inventor: Uchida, Itsuo, c/o Pharmaceutical Research
Laboratories, Japan Tobacco Inc., 6-2, Umegaoka
Midori-ku, Yokohama-shi, Kanagawa 227(JP)
Inventor: Shibata, Saizo, c/o Pharmaceutical Research
Laboratories, Japan Tobacco Inc., 6-2, Umegaoka
Midori-ku, Yokohama-shi, Kanagawa 227(JP)
Inventor: Yamada, Yasuki, c/o Pharmaceutical Research
Laboratories, Japan Tobacco Inc., 6-2, Umegaoka
Midori-ku, Yokohama-shi, Kanagawa 227(JP)
Inventor: Ikemoto, Yukinari, c/o Pharmaceutical Research
Laboratories, Japan Tobacco Inc., 6-2, Umegaoka
Midori-ku, Yokohama-shi, Kanagawa 227(JP)
Inventor: Iwata, Kunio, c/o Texicology Research Lab.
Japan Tobacco Inc., 23 Nakogi
Hatano-shi, Kanagawa 257(JP)
Inventor: Ikegami, Kiyoteru
942-47, Ogawa-cho 1-chome
Kodaira-shi, Tokyo 187(JP)
Inventor: Nakamura, Ikuro
1-803, 1-4, Moto-cho
Kiyose-shi, Tokyo 204(JP)

(74) Representative: von Kreisler, Alek, Dipl.-Chem. et al
Patentanwälte Von Kreisler-Selting-Werner,
Deichmannhaus am Hauptbahnhof
D-5000 Köln 1(DE)

(54) **Novel amino acid derivatives possessing renin-inhibitory activities.**

(57) An amino acid derivative of the general formula:



Xerox Copy Centre

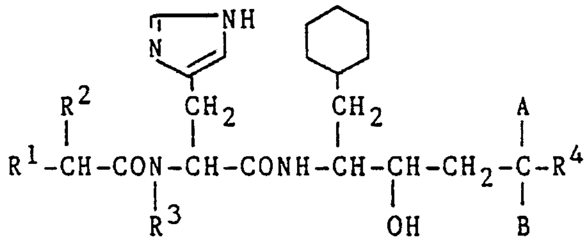

wherein $R^1$ is

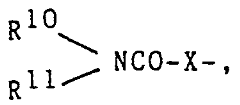 , $R^{12}$-NH-, $R^{13}$-CO-Y-, $R^{13}$-SO$_2$-CH$_2$-, 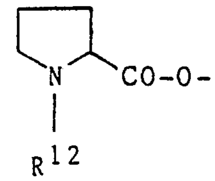 ,

HO- or 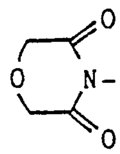

wherein, $R^{10}$ is a lower alkyl group and $R^{11}$ is

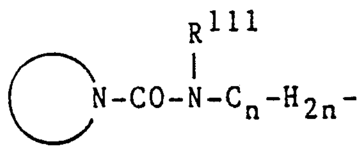

(wherein $R^{111}$ is a lower alkyl group and n is an integer of 1 to 5) or a lower alkyl group which may be substituted by hydroxy group or methoxyethoxymethoxy group, or $R^{10}$ and $R^{11}$ are

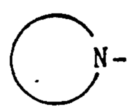

combinedly together with the adjacent nitrogen atom;
$R^{12}$ is a hydrogen atom, $C_nH_{2n+1}$-O-CO- (n is as defined above) or

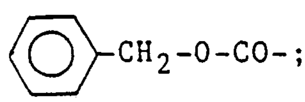

$R^{13}$ is a lower alkyl group which may be substituted by substituent(s) selected from HOOC-$(H_2C)_n$-O-, $R^{12}$-NH- (n and $R^{12}$ are as defined above) and pyridyl group;
X is -CH$_2$-, -O- or -NH- and Y is -O- or -NH-;
wherein

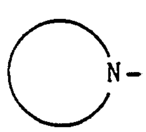 is 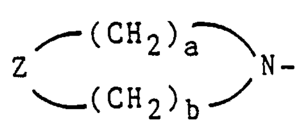

(wherein Z is -O-, -S-, -S(O)-, -S(O)$_2$-, -CH$_2$-, -CH(OH)-,

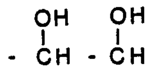 -, -NH- or

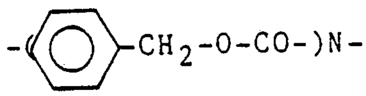

and a and b are independently an integer of 1 to 4 and the total of a and b is not more than 5) ;
$R^2$ is an aralkyl group which may be substituted by lower alkyl group(s);
$R^3$ is a hydrogen atom or a lower alkyl group;
$R^4$ is a lower alkyl group;
and A is hydroxy group and B is a hydrogen atom, or A and B are carbonyl group combinedly together with the adjacent carbon atom, a pharmaceutically acceptable acid addition salt or an ester thereof is described. The compounds of the invention possess inhibitory activities against renin and are useful as an antihypertensive agent.

# Novel Amino Acid Derivatives Possessing Renin-Inhibitory Activities

## BACKGROUND OF THE INVENTION

This invention relates to new amino acid derivatives, pharmaceutically acceptable salts or esters thereof which have inhibitory activities against renin, to processes for the preparation thereof, and to a pharmaceutical composition comprising the same.

Renin is a proteolytic enzyme synthesized and stored principally in a specific part of the kidney called the juxtaglomerular apparatus. Any of three different physiologic circumstances may cause the release of renin into the circulation: (a) a decrease in the blood pressure entering or within the kidney itself: (b) a decrease in the blood volume in the body: or (c) a fall in the concentration of sodium in the distal tubules of the kidney.

When renin is released into the blood from the kidney, the renin-angiotensin system is activated, leading to vasoconstriction and conservation of sodium, both of which result in increased blood pressure. The renin acts on a circulating protein, angiotensinogen to cleave out a fragment called angiotensin I (AI). AI itself has only slight pharmacologic activity but after additional cleavage by a second enzyme angiotensin converting enzyme (ACE) forms the potent molecule angiotensin II (AII). The major pharmacological effects of AII are vasoconstriction and stimulation of the adrenal cortex to release aldosterone, a hormone which causes sodium retention. Sodium retention causes blood volume to increase, which leads to hypertension. AII is cleaved by an aminopeptidase to form angiotensin III (AIII) which compared to AII is a less potent vasoconstrictor but a more potent inducer of aldosterone release.

Inhibitors of renin have been sought as agents for control of hypertension and as diagnostic agents for identification of cases of hypertension due to renin excess.

Some renin inhibitors possessing similar structures to those of our object amino acid derivatives have been known as described in EP300189, EP341602, EP310015, USP4725584, USP4725583, USP4845079, USP4657931 and USP4841067.

## SUMMARY OF THE INVENTION

One object of this invention is to provide new and useful amino acid derivatives, pharmaceutically acceptable salts or esters thereof which possess inhibitory activities against renin, and which are useful as a hypotensor and a therapeutic agent on heart failure, especially for oral administration.

Another object of this invention is to provide a pharmaceutical composition comprising as an active ingredient said amino acid derivatives and their esters, and pharmaceutically acceptable salts thereof.

The object amino acid derivatives of this invention are new and can be represented by the following general formula [I]:

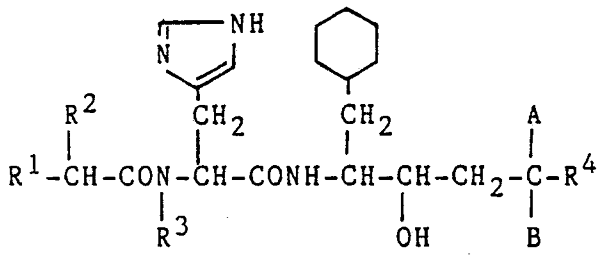

[I]

wherein $R^1$ is

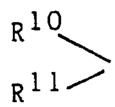 NCO-X-, $R^{12}$-NH-, $R^{13}$-CO-Y-, $R^{13}$-$SO_2$-$CH_2$-, 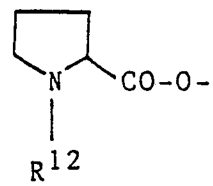 ,

HO- or 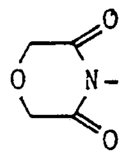

wherein, $R^{10}$ is a lower alkyl group and $R^{11}$ is

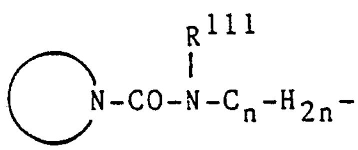

(wherein $R^{111}$ is a lower alkyl group and n is an integer of 1 to 5) or a lower alkyl group which may be substituted by hydroxy group or methoxyethoxymethoxy group, or $R^{10}$ and $R^{11}$ are

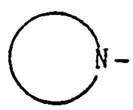

combinedly together with the adjacent nitrogen atom;

$R^{12}$ is a hydrogen atom, $C_nH_{2n+1}$-O-CO- (n is as defined above) or

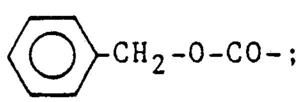

$R^{13}$ is a lower alkyl group which may be substituted by substituent(s) selected from HOOC-$(H_2C)_n$-O-, $R^{12}$-NH- (n and $R^{12}$ are as defined above) and pyridyl group;

X is -$CH_2$-, -O- or -NH- and Y is -O- or -NH-;

wherein

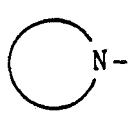 is 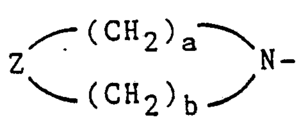

(wherein Z is -O-, -S-, -S(O)-, $-S(O)_2-$, $-CH_2-$, -CH(OH)-,

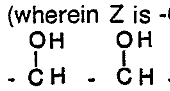

-, -NH- or

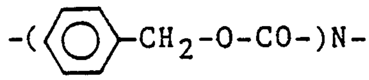

and a and b are independently an integer of 1 to 4 and the total of a and b is not more than 5) ;
$R^2$ is an aralkyl group which may be substituted by lower alkyl group(s);
$R^3$ is a hydrogen atom or a lower alkyl group;
$R^4$ is a lower alkyl group;
and A is hydroxy group and B is a hydrogen atom, or A and B are carbonyl group combinedly together with the adjacent carbon atom.

## DETAILED DESCRIPTION OF THE INVENTION

The object compound [I] or its salt can be prepared by processes as illustrated in the following reaction schemes, but preparations of the object compound [I] are not limited to the following processes.

### Process 1

#### Step 1

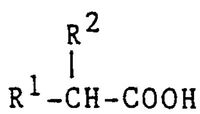

+

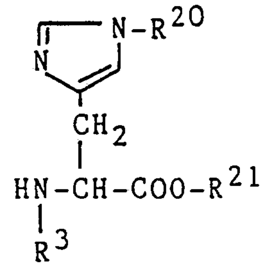

[II]

or its reactive derivative at the carboxy group or a salt thereof

[III]

or its reactive derivative at the amino group or a salt thereof

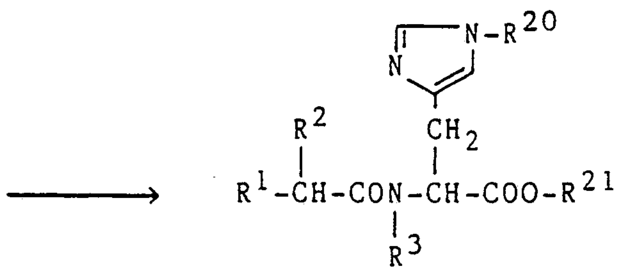

[IV]

or its salt

Step 2

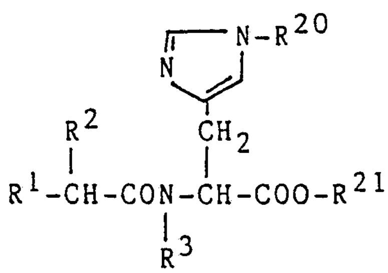

[IV]

or its salt

Elimination of the carboxy-protective group $R^{21}$, and if necessary, derivation to its reactive derivative at the carboxy group or a salt thereof

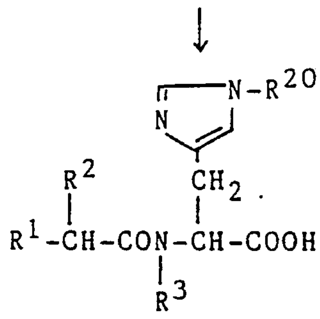

[V]

or its reactive derivative at the carboxy group or a salt thereof

Step 3

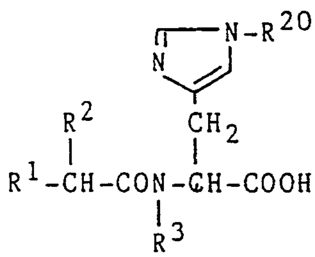

+

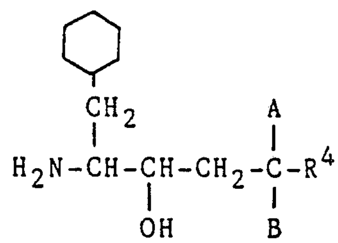

[V]

or its reactive derivative at the carboxy group or a salt thereof

[VI]

or its reactive derivative at the amino group or a salt thereof

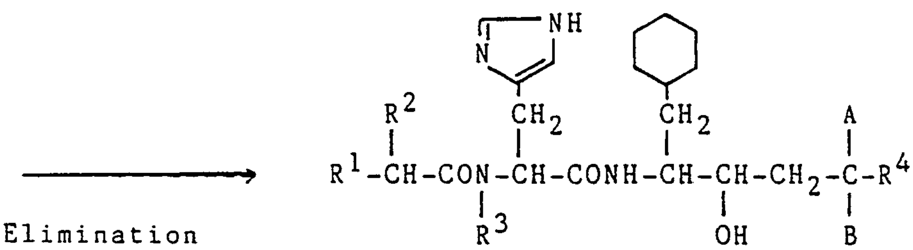


[I]

or its salt

Process 2

Step 1

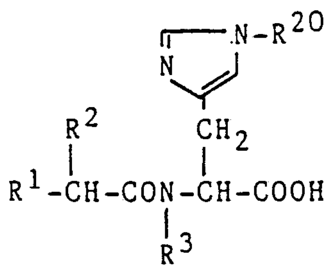

+

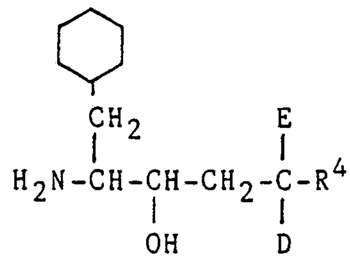

[V]

[VII]

or its reactive derivative at the carboxy group or a salt thereof

or its reactive derivative at the amino group . or a salt thereof

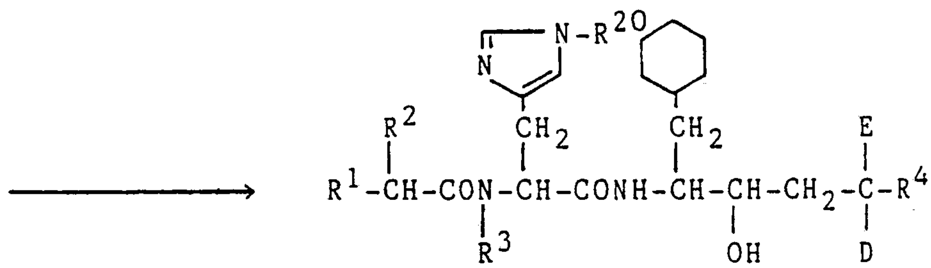

[VIII]

or its salt

Step 2

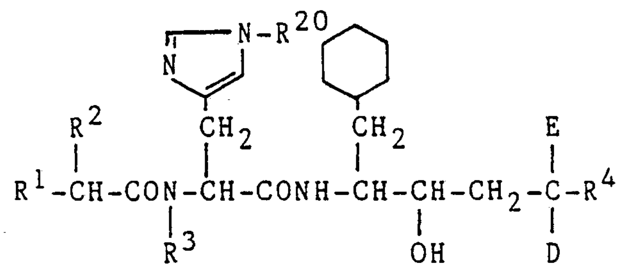

[VIII]

or its salt

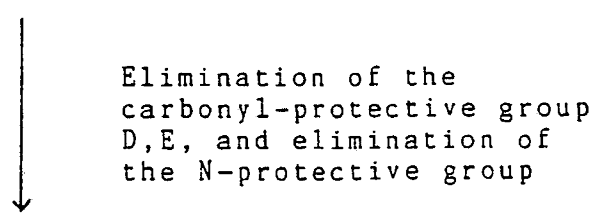

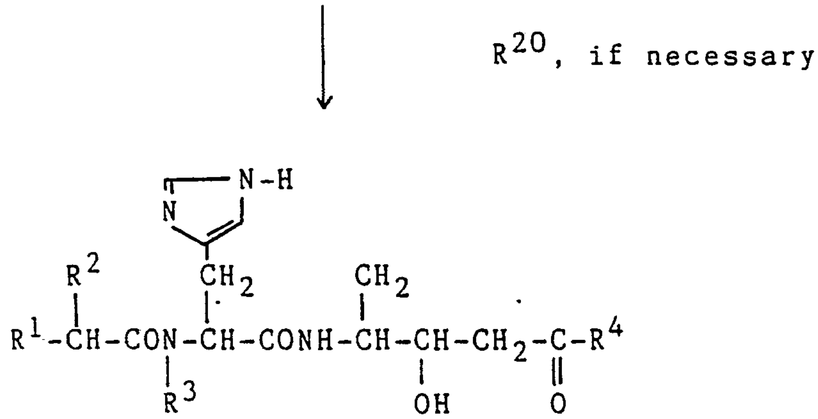


[Ia]

or its salt

Process 3

Step 1

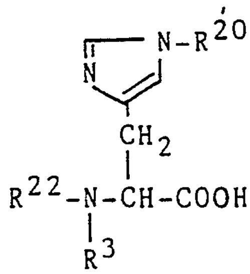

+

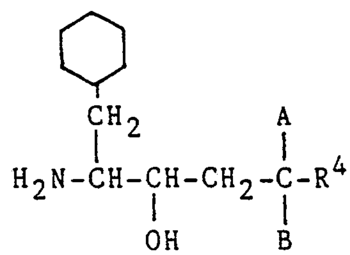

[IX]

or its reactive derivative at the carboxy group or a salt thereof

[VI]

or its reactive derivative at the amino group or a salt thereof

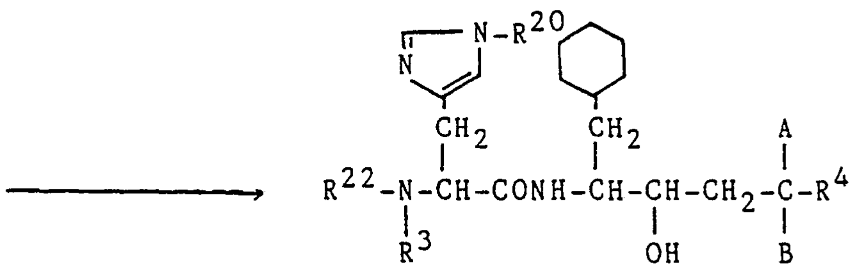

[X]

or its salt

Step 2

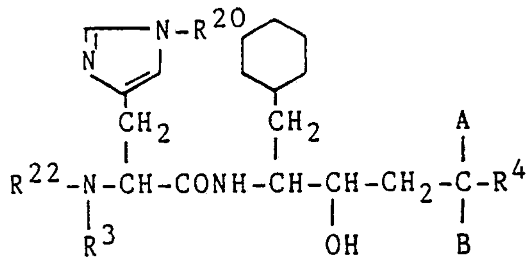

[X]

or its salt

Elimination of the N-protective group $R^{22}$

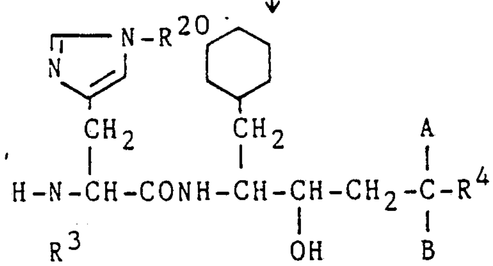

[XI]

or its salt

<u>Step 3</u>

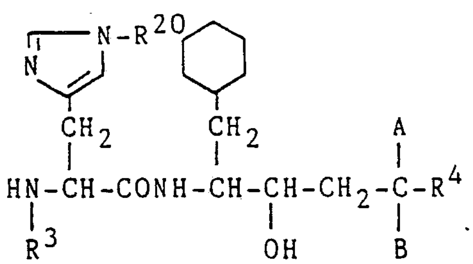

+

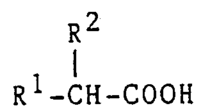

[XI]

or its reactive derivative at the amino group or a salt thereof

[II]

or its reactive derivative at the carboxy group or a salt thereof

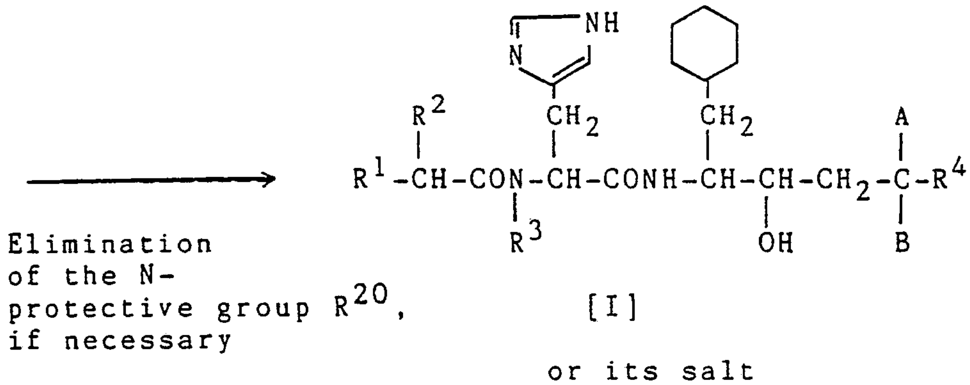


[I]

or its salt

Process 4

Step 1

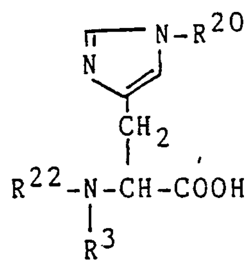

+

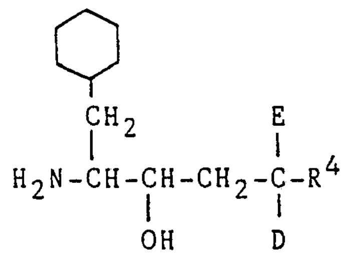

[IX]

or its reactive derivative at the carboxy group or a salt thereof

[VII]

or its reactive derivative at the amino group or a salt thereof

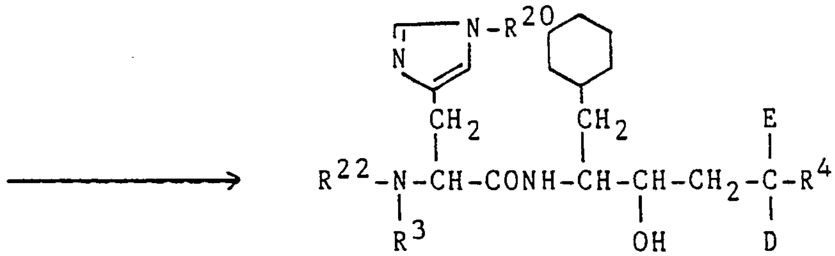

[XII]

or its salt

Step 2

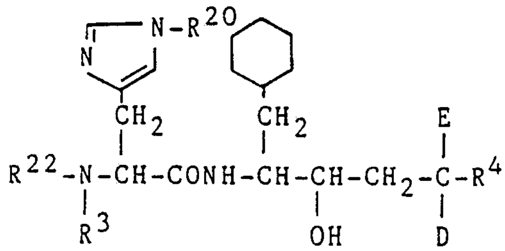

[XII]

or its salt

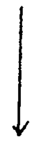

Elimination of the N-protective group $R^{22}$

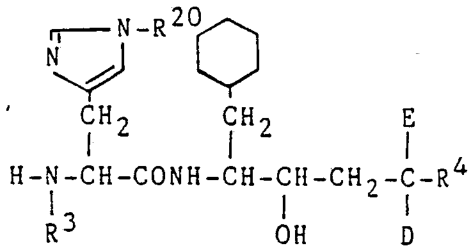

[XIII]

or its salt

Step 3

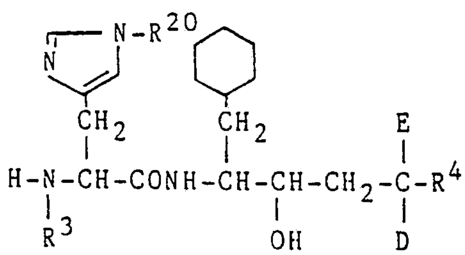

+

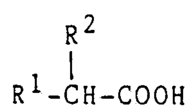

[XIII]

or its reactive derivative at the amino group or a salt thereof

[II]

or its reactive derivative at the carboxy group or a salt thereof

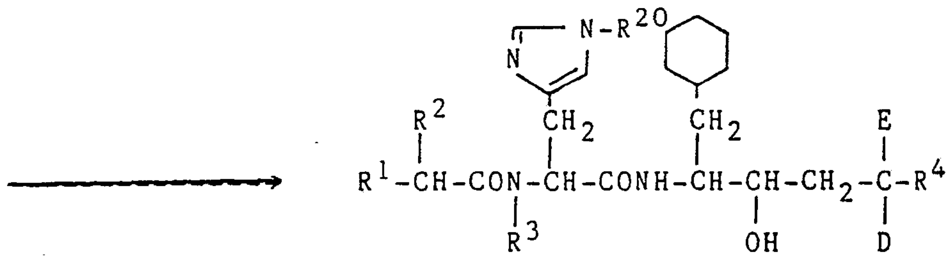

[VIII]

or its salt

Step 4

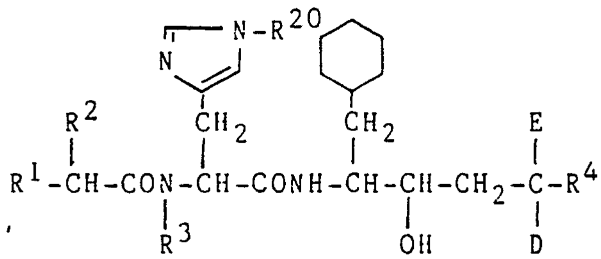

[VIII]

or its salt

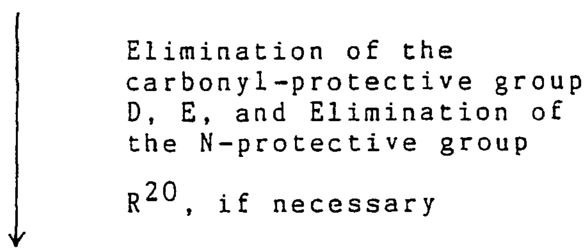


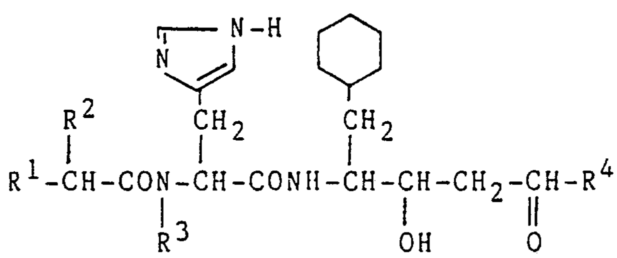

[Ia]

or its salt

Step 5

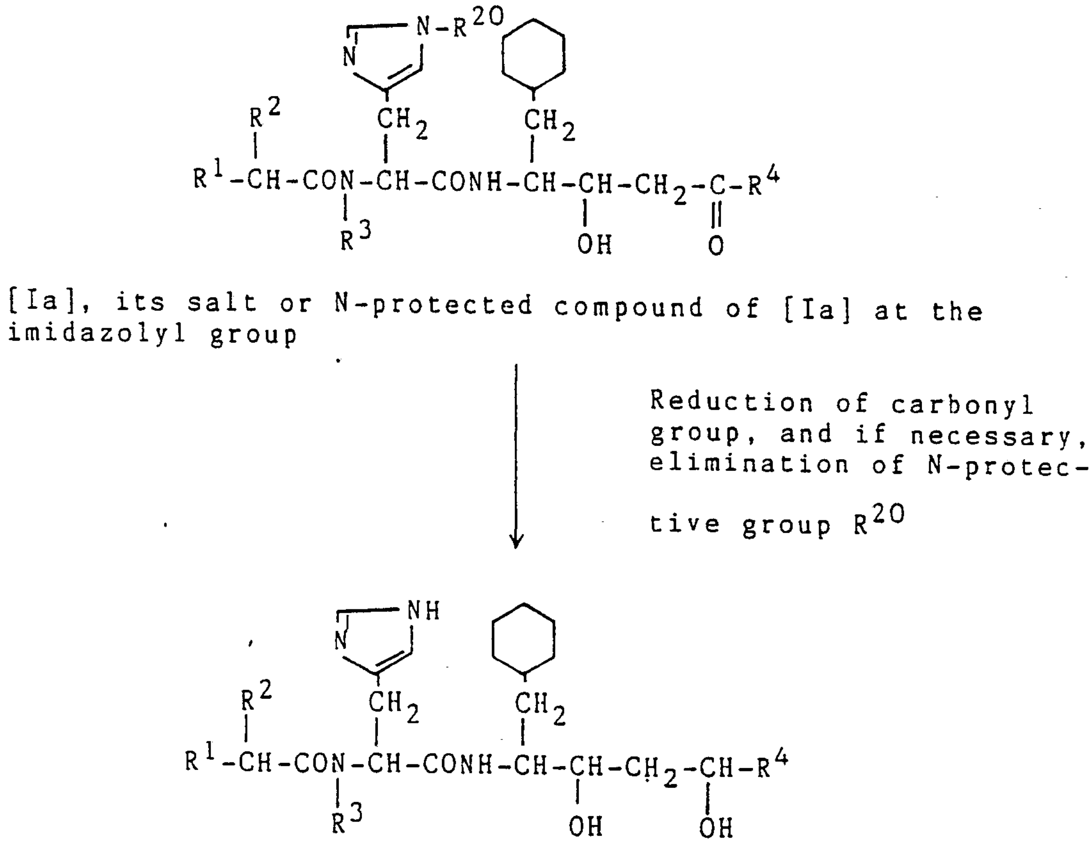


[Ia], its salt or N-protected compound of [Ia] at the imidazolyl group

[Ib]

or its salt

[in which $R^1$,$R^2$,$R^3$,$R^4$, A and B are each as defined above; $R^{20}$ is hydrogen or N-protective group; $R^{21}$ is carboxy-protective group; $R^{22}$ is hydrogen or N-protective group such as t-butoxycarbonyl and the like, and includes $R^{12}$; E and D are taken together with the attached carbon atom to form a carbonyl-protective group. Compounds [Ia], [Ib] are enbodiment of this invention, and included in the compound [I].]

In the above and subsequent description of the present specification, suitable examples of the various definitions to be included within the scope of the invention are explained in detail in the following.

The term "lower" is intended to mean a group having 1 to 7 carbon atom(s), unless otherwise provided. "Lower alkyl" may be a straight or branched one, such as methyl, ethyl, n-propyl, isopropyl, n-butyl, isobutyl, sec-butyl, tert-butyl, 2-methylhexyl, n-pentyl, 1-methylbutyl, 2,2-dimethylbutyl, 2-methylpentyl, 2,2-dimethylpropyl, n-hexyl, methylhexyl and the like. Suitable "lower alkyl" may be $C_{1-5}$ alkyl, in which more preferable one may be $C_{1-4}$ alkyl such as methyl, ethyl, propyl, isopropyl or the like.

"Aralkyl" is lower alkyl substituted with aryl group such as phenyl, 1-naphthyl, 2-naphthyl and the like, in which more preferable ones are phenyl-lower-alkyl and naphthyl-lower-alkyl.

"Lower alkoxy" may be a straight or branched $C_{1-7}$ one such as methoxy, ethoxy, propoxy, isopropoxy, butoxy, isobutoxy, tert-butoxy, pentyloxy, hexyloxy, and the like, in which more preferable one may be $C_{1-4}$ alkoxy.

"N-protective group" may be substituted or unsubstituted lower alkanoyl (e.g. formyl, acetyl, propionyl, trifluoroacetyl, etc.), phthaloyl, lower alkoxycarbonyl (e.g. tert-butoxycarbonyl (Boc), tert-amyloxycarbonyl, etc.), substituted or unsubstituted aralkyloxycarbonyl (e.g. benzyloxycarbonyl (Z), p-nitrobenzyloxycarbonyl, etc.), substituted or unsubstituted arylsulfonyl (e.g. benzenesulfonyl, tosyl, etc.), aralkyl (e.g. trityl, benzyl, etc.) or the like.

"Carboxyl-protective group" is a group forming an ester with carboxy group, which is exemplified by methyl group, ethyl group, tert-butyl group, benzyl group, phenacyl group, trichloroethyl group, p-nitrobenzyl group and diphenylmethyl group, and any one used conventionally in this field can be employed, and thus the carboxyl-protective group is not particularly limited to them.

"Carbonyl-protective group" is a protective group used for protecting carbonyl group against undesirable reactions in the synthetic procedures, and carbonyl group can be protected by forming, for example, a chain ketal such as dimethylketal, diethylketal and dibenzylketal; a cyclic ketal such as 1,3-dioxane and 1,3-dioxolane; a chain dithioketal such as S,S′-dimethylketal, S,S′-diethylketal and S,S′-diphenylketal; and a cyclic dithioketal such as 1,3-dithiane and 1,3-dithiolane.

"Carbonyl-protective group-eliminating reaction" means a reaction for removing the protective group from a protected carbonyl group, resulting in producing carbonyl group. This reaction includes acid hydrolysis, reduction reactions, oxidation reactions, reactions using inorganic mercuric salt or inorganic silver salt, but the reaction is not limited to them.

"N-protective group-eliminating reaction" means a reaction for removing a protective group from the protected amino or imino group, and thereby producing amino or imino group.

Suitable pharmaceutically acceptable salts of the object compounds [I] are conventional non-toxic salts and include an organic acid addition salt (e.g. formate, acetate, trifluoroacetate, maleate, tartrate, methanesulfonate, benzenesulfonate, toluenesulfonate, etc.), an inorganic acid addition salt (e.g. hydrochloride, hydrobromide, sulfate, phosphate, etc.), a salt with an amino acid (e.g. aspartic acid salt, glutamic acid salt, etc.), or the like.

The compounds of the present invention can also be used in the form of esters. Examples of such esters include a hydroxyl-substituted compound of formula I which has been acylated with a blocked or unblocked amino acid residue, a phosphate function, or a hemisuccinate residue. The amino acid esters of particular interest are alanine and lysine: however, other amino acid residues can also be used. These esters serve as pro-drugs of the compounds of the present invention and serve to increase the solubility of these substances in the gastrointestinal tract. These pro-drugs are metabolized in vivo to provide the hydroxyl-substituted compound of formula I. Typical examples of preparation methods of the pro-drug esters are as follows and the methods are not limited to the examples. In one method, a pro-drug ester is prepared by reacting a hydroxyl-substituted compound of formula I with an activated amino acyl, phosphoryl or hemisuccinyl derivative, and the resulting product is then deprotected to provide the desired pro-dug ester. In another method, a pro-drug ester is prepared by reacting a hydroxyl-substituted compound (for example, compound [II] or [VI] in process 1), which is a building block of a compound of formula I, with an active amino acyl, phosphoryl or hemisuccinyl derivative. Then an ester derivative of compound of formula I is prepared using the resulting ester of the building block. The obtained ester derivative of compound of formula I is then deprotected to provide the desired pro-drug ester.

The processes for preparing the object compounds [I] are explained in detail in the following.

Process 1

Step 1

The compound [IV] or its salt can be prepared by reacting a compound [II] or its reactive derivative at the carboxy group or a salt thereof with a compound [III] or its reactive derivative at the amino group or a salt thereof.

This reaction is what is called a peptide synthesis reaction which can be conducted by a per se known method.

$R^{20}$ in the compound [III] means hydrogen or an N-protective. group as mentioned above. $R^{21}$ means a carboxy-protective group as mentioned above. The reactive derivative means a derivative obtained by activating a group concerned with the reaction such as carboxy group or amino group by an optional method.

Suitable salts of the compound [IV] can be referred to the ones as exemplified for the compound [I].

Suitable reactive derivative at the carboxy group of the compound [II] may include an acid halide, an acid anhydride, an activated amide, an activated ester, and the like. Suitable examples of the reactive derivatives may be an acid chloride: an acid azide: a mixed acid anhydride with an acid such as substituted phosphoric acid (e.g. dialkylphosphoric acid, phenylphosphoric acid, diphenylphosphoric acid, dibenzylphosphoric acid, halogenated phosphoric acid, etc.), dialkylphosphorous acid, sulfurous acid, thiosulfuric acid sulfuric acid, sulfonic acid (e.g. methanesulfonic acid, etc.), aliphatic carboxylic acid (e.g. acetic acid, propionic acid, pivalic acid, pentanoic acid, isopentanoic acid, 2-ethylbutylic acid, trichloroacetic acid, etc.) or aromatic carboxylic acid (e.g. benzoic acid, etc): a symmetrical acid anhydride: an activated amide with imidazole, 4-substituted imidazole, dimethylpyrazole, triazole or tetrazole: or an activated ester (e.g. cyanomethyl ester, methoxymethyl ester, dimethyliminomethyl $[(CH_3)_2N^+=CH-]$ ester, vinyl ester, propargyl ester, p-nitrophenyl ester, 2,4-dinitrophenyl ester, trichlorophenyl ester, pentachlorophenyl ester, methylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester, etc.), or an ester with a N-hydroxy compound (e.g. N,N-dimethylhydroxylamine, 1-hydroxy-2-(1H)-pyridone, N-hydroxysuccinimide, N-hydroxyphthalimide, 1-hydroxy-1H-benzotriazole, etc.), and the like. These reactive derivatives can optionally be selected from them according to the kind of the compound [II] to be used.

Suitable salts of the compound [II] and its reactive derivative may be a base salt such as an alkali metal salt (e.g. sodium salt, potassium salt, etc.), an alkaline earth metal salt (e.g. calcium salt, magnesium salt, etc.), an ammonium salt, an organic base salt (e.g. trimethylamine salt, triethylamine salt, pyridine salt, picoline salt, dicyclohexylamine salt, N,N′-dibenzylethylenediamine salt, etc.), or the like.

Suitable reactive derivative at the amino group of the compound [III] may include Schiff's base type imino or its tautomeric enamine type isomer formed by the reaction of the compound [III] with a carbonyl compound such as aldehyde, ketone or the like: a silyl derivative formed by the reaction of the compound [III] with a silyl compound such as bis(trimethylsilyl)acetamide, mono(trimethylsilyl)acetamide, bis-(trimethylsilyl)urea or the like: a derivative formed by reaction of the compound [III] with phosphorus trichloride or phosgene, and the like.

Suitable salts of the compound [III] and its reactive derivative can be referred to the ones as exemplified for the compound [I].

The reaction is usually carried out in a conventional solvent such as water, alcohol (e.g. methanol, ethanol, etc.), acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvent may also be used in a mixture with water.

In this reaction, when the compound [II] is used in a free acid form or its salt form, the reaction is preferably carried out in the presence of a conventional condensing agent such as N,N′-dicyclohexylcarbodiimide: N-cyclohexyl-N′-morpholinoethylcarbodiimide: N-cyclohexyl-N′-(4-diethylaminocyclohexyl)carbodiimide: N,N′-diethylcarbodiimide, N,N′-diisopropylcarbodiimide: N-ethyl-N′-(3-dimethylaminopropyl)carbodiimide: N,N′-carbonylbis-(2-methylimidazole): pentamethyleneketene-N-cyclohexylimine: diphenylketene-N-cyclohexylimine: ethoxyacetylene: 1-alkoxy-1-chloroethylene: trialkyl phosphite, ethylpolyphosphate: isopropyl polyphosphate: phosphorus oxychloride (phosphoryl chloride): phosphorus trichloride: diphenylphosphoryl azide: diethyl cianophosphate, thionyl chloride: oxalyl chloride: lower alkyl haloformate (e.g. ethyl chloroformate, isopropyl chloroformate, etc.): triphenylphosphine: 2-ethyl-7-hydroxybenzisoxazolium salt: 2-ethyl-5-(m-sulfophenyl)isoxazolium hydroxide intramolecular salt: 1-(p-chlorobenzenesulfonyloxy)-6-chloro-1H-benzotriazole: so- called Vilsmeier reagent prepared by the reaction of N,N-dimethylformamide with thionyl chloride, phosgene, trichloromethyl chloroformate, phosphorus oxychloride, etc: or the like.

The reaction may also be carried out in the presence of an inorganic or organic base such as an alkali metal bicarbonate, tri(lower)alkylamine, pyridine, N-(lower)alkylmorpholine, N,N-di(lower)alkylbenzylamine, or the like.

The reaction temperature is not critical, and the reaction is usually carried out under cooling to warming.

Step 2

The compound [V] or its salt can be prepared by subjecting a compound [IV] or its salt to elimination reaction of the carboxy-protective group $R^{21}$.

Suitable salt of the compound [V] can be referred to the base addition salt as exemplified for the compound [II] and to the acid addition salt as exemplified for the compound [I].

This reaction is carried out in accordance with a conventional method such as hydrolysis, reduction or the like.

The hydrolysis is preferably carried out in the presence of a base or an acid.

Suitable base may include an inorganic base such as an alkali metal (e.g. sodium, potassium, etc.), the hydroxide or carbonate thereof.

Suitable acid may include an inorganic acid (e.g. hydrochloric acid, hydrobromic acid, sulfuric acid, hydrogen chloride, hydrogen bromide, hydrogen fluoride, etc.).

The hydrolysis reaction is usually carried out in a solvent such as water, an alcohol (e.g. methanol, ethanol, etc.). methylene chloride, chloroform, tetrachloromethane, tetrahydrofuran, a mixture thereof or any other solvent which does not adversely influence the reaction. A liquid base or acid can be also used as the

solvent. The hydrolysis reaction temperature is not critical and the reaction is usually carried out under cooling to heating.

The reduction method applicable for the elimination reaction may include chemical reduction and catalytic reduction.

Suitable reducing agents to be used in chemical reduction are a combination of metal (e.g. tin, zinc, iron, etc.) and an acid (e.g. formic acid, acetic acid, propionic acid, trifluoroacetic acid, etc.).

Suitable catalysts to be used in catalytic reduction are conventional ones such as platinum catalysts (e.g. platinum black, platinum oxide, etc.), palladium catalysts (e.g. palladium black, palladium oxide, palladium on carbon, etc.), nickel catalysts (e.g. reduced nickel, Raney nickel, etc.), iron catalysts (e.g. reduced iron, Raney iron, etc.), and the like.

The reduction is usually carried out in a conventional solvent which does not adversely influence the reaction such as water, methanol, ethanol, propanol, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, etc., or a mixture thereof.

The reaction temperature of this reduction is not critical and the reaction is usually carried out under cooling to heating.

The reactive derivative in the carboxy group of compound [V] or the salt thereof can be produced not only from compound [V] or its salt but also from compound [IV] or its salt. For example, when hydrazine, benzyloxycarbonylhydrazide and the like are used for carboxy-protective group-eliminating reaction, the acid azide derivative as a reactive derivative of the carboxy group of compound [V] can be produced via the acid hydrazide derivative of compound [V] instead of via compound [V] from compound [IV] or its salt.

As for preferred reactive derivatives in the carboxy group of compound [V], reference may be made to the reactive derivatives as mentioned as to compound [II].

For preferred salts of the reactive derivatives of compound [V], reference may be made to the salts as mentioned concerning the salts of compound [V].

Step 3

The compound [I] or its salts can be prepared by reacting a compound [V] or its reactive derivative at the carboxy group or a salt thereof with a compound [VI] or its reactive derivative at the amino group or a salt thereof, and if necessary, eliminating the N-protective group.

Suitable reactive derivatives at the amino group of the compound [VI] and its salts can be referred to ones as exemplified for the compound [III].

This reaction can be carried out in substantially the same manner as Step 1, and therefore the reaction mode and reaction conditions of this reaction are to be referred to those as explained in Step 1.

In case that the imidazolyl group of the compound [V] is protected, the object compound [I] can be prepared by further eliminating the N-protective group of the reaction product of the compound [V] with the compound [VI].

This elimination reaction is carried out in accordance with a conventional method such as hydrolysis, reduction or the like.

The hydrolysis is preferably carried out in the presence of a base or an acid including Lewis acid.

Suitable base may include an inorganic base and an organic base such as an alkali metal (e.g. sodium, potassium, etc.), an alkaline earth metal (e.g. magnesium, calcium, etc.), the hydroxide or carbonate or bicarbonate thereof, hydrazine, trialkylamine (e.g. trimethylamine, triethylamine, etc.), picoline, 1,5-diazabicyclo[4.3.0]non-5-ene, 1,4-diazabicyclo[2.2.2]octane, 1,8-diazabicyclo[5.4.0]undec-7-ene, or the like.

Suitable acid may include an organic acid (e.g. formic acid, acetic acid, propionic acid, trichloroacetic acid, trifluoroacetic acid, 1-hydroxybenzotriazole, etc.), an inorganic acid (e.g. hydrochloric acid, hydrobromic acid, sulfuric acid, hydrogen chloride, hydrogen bromide, hydrogen fluoride, etc.) and an acid addition salt compound (e.g. pyridine hydrochloride, etc).

The elimination using Lewis acid such as trihaloacetic acid (e.g. trichloroacetic acid, trifluoroacetic acid, etc.) or the like is preferably carried out in the presence of cation trapping agents (e.g. anisole, phenol, etc.).

Hydrolysis can be also conducted using an alcohol such as methanol after reaction of an acid anhydride such as acetic anhydride with the N-protected product in the presence of a base such as pyridine.

The reaction is usually carried out in a solvent such as water, an alcohol (e.g. methanol, ethanol, etc.), methylene chloride, chloroform, tetrachloromethane, tetrahydrofuran, a mixture thereof or any other solvent which does not adversely influence the reaction. A liquid base or acid can be also used as the solvent. The reaction temperature is not critical and the reaction is usually carried out under cooling to heating.

The reduction method applicable for the elimination reaction may include chemical reduction and catalytic reduction.

This reduction is substantially the same as the carboxy-protective group-eliminating reaction as mention above, and with regard to the reaction conditions such as reducing agents and catalysts to be used, reference may be made to the explanation of the chemical reduction and catalytic reduction in Step 2 of Process 1.

The elimination reaction of N-protective group for imidazolyl group in the aforementioned Process 1 can be carried out after Step 1 or Step 2.

Process 2

Step 1

The compound [VIII] or its salt can be prepared by reacting a compound [V] or its reactive derivative at the carboxy group or a salt thereof with a compound [VII], its reactive derivative at the amino group or a salt thereof.

Suitable salts of the compound [VIII] can be referred to ones as exemplified for the compound [I].

Suitable reactive derivative at the amino group of the compound [VII] can be referred to ones as exemplified for the compound [III].

This reaction can be carried out in substantially the same manner as Step 1 in Process 1, and therefore the reaction mode and reaction condition of this reaction are to be referred to those as explained in Step 1 in Process 1.

Step 2

The compound [Ia] or its salt can be prepared by subjecting a compound [VIII] or its salt to elimination reaction of the carbonyl protective group, and if necessary, eliminating the N-protective group.

This elimination reaction of the carbonyl group is carried out in accordance with a conventional method such as hydrolysis, reduction, oxidation on the reaction with inorganic salt.

The hydrolysis is preferably carried out in the presence of an acid.

Suitable acid may include an inorganic acid (e.g. hydrochloric acid, hydrobromic acid, hydrogen chloride, hydrogen bromide, hydrogen fluoride, etc.) and an organic acid (e.g. formic acid, acetic acid, propionic acid, p-toluenesulfonate, etc.).

As the preferred reduction method for carbonyl-protective group-eliminating reaction, mention can be made of catalytic reduction.

As to the detail of the preferred reaction conditions for catalysts, solvent and reaction temperatures to be adopted, reference may be made to the explanation concerning catalytic reduction in Step 2 of Process 1 as mentioned above.

As the preferred oxidation method for carbonyl- protecting group-removing reaction, there can be mentioned reactions using triethyloxonium tetrafluoroborate, triphenylcarbenium tetrafluoroborate and so on.

The oxidation reaction can be usually conducted in a solvent such as dichloromethane and chloroform at room temperature. However, the reaction is not limited to these conditions.

The aforementioned carbonyl-protective group-eliminating reaction can be conducted in the presence of inorganic salts, sulfuryl chloride, iodine and the like. As the preferable inorganic salts, there can be mentioned, for example, mercuric chloride (II), mercurous perchlorate (I), thallium nitrate (III), and combinations of silver nitrate (I) and silver oxide (I). As the solvents to be used for the reaction, mention may be made of, for example, water, acetonitrile, benzene, acetone, dichloromethane and methanol. The reaction can be conducted in a conventional solvent or a mixture of conventional solvents which does not interfere with the reaction. The reaction temperature of the reaction is not particularly limited and the reaction can be carried out from under cooling to under heating.

When the imidazolyl group of compound [VIII] is protected, the objective compound [Ia] can be

produced by eliminating the N-protective group $R^{20}$.

As for the N-protective group-eliminating reaction, reference is made to the explanation of the N-protective group-eliminating reaction in Step 3 of Process 1.

In Process 2, the N-protective group-eliminating reaction for the imidazolyl group can be conducted after Step 1.

Process 3

Step 1

Compound [X] or its salt can be produced by reacting compound [IX] or a reactive derivative in the carboxy group or a salt thereof with compound [VI] or a reactive derivative in the amino group or a salt thereof.

As for the preferred reactive derivatives in the carboxy group of compound [IX] and their salts, reference is made to the exemplification for compound [V] as mentioned above.

For the preferred salts of compound [X], reference is made to the above exemplification for compound [I].

This reaction is a peptide forming reaction, and can be carried out in substantially the same manner as the aforementioned Step 1 of Process 1. Thus, concerning the reaction method and conditions of the reaction, reference is made to the explanation of Step 1 of Process 1.

Step 2

Compound [XI] or its salt can be produced by subjecting compound [X] or its salt to elimination reaction of the N-protective group $R^{22}$.

As to the preferred salts of compound [XI], reference is made to the exemplification for compound [I]. This N- protective group-eliminating reaction is the same as the elimination reaction in Step 3 of Process 1, and thus, for the reaction, reference is made to the above explanation.

Step 3

The objective compound [I] or its salt can be produced by reacting compound [XI] or a reactive derivative in the amino group of compound [XI] or a salt thereof with compound [II] or a reactive derivative in the carboxy group or a salt thereof, if necessary, followed by removal of the N-protective group $R^{20}$ for imidazolyl group.

The reaction step can be carried out in substantially the same manner as in Step 3 of Process 1, and thus, for the reaction step, reference is made to the explanation of Step 3 of Process 1.

The removal of the N-protecting group $R^{20}$ for imidazolyl group can be conducted after Step 1 or Step 2.

Process 4

Step 1

Compound [XII] or its salt can be produced by reacting compound [IX] or a reactive derivative in the carboxyl group or a salt thereof with compound [VII] or a reactive derivative in the amino group or a salt thereof.

As to the preferred salts of compound [XII], reference is made to the exemplification for compound [I].

This reaction step can be conducted in substantially the same manner as Step 1 of Process 1, and thus, for the reac tion step, reference is made to the explanation of Step 1 of Process 1.

Step 2

Compound [XIII] or its salt can be produced by subjecting compound [XII] or its salt to the elimination reaction of the N-protective group $R^{22}$.

As to the preferred salts of compound [XIII], reference is made to the exemplification for compound [I].

For the elimination reaction, reference is made to the explanation for the elimination reaction of the N-protective group in Step 3 of Process 1.

Step 3

Compound [VIII] or its salt which is the same as the compound obtainable in Step 1 of Process 2 can be produced by reacting compound [XIII] or a reactive derivative in the amino group or a salt thereof with compound [II] or a reactive derivative in the carboxy group or a salt thereof.

Since this reaction step can be conducted in substantially the same manner as Step 1 of Process 1, for the step, reference may be made to the explanation of Step 1 of Process 1.

Step 4

The objective compound [Ia], which is the same compound as the one produced in Step 2 of Process 2 as mentioned above, can be synthesized in quite the same manner as in Step 2 of Process 2. Thus, reference can be made to the detail for Step 2 of Process 2 as mentioned above. In Process 4, elimination of the N-protective group $R^{20}$ for imidazolyl group can be carried out after Step 1, Step 2 or Step 3.

Process 5

The objective compound [Ib] wherein A is hydroxy group or its salt can be produced by subjecting compound [Ia] or a salt or an imidazolyl group N-protected compound thereof which is obtainable in Step 2 of Process 2 or Step 4 of Process 4 to reduction reaction for reduction of carbonyl group to hydroxy group, followed by, if necessary, removal of the N-protective group $R^{20}$.

As the reduction method applicable to the aforementioned reduction reaction of carbonyl group, there can be mentioned chemical reduction method and catalytic reduction method.

Examples of the preferred reducing agent for chemical reduction include metal hydrides such as sodium borohydride, lithium borohydride, zinc (II) borohydride and lithium aluminium hydride, metals such as lithium, sodium and zinc, aluminium alkoxide, triisobutyl aluminium, diborane and so on.

As for the preferred catalysts to be used for the catalytic reduction, reference can be made to the preferred catalysts usable for the catalytic reduction in Step 2 of Process 1.

The reduction is usually carried out in a conventional solvent such as water, methanol, ethanol, propanol, tetrahydrofuran, ether or N,N-dimethylformamide which does not interfere with the reaction, or a mixture thereof.

The reaction temperature of this reaction is not particularly limited, and the reaction can be usually conducted from under cooling to under heating.

For the N-protective group-eliminating reaction, reference can be made to the explanation of the N-protective group-eliminating reaction in Step 3 of Process 1.

Next, the intermediate compounds for the synthesis of the object compound [I] is mentioned below. The starting compounds [VI] and [VII] are novel compounds, and can be produced by the processes shown by the following reaction schemes (Processes A to E), although the production thereof is not limited to the following processes.

Process A

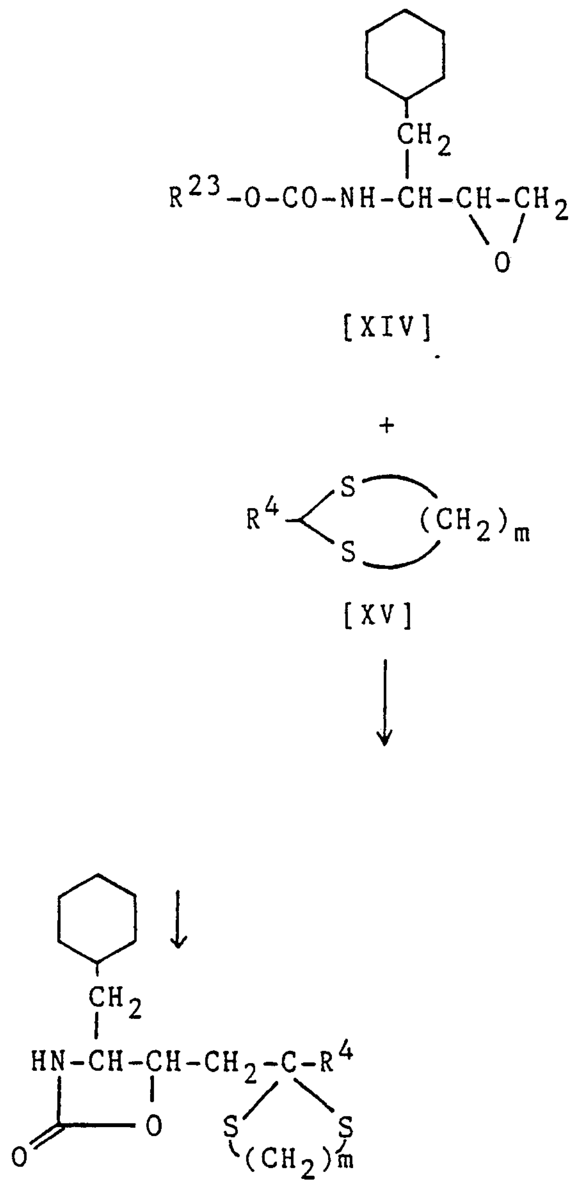

[XIV]

+

[XV]

[XVIa]

Process B

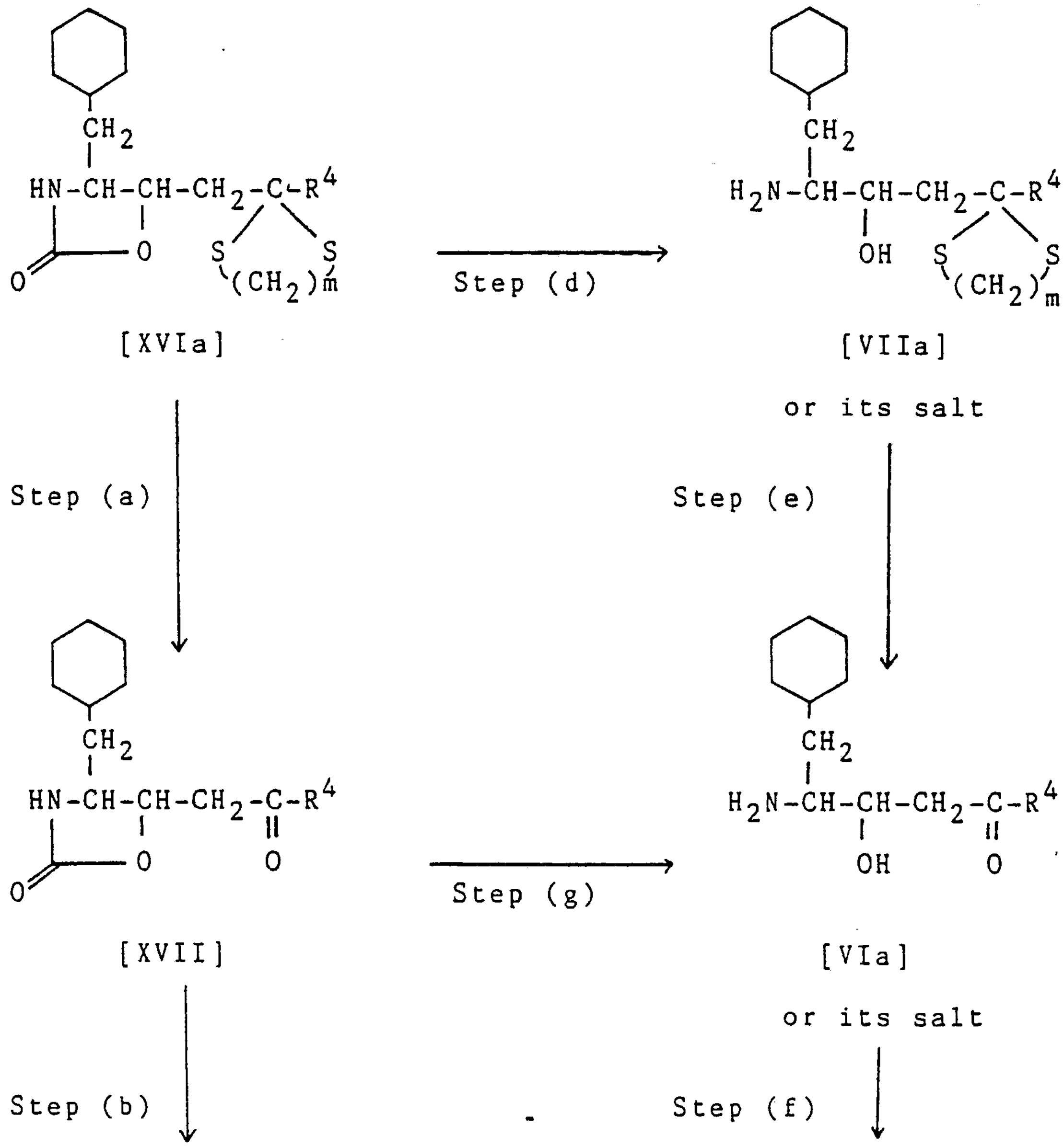


[XVIa]

[VIIa] or its salt

[XVII]

[VIa] or its salt

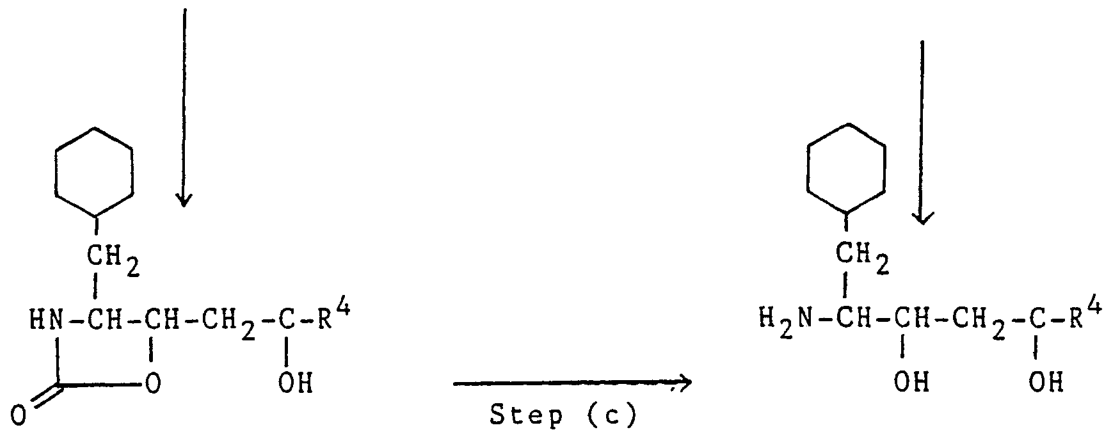


[XVIII]

[VIb]

or its salt

Process C

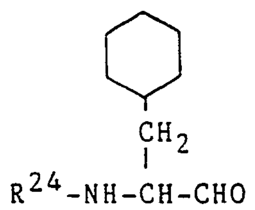

[XIX]

+

$CH_3-CO-R^4$

[XX]

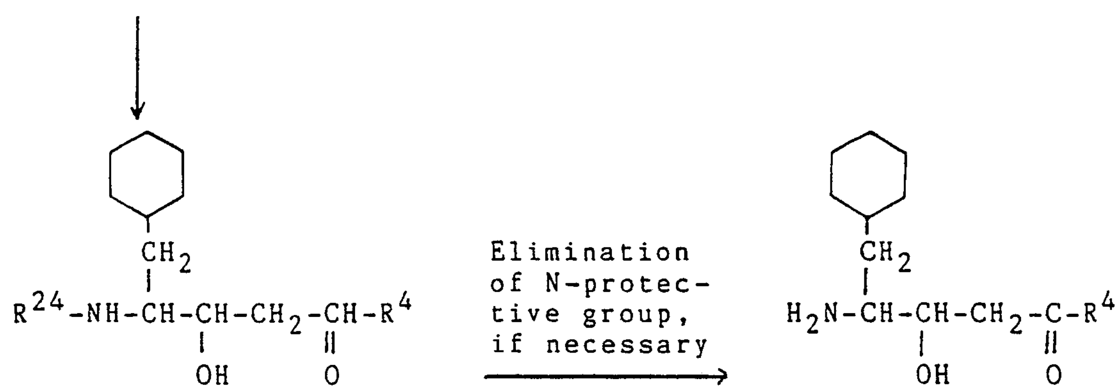


[XXIa]

[VIa]

or its salt

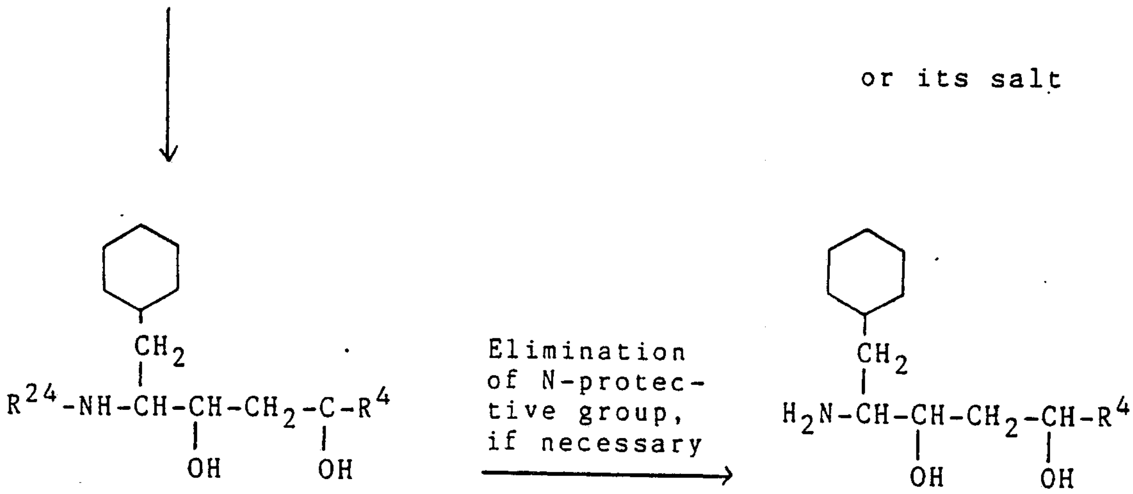


[XXIb] [VIb]

Process D

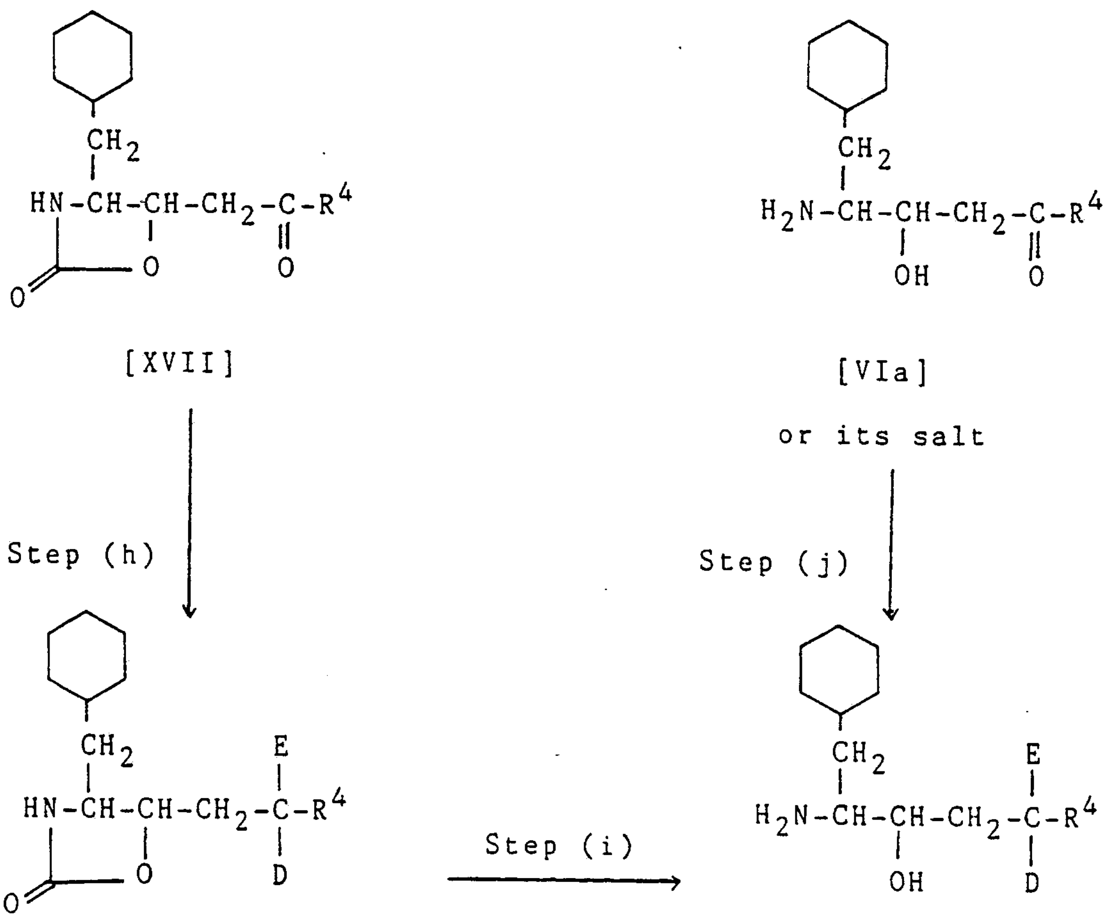


[XVII]

[VIa]

or its salt

[XVI]

[VII]

or its salt

wherein $R^4$, E and D are of the same meanings as defined above; $R^{23}$ is a lower alkyl group which may be substituted by an aryl group such as phenyl group and nitrophenyl group, (e.g. benzyl group, tert-butyl) or forms an amino-protective group together with the adjacent oxycarbonyl group; $R^{24}$ is hydrogen or an N-protective group; m is an integer of 2 to 4; compound [VIa] and compound [VIb] are included in compound [VI]; compound [VIIa] is included in compound [VII], and compound [XVIa] is included in compound [XVI].

The production methods of the starting compounds are in further detail described below.

Process A

Novel compound [XVIa] can be produced by coupling reaction of compound [XIV][The specific examples of the synthetic method are in detail described in Journal of Organic Chemistry, Vol. 52, pp 1487 - 1492 (1987)] and a lithium derivative of compound [XV][The specific examples of the synthetic method are in detail described in Journal of Organic Chemistry, Vol. 40, pp 231 - 237 (1975)].

The lithium derivative of compound [XV] can be produced by lithiation reaction of compound [XV] and a lithiating agent. As the preferred lithiating agents, there can be mentioned, for example, alkyllithiums such as n-butyllithium, sec-butyllithium and methyllithium.

The lithiation reaction can be carried out in a conventional solvent such as dried tetrahydrofuran, dried diethyl ether and dried toluene which does not interfere with the reation, or a mixture thereof. In some cases, by adding dried N,N,N′,N′-tetramethylenediamine [TMEDA], the reaction proceeds smoothly.

This lithiation reaction is preferably carried out under a dried inert gas atmosphere, usually from at room temperature to under cooling, preferably at -80 to 10 °C. The reaction temperature is not limited.

As for the solvent to be used for the coupling reaction of compound [XIV] and a lithium derivative of compound [XV], reference is made to the solvents usable for the aforementioned lithiation reaction.

The lithium derivative of compound [XV] can be usually used for the coupling without being isolated.

This coupling reaction is conducted preferably under a dried inert gas atmosphere, usually from at room temperature to under cooling, preferably at -60 to 10 °C. The reaction temperature is not particularly limited.

Process B

Step (a)

Compound [XVII] can be produced by subjecting compound [XVIa] to carbonyl-protective group-eliminating reaction.

The carbonyl-protective group-eliminating reaction can be carried out in the presence of an inorganic salt, sulfuryl chloride, iodine or the like. As the preferred inorganic salts, mention is made of mercuric [II] chloride, silver [I] perchlorate, thallium [III] nitrate, a combination of silver (I) nitrate and silver (I) oxide, or the like. Examples of the solvents to be used for this reaction include conventional solvents such as water, acetonitrile, benzene, acetone, dichloromethane and methanol, and mixtures thereof. The reaction temperature is not particularly limited and the reaction is usually conducted from under cooling to under heating.

Step (b)

Compound [XVIII] can be produced by subjecting compound [XVII] to reduction reaction for the reduction of the carbonyl group.

This reduction reaction can be conducted in substantially the same manner as the reduction reaction of Process 5, and thus, for the reaction conditions, reference may be made to the explanation of Process 5.

Step (c)

Compound [VIb] or its salt can be produced by hydrolysis of compound [XVIII].

The hydrolysis can be conducted preferably in the presence of a base or an acid.

As the preferred bases, mention can be made of barium hydroxide, sodium hydroxide, potassium hydroxide and so on.

Examples of the preferred acids include hydrochloric acid, sulfuric acid, hydrobromic acid, hydrogen chloride, hydrogen bromide and hydrogen fluoride.

The hydrolysis can be usually conducted in a solvent such as water, an alcohol (e.g. methanol, ethanol), dioxane or tetrahydrofuran, or a mixture thereof. Any other solvent can be used for the reaction unless it interferes with the reaction.

The hydrolysis is usually conducted under heating, but it is not limited.

Step (d)

Compound [VIIa] or its salt can be produced by hydrolysis of compound [XVIa].

This hydrolysis can be carried out in substantially the same manner as that in Step (c). However, it can be more preferably conducted in the presence of a base.

As for the other reaction conditions, reference can be made to the explanation of Step (c).

Step (e)

Compound [VIa] or its salt can be produced by subjecting compound [VIIa] or its salt to carbonyl-protective group-eliminating reaction.

This carbonyl-protective group-removing reaction can be conducted in substantially the same manner as the carbonyl-protecting group-removing reaction in Step (a), and thus, for the reaction conditions, reference can be made to the explanation of Step (a).

Step (f)

Compound [VIb] or its salt can be produced by subjecting compound [VIa] or its salt to reduction reaction for reduction of the carbonyl group.

This reduction reaction can be conducted in substantially the same manner as the reduction reaction of Process 5, and thus, as to the reaction conditions, reference can be made to the explanation of Process 5.

Step (g)

Compound [VIa] or its salt can be produced by hydrolysis of compound [XVII].

This hydrolysis can be carried out in substantially the same manner as the hydrolysis in Step (c), but it is preferably conducted in the presence of a base.

For the other reaction conditions, reference can be made to the explanation of Step (c).

Process C

Compound [XXIa] can be produced by subjecting compound [XIX][The specific examples of the synthesis are in detail described in Journal of Organic Chemistry, Vol. 52, pp 1487 -1492 (1987) and Japanese Unexamined Patent Publication (Kokai) No. 234071/1987] and compound [XX] to condensation reaction.

This condensation reaction is carried out in the presence of a base.

As the bases preferred for the condensation reaction, there can be mentioned, for example, lithium amides such as lithium isopropylamide and lithium dicyclohexylamide and metal hydrides such as sodium hydride and potassium hydride.

This condensation reaction is conducted preferably by producing a carboanion ($^{\ominus}CH_2$-CO-$R^4$) by

reaction of compound [XX] with a base in a dried solvent and reacting the resulting carboanion with compound [XIX].

This condensation reaction is usually carried out in a solvent such as dried tetrahydrofuran, dried diethyl ether or dried toluene, which does not interfere with the reaction, or a mixture thereof.

This condensation reaction is preferably conducted under a dried inert gas atmosphere, usually from at room temperature to under cooling, preferably at -80 to 30 °C. The reaction temperature is not particularly limited.

Compound [XXIb] can be produced by subjecting compound [XXIa] to reduction reaction for reduction of the carbonyl group.

Since this reduction reaction can be conducted in substantially the same manner as the reduction reaction of Process 5, for the reaction conditions, reference can be made to the explanation of Process 5.

Compound [VIa] and compound [VIb] or their salts can be produced by subjecting, if necessary, compound [XXIa] and compound [XXIb] respectively to N-protective group-eliminating reaction.

This elimination reaction can be conducted in substantially the same manner as the N-protective group-eliminating reaction in Step 3 of Process 1, and thus, concerning the reaction conditions, reference can be made to the explanation of Step 3 of Process 1.

Process D

Step (h)

Compound [XVI] can be produced by converting the carbonyl group of compound [XVII] into a carbonyl-protective group.

Examples of the preferred carbonyl-protective group include protective groups forming chain ketals such as dimethylketal and dibenzylketal; cyclic ketals such as 1,3-dioxane and 1,3-dioxolane; chain dithioketals such as S,S′-dimethylketal, S,S′-diethylketal and S,S′-diphenylketal and cyclic dithioketals such as 1,3-dithiane and 1,3-dithiolane.

The conversion of the carbonyl group of compound [XVII] into a carbonyl-protective group can be carried out easily by a known method [See Protective Groups in Organic Synthesis, pp 114 - 151 (John. Wiley and Sons, Inc. 1981)].

Step (i)

Compound [VII] or its salt can be produced by hydrolysis of compound [XVI].

This hydrolysis can be conducted in substantially the same manner as the hydrolysis in Step (C), but it is preferably conducted in the presence of a base.

As for the other reaction conditions, reference can be made to the explanation of Step (c).

Step (j)

Compound [VII] or its salt can be produced by converting the carbonyl group of compound [VIa] or its salt into a carbonyl-protecting group.

This step can be carried out in substantially the same manner as in Step (h), and thus for this step, reference can be made to the explanation of Step (h).

Process E

The starting compound [VIb] or its salt can also be produced by the production method as shown in the following reaction schemes.

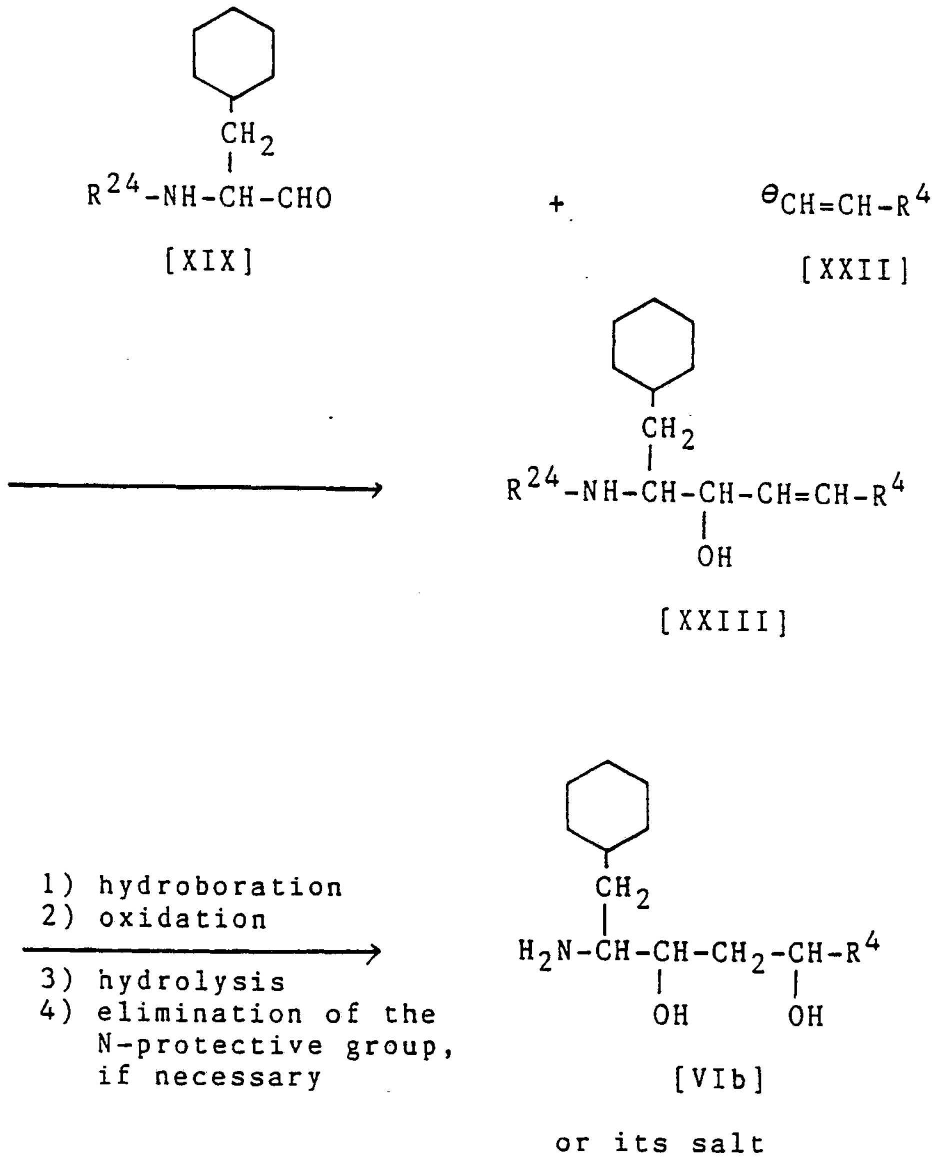


[XIX] + [XXII] → [XXIII] → [VIb] or its salt

wherein $R^4$ is a lower alkyl group; $R^{24}$ is hydrogen or an N-protective group].

Compound [XXIII] can be produced by reaction of compound [XIX] with vinylanion [XXII].

This reaction can be carried out by a per se known method [See Advanced Organic Chemistry, Second Edition,. Vol. B, pp 249 - 305 (Plenum Press 1983)].

Compound [VIb] or its salt can be produced by subjecting compound [XXIII] to (1) hydroboration reaction, (2) oxidation reaction, (3) hydrolysis reaction, if necessary, followed by N-protective group eliminating reaction.

The hydroboration reaction, oxidation reaction and hydrolysis reaction can be conducted by known methods [See Advanced Organic Chemistry, Second Edition, Vol. B, pp 167 -191 (Plenum Press 1983)], and for the N-protective group-elimination reaction, reference can be made to the explanation of Step 3 of Process 1.

The starting compounds [II] as referred to in Process 1 are known in literatures or can be produced by known methods [See, for example, Journal of Medicinal Chemistry, Vol. 31, pp 1839 - 1846 and pp 2277 - 2288 (1988), Japanese Patent Unexamined Publication (Kokai) No. 236770/1986, Kokai No. 19071/1989, European Patent No. 0229667].

The starting compounds [III] and [IX] are known in literatures or can be produced by known methods [See, for example, Canadian Journal of Chemistry, Vol. 49, pp 1968 -1971 (1971), Vol. 51, pp 1915 - 1919 (1973), and Vol. 55, pp 906 - 910 (1977)].

The compounds obtained in accordance with the aforementioned production methods can be isolated

and purified by a conventional means such as pulverization, recrystallization, column chromatography or reprecipitation.

The compounds [I] and the other compounds of the present invention have at least one stereoisomer(s) based on the asynmetric carbon, and all of these isomers and the mixtures thereof are encompassed in the scope of this invention.

The compounds [I] and their esters, and their salts of the present invention can be used in a form of pharmaceutical preparations suitable for oral administration, non-oral administration or external administration by mixing them as an active ingredient with solid or liquid organic or inorganic excipients. Examples of the pharmaceutical preparations include capsules, tablets, sugar-coated tablets, granules, solutions, suspensions and emulsions. If desired, there can be contained adjuvants, stabilizing agents, wetting agents, emulsifier, buffer and other conventionally usable additives in the above preparations.

While the dosage of compounds [I] varies depending on the age and conditions of the patients, the mean dosage is usually about 0.1 mg, 1 mg, 10 mg, 50 mg, 100 mg, 250 mg, 500 mg or 1000 mg, at which the compounds [I] are effective for the treatment of hypertension and cardiac insufficiency. In general, the compounds [I] are administered at a daily dosage ranging from 0.1 mg/individual to about 1000 mg/individual.

For the purpose of showing utility of the objective compounds [I], pharmacological experiments were conducted using the representative compounds [I].

Experiment Example 1

Inhibitory effects against human plasma renin activity

The incubation mixture contained 200 µl of human plasma, 20 µl of pH generator, 10 µl of phenylmethylsulfonyl fluoride (PMSF) and 10 µl of dimethylsulfoxide (DMSO) solution of the present compounds or DMSO alone as control.

After 1 hour of incubation (37° C), the amount of angiotensin I (AT I) formed was measured by a radioimmunoassay. Plasma renin activity (PRA) was calculated as the rate of AT I formation.

The inhibitory effects of the present compounds were estimated as percent inhibition in accordance with the following formula.

$$\%\ \text{Inhibition} = \frac{\text{PRA value of control} - \text{PRA value in the presence of the compound of the present invention}}{\text{PRA value of control}} \times 100$$

The concentration of a compound producing 50% inhibition ($IC_{50}$) was determined graphically on a concentration-inhibition curve. [RENIN• RIABEAD kit (Dinabot Corp.) was used for this experiment.]

| Test Result | |
|---|---|
| Compound No. | $IC_{50}$ (M) |
| 1 | $5.3 \times 10^{-10}$ |
| 2 | $1.9 \times 10^{-8}$ |
| 3 | $2.9 \times 10^{-9}$ |
| 4 | $3.4 \times 10^{-9}$ |
| 6 | $1.5 \times 10^{-9}$ |
| 7 | $3.9 \times 10^{-9}$ |
| 8 | $6.1 \times 10^{-9}$ |
| 9 | $3.8 \times 10^{-9}$ |
| 11 | $4.0 \times 10^{-9}$ |
| 14 | $2.6 \times 10^{-9}$ |
| 16 | $6.8 \times 10^{-10}$ |
| 17 | $8.6 \times 10^{-10}$ |
| 18 | $4.2 \times 10^{-8}$ |
| 19 | $2.9 \times 10^{-8}$ |
| 20 | $7.4 \times 10^{-10}$ |
| 21 | $2.8 \times 10^{-10}$ |
| 22 | $1.3 \times 10^{-9}$ |
| 23 | $2.3 \times 10^{-8}$ |
| 24 | $3.3 \times 10^{-8}$ |
| 25 | $2.0 \times 10^{-9}$ |
| 28 | $6.6 \times 10^{-8}$ |
| 29 | $4.9 \times 10^{-8}$ |
| 33 | $2.5 \times 10^{-9}$ |
| 34 | $5.7 \times 10^{-8}$ |
| 36 | $5.7 \times 10^{-9}$ |
| 37 | $8.6 \times 10^{-10}$ |
| 38 | $4.9 \times 10^{-9}$ |
| 40 | $5.2 \times 10^{-10}$ |
| 42 | $7.4 \times 10^{-10}$ |

Experiment Example 2

Hypotensive effects in conscious, sodium-restricted marmosets

Marmosets weighing 330 - 420 g were fed a low sodium diet (0.02% sodium chloride, about one-tenth of oridinary diet) for 1 week. The test compound was dissolved in 0.1 M citric acid and given orally (10 mg/kg) in a volume of 1 ml/kg. Blood pressure was measured by the indirect tail-cuff method before and 1, 3, 5 and 7 hours after the administration of the compound.

Hypotensive effects were estimated as percent change of blood pressure against the value before administration.

| Compound No. | Dose (mg/kg.po) | Hypotensive effects (%) | | | |
|---|---|---|---|---|---|
| | | 1 hr. | 3 hrs. | 5 hrs. | 7 hrs. |
| 1 | 10 | 11.8 | 13.0 | 7.8 | 3.5 |
| 16 | 10 | 16.8 | 15.0 | 12.4 | 6.2 |

As is clear from the aforementioned Experiment Example 1, the object compound [I] of the present invention possesses potent inhibitory activities against renin and in addition, as is clear from Experiment Example 2 described above, the compound [I] exhibits specific hypotensive action in in vivo experiment by oral administration.

Thus, the object compound [I] of the present invention is extremely useful as a hypotensor and an agent for heart failure for oral administration.

The preferred production methods for the objective compounds [I] and the intermediate compounds therefor are described by working examples. In the following production examples are specifically shown the production methods of the starting compounds to be used for the working examples. The production methods shown in the following working examples and production examples are far from being limitative. The abbreviations used in the working examples and production examples have the following meanings.
NMR Nuclear Magnetic Resonance Spectra ($^1$H-NMR)
SIMS Secondary-Ion Mass Spectra
(SIMS is measured in a low resolution measurement, and the measurement correctness is ±0.3 mass unit).

In the working examples and production examples, Rf values of thin layer chromatography show the results obtained with the use of Pre-coated TLC Plates SILICA GEL 60F-254 (layer thickness of 0.25 mm), and preparatory thin layer chromatography and column chromatography were perfected respectively with use of Pre-Coated TLC Plates SILICA GEL 60 F-254 (layer thickness of 0.25 - 2 mm) and Kieselgel 60 (70-230 mesh) of Merck Corp.

Preparation 1

(3S)-3-(N-tert-Butoxycarbonyl)amino-4-cyclohexyl-1,2-epoxybutane (Compound 1)

To N-(tert-butoxycarbonyl)-L-phenylalanine (59.1 g) in methanol (100 ml) is added 5% rhodium alumina (6 g), and the mixture is hydrogenated under the pressure of 3 kg/cm$^2$. After filtration of the catalyst, the solvent is distilled off under reduced pressure to give (2S)-2-(N-tert-butoxycarbonyl)amino-3-cyclohexylpropionic acid (59.2 g) as colorless, sticky oil.

(2S)-2-(N-tert-Butoxycarbonyl)amino-3-cyclohexylpropionic acid (81.3 g) in dry tetrahydrofuran (150 ml) is dropwise added to a 1 M borane tetrahydrofuran solution (600 ml) under an argon atmosphere keeping the internal tempera ture at 5 - 8° C, followed by stirring for 2 hours. To the reaction mixture is added 10% acetic acid in methanol to adjust the pH thereof to 4, and the solvent is distilled off under reduced pressure. To the residue is added diethyl ether (500 ml), and the mixture is washed with an aqueous solution of 0.5 M citric acid (30 ml x 3 times), a saturated aqueous solution of sodium hydrogencarbonate (20 ml x five times) and saturated brine (20 ml x 3 times). The thus-obtained mixture is dried over anhydrous magnesium sulfate, and the solvent is distilled off under reduced pressure to afford 78.5 g of (2S)-2-(N-tert-butoxycarbonyl)amino-3-cyclohexylpropanol as colorless, sticky oil.

A mixture of (2S)-2-(N-tert-butoxycarbonyl)amino-3-cyclohexylpropanol (82.4 g), dry triethylamine (223 ml), dry benzene (104 ml) and dry dimethyl sulfoxide (228 ml) is cooled to 15° C (internal temperature), and thereto is added sulfur trioxide pyridine complex salt (255 g), during which addition the internal temperature is kept at 15 -25° C, followed by stirring for 1 hour. The reaction mixture is poured into 500 ml of water and extracted with ethyl acetate (200 ml x 4 times). The obtained ethyl acetate solutions are combined, washed with a saturated aqueous solution of sodium hydrogencarbonate (50 ml x 3 times) and saturated brine (20 ml x 3 times). The mixture is dried over anhydrous magnesium sulfate, and the solvent is distilled off under reduced pressure to afford 88.3 g of (2S)-2-(N-tert- butoxycarbonyl)amino-3-cyclohexylpropanal.

To a mixed solution of dry tetrahydrofuran (1000 ml) and dry dimethylformamide (2000 ml) is added a potassium hydride-dispersion (35% in oil) under an argon atmosphere, followed by dropwise addition of distilled 1,1,1,3,3,3-hexamethyldisilazane (47.2 g) while stirring at 0° C. The mixture is stirred at 0° C for 1 hour, and dropwise added to methyltriphenylphosphonium bromide (105 g) at 0° C, followed by vigorous stirring at 0° C for 1 hour and cooling to -78° C. To this mixture is added a solution of (2S)-2-(N-tert-butoxycarbonyl)amino-3-cyclohexylpropanal (88.3 g) in dry tetrahydrofuran, and the mixture is stirred at -78° C for 15 minutes. Thereafter, the temperature of the solution is gradually raised to room temperature, and the mixture is stirred at 40° C for 12 hours. The reaction mixture is cooled to room temperature, and added methanol (7.66 ml) and subsequently an aqueous solution of potassium sodium tartrate (a mixture of

a saturated aqueous solution of potassium sodium tartrate and 500 ml of water). The mixture is extracted with ethyl acetate, and the ethyl acetate solution is washed with water and saturated brine, followed by drying over anhydrous magnesium sulfate. The solvent is distilled off under reduced pressure, and the residue is purified by silica gel column chromatography (eluent : ethyl acetate/hexane = 1/9, v/v) to give 8.84 g of (3S)-3-(N-tert-butoxycarbonyl)amino-4-cyclohexyl-1- butene as colorless, sticky oil.
NMR ($CDCl_3$) $\delta$ : 0.8 - 1.85 (m, 13H), 1.45 (s, 9H), 4.18 (br, s, 1 H), 4.37 (br, s, 1 H), 5.02 - 5.19 (m, 2H), 5.74 (m, 1H)

Thereafter, the obtained (3S)-3-(N-tert-butoxycarbonyl)amino-4-cyclohexyl-1-butene (7.0 g) is dissolved in dichloromethane (150 ml), and 3-chloroperbenzoic acid (19.0 g) is added thereto, followed by stirring at room temperature for 4 hours. To the reaction mixture is added ether (300 ml), and the mixture is washed with an aqueous solution of 10% sodium sulfate which has been cooled to 0 °C, a saturated aqueous solution of sodium hydrogencarbonate and saturated brine, followed by drying over anhydrous magnesium sulfate. The solvent is distilled off under reduced pressure and the residue is purified by silica gel column chromatography (eluent : ethyl acetate/hexane = 1/9, v/v) to give 1.77 g of the title Compound 1 as white amorphous solid.

Preparation 2

2-Isopropyl-1,3-dithiane (Compound 2)

Distilled isobutylaldehyde (72.1 g) and 1,3-propanedithiol (108.2 g) are dissolved in chloroform (2 l), and the mixture is stirred at room temperature for 1 hour. The mixture is cooled to -20 °C, and boron trifluoride-diethyl ether complex (28.4 g) is added thereto. The mixture is allowed to become room temperature while stirring. After stirring at room temperature for 1 hour, the mixture is washed with water, an aqueous solution of 10% potassium hydroxide and water successively, and dried over anhydrous potassium carbonate. The solvent is distilled off under reduced pressure, and the residue is distilled under reduced pressure to afford 116 g of the title Compound 2 as colorless oil.
Boiling point : 80 - 80.5 °C/3mmHg

Preparation 3

3-Morpholinocarbonyl-2-(1-naphthylmethyl)propionic acid (Compound 3)

To ethyl succinate (32.3 g) and 1-naphthaldehyde (29.0 g) in absolute ethanol (320 ml) is added a sodium hydride-dispersion (55% in oil, 9.72 g) under ice-cooling. Thereafter, the mixture is refluxed under heating for 30 minutes. To this solution is added an aqueous solution of 1 M sodium hydroxide (230 ml), and the mixture is refluxed under heating for 1 hour. The solvent is distilled off under reduced pressure and to the residue is added water (230 ml). After the neutral moiety is extracted with ether, cencentrated hydrochloric acid is added to the water layer for acidifying, and the mixture is extracted with ether. The ether layer is washed with saturated brine, and dried over anhydrous magnesium sulfate. The solvent is distilled off under reduced pressure, and to the residue is added benzene. The precipitated crystals are filtrated to afford 28.6 g of 2-(1-naphthylmethylene)succinic acid as yellow crystals.

To the obtained 2-(1-naphthylmethylene)succinic acid (24.5 g) is added acetic anhydride (260 ml), and the mixture is heated at 60 °C for 1 hour. The solvent is distilled off under reduced pressure, and to the residue is added a mixed solution of benzene/hexane = 1/1 (v/v). The precipitated crystals are filtrated to afford 14.5 g of 2-(1-naphthylmethylene)succinic anhydride as yellow-reddish crystals.

2-(1-Naphthylmethylene)succinic anhydride (14.0 g) and morpholine (5.18 g) are dissolved in dry dichloromethane (340 ml), and stirred at room temperature for 2 hours. The solvent is distilled off under reduced pressure, and the residue is crystallized from a mixed solution of ethyl acetate/benzene/hexane = 1/1/1 (v/v) to afford 14.3 g of 3-morpholinocarbonyl-2-(1-naphthylmethylene)propionic acid as coloreless crystals. To the propionic acid (7.0 g) in methanol (280 ml) is added 10% palladium-carbon (0.7 g), and the mixture is hydrogenated under atmospheric pressure. The catalyst is filtered off and the solvent is distilled

off under reduced pressure to afford 7.0 g of the title Compound 3 as colorless, sticky oil.

Preparation 4

$N^{\alpha}$-[3-Morpholinocarbonyl-2-(1-naphthylmethyl)propionyl]-L-histidine methyl ester (Compound 4)

3-Morpholinocarbonyl-2-(1-naphthylmethyl)propionic acid (Compound 3, 3.50 g) and L-histidine methyl ester dihydrochloride (3.11 g) are suspended in 90.5 ml of N,N-dimethyl formamide. Thereto are added diphenylphosphoryl azide (2.73 ml) and triethylamine (5.9 ml) while stirring under ice-cooling, and the mixture is stirred at 0° C for 18 hours. The solvent is distilled off under reduced pressure, and to the residue is added an aqueous solution of 5% sodium hydrogencarbonate (100 ml), followed by extraction with ethyl acetate. The ethyl acetate solution is washed with saturated brine and dried over anhydrous magnesium sulfate. The solvent is distilled off under reduced pressure, and to the residue is added diethyl ether, followed by filtration after stirring to give white powder (3.53 g). The obtained white powder (3.45 g) is separated and purified by silica gel column chromatography (eluent : chloroform/methanol/28% ammonia water = 95/3/0.6, v/v) to give 1.46 g of the title Compound 4 as white powder.

Preparation 5

$N^{\alpha}$-[3-Morpholinocarbonyl-2-(1-naphthylmethyl)propionyl]-L-histidinehydrazide (Compound 5)

The above-mentioned Compound 4 (1.25 g) is dissolved in methanol (12.8 ml), and thereto is added hydrazine monohydrate (1.9 g), followed by stirring at room temperature for 4.5 hours. The solvent is distilled off under reduced pressure, and the residue is purified by silica gel column chromatography (eluent : chloroform/methanol/28% ammonia water = 93/7/1, v/v) to give 1.04 g of the title Compound 5 as white powder.

Preparation 6

$N^{\alpha}$-tert-Butoxycarbonyl-$N^{\alpha}$-methyl-L-histidinehydrazide (Compound 6)

$N^{\alpha}$-tert-Butoxycarbonyl-$N^{\alpha}$-methyl-$N^{im}$-tosyl-L-histidine methyl ester (15 g) obtained from $N^{\alpha}$-tert-butoxycarbonyl-$N^{im}$-tosyl-L-hystidine by the method described in Canadian Journal of Chemistry, 49, 1968 - 1971 (1971) and ibid, 55, 906 - 910 (1977) is dissolved in a mixed solution of acetic anhydride (150 ml) and pyridine (3 ml), followed by stirring at room temperature for 5 hours. The mixture is concentrated under reduced pressure, and to the residue is added methanol (250 ml), followed by stirring at room temperature for 2 hours. The obtained reaction mixture is concentrated under reduced pressure, and water (150 ml) is added to the residue. Thereto is added sodium hydrogencarbonate powder to adjust the pH to 8. Thereafter, the mixture is extracted with chloroform (100 ml x 2 times), and the combined chloroform solution is washed with saturated brine, and dried over anhydrous magnesium sulfate. The solvent is distilled off under reduced pressure to afford 10 g of orange-reddish, oily $N^{\alpha}$-tert-butoxycarbonyl-$N^{\alpha}$-methyl-L-histidine methyl ester.

Rf : 0.21 (solvent : chloroform/methanol = 10/1, v/v)

The thus-obtained ester (9.5 g) is dissolved in methanol (200 ml), and thereto is added hydrazine monohydrate (16.3 ml), followed by stirring at room temperature for 16 hours. The solvent is distilled off under reduced pressure and the residue is purified by silica gel column chromatography (eluent : chloroform/methanol = 10/1, v/v) to give 6.9 g of the title Compound 6 as white amorphous solid.

Preparation 7

$N^{\alpha}$-(N-tert-Butoxycarbonyl-L-phenylalanyl)-$N^{\alpha}$-methyl-L-histidinehydrazide (Compound 7)

In the same manner as in Preparation 6, there is obtained 1.03 g of $N^{\alpha}$-benzyloxycarbonyl-$N^{\alpha}$-methyl-L-histidine methyl ester as pale-yellow sticky oil from $N^{\alpha}$-benzyloxycarbonyl-$N^{im}$-tosyl-L-histidine.
Rf : 0.21 (solvent : chloroform/methanol/28% ammonia water = 90/10/1, v/v)

$N^{\alpha}$-Benzyloxycarbonyl-$N^{\alpha}$-methyl-L-histidine methyl ester (0.5 g) is dissolved in methanol (25 ml), and thereto is added 10% palladium-carbon (50 mg), followed by 3 hours' hydrogenation under atmospheric pressure. The catalyst is filtered off, and the filtrate is concentrated under reduced pressure to afford 0.28 g of $N^{\alpha}$-methyl-L-histidine methyl ester as colorless, sticky oil.
Rf : 0.16 (solvent : chloroform/methanol/28% ammonia water = 90/10/1, v/v)

$N^{\alpha}$-Methyl-L-histidine methyl ester (0.24 g) and N-tert-butoxycarbonyl-L-phenylalanine (0.71 g) are dissolved in dichloromethane (15 ml), and cooled to 0° C. To this solution is added diethyl cyanophosphonate (0.44 g) in dichloromethane (5 ml), and triethylamine (0.27 g) is further added thereto, followed by stirring at 0° C for 30 minutes. The mixture is further stirred at room temperature for 24 hours. To the reaction mixture is added dichloromethane (10 ml), and washed with a saturated aqueous solution of sodium hydrogencabonate, saturated brine, citric acid buffer (pH 5) and saturated brine, followed by drying over anhydrous magnesium sulfate. The solvent is distilled off under reduced pressure. The residue is purified by preparative thin-layer chromatography (solvent : chloroform/methanol/28% ammonia water = 80/20/5, v/v) to give 0.98 g of $N^{\alpha}$-(N-tert-butoxycarbonyl-L-phenylalanyl)-$N^{\alpha}$-methyl-L-hystidine methyl ester in pale-yellow solid.
Rf : 0.54 (solvent : chloroform/ methanol = 5/1, v/v)

The thus-obtained ester (0.36 g) is dissolved in methanol (10 ml), and thereto is added hydrazine monohydrate (0.42 g), followed by stirring at room temperature for 40 hours. The reaction mixture is concentrated under reduced pressure. The residue is purified by preparative thin-layer chromatography (solvent : chloroform/methanol/28% ammonia water = 80/20/5, v/v) to give 0.26 g of the title Compound 7 as pale-yellow solid.

Preparation 8

N-Morpholinocarbonyl-L-phenylalanine (Compound 8)

To L-phenylalanine benzyl ester (5 g) in tetrahydrofuran (25 ml) are added activated carbon (50 mg) and trichloromethyl chloroformate (1.8 ml) while stirring. After stirring for 10 minutes under ice-cooling, the activated carbon is filtered off with celite, and the celite is washed with ethyl acetate. The filtrate and the washing solution are combined, and the solvent is distilled off under reduced pressure to afford red, oily substance. This substance is dissolved in dichloromethane (50 ml), and thereto are added pyridine (16 ml), 4-dimethylaminopyridine (2.4 g) and morpholine (3.5 ml). After the reaction mixture is stirred at room temperature for 2 hours, a precipitate is filtered off and washed with dichloromethane. The filtrate is combined with the washing solution, and the solvent is distilled off under reduced pressure. The residue is dissolved in diethyl ether (100 ml), washed with an aqueous solution of 0.5 M succinic acid and saturated brine, and dried over anhydrous magnesium sulfate. The solvent is distilled off under reduced pressure and the residue is purified by silica gel column chromatography (eluent : ethyl acetate/hexane = 1/1, v/v) to give 4.3 g of N-morpholinocarbonyl-L-phenylalanine benzyl ester as colorless oil.

To N-morpholinocarbonyl-L-phenylalanine benzyl ester (4 g) in methanol (120 ml) is added 10% palladium-carbon (0.4 g), followed by hydrogenation under atmospheric pressure. The catalyst is filtered off, and the solvent is distilled off under reduced pressure to afford 2.8 g of the title Compound 8 as white powder.

Preparation 9

(2S)-2-Morpholinocarbonyloxy-3-phenylpropionic acid (Compound 9)

To L-phenylalanine (50 g) suspended in chloroform is added 12 M hydrochloric acid (30 ml), and the mixture is stirred at room temperature for 5 minutes. The solvent is distilled off under reduced pressure to afford L-phenylalanine hydrochloride as white solid. The obtained hydrochloride is dissolved in a 5% sulfuric acid aqueous solution (900 ml), and thereto is dropwise added a solution of sodium nitrite (45 g) in water (240 ml) while stirring in an ice bath over a period of 40 minutes, during which addition the temperature of the reaction mixture is maintained at 2 - 3° C (internal temparature). The reaction mixture is further stirred for 4 hours under ice-cooling and at room temperature for 15 hours. The reaction mixture is extracted with diethyl ether (500 ml x 2 times). The extracts are dried over anhydrous magnesium sulfate and the solvent is distilled off under reduced pressure. The residue is dissolved in benzene and the solvent is distilled off under reduced pressure (this procedure is repeated one more time). The residue is recrystallized from benzene (400 ml) to afford 29 g of (2S)-2-hydroxy-3-phenylpropionic acid as white crystals.

The obtained (2S)-2-hydroxy-3-phenylpropionic acid (9.96 g) is suspended in benzene (300 ml), and thereto is added 1,8-diazabicyclo[5,4,0]-7-undecene (9.13 g), followed by stirring at room temperature. After the (2S)-2-hydroxy-3-phenylpropionic acid is dissolved, benzyl bromide (12.3 g) in benzene (50 ml) is added thereto, and the mixture is refluxed under heating for 2 hours. While the reaction mixture is left standing for cooling, an oily substance produced therein solidifies as white crystals. This reaction mixture is filtrated and the white crystals are washed with benzene. The filtrate is combined with the washing solution, washed with water, 1 M hydrochloric acid, an aqueous solution of 0.5 M sodium hydrogencarbonate and saturated brine, and dried over anhydrous magnesium sulfate. The solvent is distilled off under reduced pressure and the residue is purified by silica gel column chromatography (eluent : chloroform) to give 12.1 g of (2S)-2-hydroxy-3-phenylpropionic acid benzyl ester as colorless oil.

The obtained (2S)-2-hydroxy-3-phenylpropionic acid benzyl ester (0.5 g) is dissolved in tetrahydrofuran (2.5 ml), and thereto is added activated carbon (5 mg), followed by addition of trichloromethyl chloroformate (0.18 ml) while stirring at room temperature. This reaction mixture is heated to 55° C over a period of 2 hours, and stirred at 55° C for 30 minutes. The activated carbon is filtered off with celite, and the solvent is distilled off under reduced pressure to give pale-yellow, oily substance. The thus-obtained oily substance is dissolved in dichloromethane (10 ml), and thereto are added pyridine (1.6 ml) and 4-dimethylaminopyridine (0.25 g). Thereafter, morpholine (0.35 ml) is added and the mixture is stirred at room temterature for 14 hours. The precipitated white solid is filtered off, and the white solid is washed with chloroform. The filtrate is combined with the washing solution, and the solvent is distilled off under reduced pressure. The residue is extracted with diethyl ether (10 ml), and the diethyl ether solution is washed with an aqueous solution of 0.3 M succinic acid and saturated brine. The diethyl ether solution is dried over anhydrous magnesium sulfate. The solvent is distilled off under reduced pressure and the residue is purified by preparative thin-layer chromatography (solvent : ethyl acetate/hexane = 35/65, v/v) to give 0.37 g of (2S)-2-morpholinocarbonyloxy-3-phenylpropionic acid benzyl ester as colorless oil.

To the obtained (2S)-2-morpholinocarbonyloxy-3-phenyl-propionic acid benzyl ester (0.37 g) in methanol (15 ml) is added 10% palladium-carbon (37 mg) and hydrogenated under atmospheric pressure. The catalyst is filtered off and the solvent is distilled off under reduced pressure to afford 0.3 g of the title Compound 9 as colorless, sticky oil.

## Preparation 10

2-Benzyl-3-tert-butylsulfonylpropionic acid (Compound 10)

To dimethyl benzylmolonate (97 g) in methanol (300 ml) is added an aqueous solution of 1 M sodium hydroxide, followed by stirring at room temperature for 15 minutes. The reaction mixture is added to water (1 l), and thereto is dropwise added 6 M hydrochloric acid for acidifying (pH 3). This mixture is extracted with chloroform (750 ml x 2 times). The extract is dried over anhydrous magnesium sulfate and the solvent is distilled off under reduced pressure to afford the colorless, oily residue (75 g). The residue is dissolved in pyridine (40 ml), and thereto are added piperidine (2.4 ml) and paraformaldehyde (7.2 g), followed by heating in an oil bath (130° C) for 1.5 hours. After cooling, the reaction mixture is poured into water (500 ml), and extracted with n-hexane (200 ml x 2 times). The combined n-hexane solution is washed with water,

1 M hydrochloric acid, water, a saturated aqueous solution of sodium hydrogencarbonate and saturated brine successively. The solvent is distilled off under reduced pressure to give methyl 2-benzylacrylate (35 g) as colorless oil.

To methyl 2-benzylacrylate (14.7 g) in tetrahydrofuran (160 ml) are added tert-butyl mercaptan (9.4 ml) and a sodium hydride-dispersion (60% in oil, 1.7 g) while stirring in an ice bath. After stirring at room temperature for 4 hours, the mixture is gradually added to 1 M hydrochloride (300 ml) under ice-cooling, followed by extraction with ethyl acetate (200 ml). The ethyl acetate solution is washed with saturated brine (100 ml), and dried over anhydrous magnesium sulfate. The solvent is distilled off under reduced pressure and the residue is purified by silica gel column chromatography (eluent : hexane and then ethyl acetate/hexane = 1/1, v/v) to give 19.7 g of 2-benzyl-3-tert-butylthiopropionic acid methyl ester as pale-yellow oil.

To 2-benzyl-3-tert-butylthiopropionic acid methyl ester (5 g) in methanol (80 ml) is dropwise added OXONE (trademark) [monopersulfate compound, 50% $KHSO_5$, Aldrich Corp., 16 g] in water (70 ml) under ice-cooling. The reaction mixture is stirred at room temperature for 24 hours and filtrated. The filtrate is concentrated under reduced pressure and methanol is distilled off. The residual water layer is extracted with chloroform (150 ml x 2 times), and the combined chloroform solution is washed with a saturated aqueous solution of sodium hydrogencarbonate and saturated brine, followed by drying over anhydrous magnesium sulfate. The solvent is distilled off under reduced pressure to give 2-benzyl-3-tert-butylsulfonylpropionic acid methyl ester (3.0 g) as white, amorphous solid.

To 2-benzyl-3-tert-butylsulfonylpropionic acid methyl ester (0.7 g) are added 6 M hydrochloric acid (6 ml) and acetic acid (1.2 ml), followed by reflux under heating for 7 hours. After cooling, water (15 ml) is added to the reaction mixture, and the mixture is extracted with chloroform (15 ml x 2 times). The chloroform solution is dried over anhydrous magnesium sulfate, and the solvent is distilled off under reduced pressure to give 0.66 g of the title Compound 10 as white amorphous solid.

## Preparation 11

### (2S)-2-Benzyl-3-tert-butylsulfonylpropionic acid (Compound 11)

To 2-benzyl-3-tert-butylsulfonylpropionic acid methyl ester (Compound 10, 500 mg) in N,N-dimethylformamide (8 ml) are added 1-hydroxybenzotriazole monohydrate (262 mg) and dicyclohexylcarbodiimide (472 mg) under ice-cooling, followed by stirring for 40 minutes. To this mixture is added (2S)-2-amino-3-phenylpropanol (293 mg) in N,N-dimethylformamide (8 ml) under ice-cooling, and the mixture is stirred at room temperature for 43 hours. The reaction mixture is filtrated, and the solvent is distilled off under reduced pressure. To the residue is added ethyl acetate (20 ml), followed by stirring. After filtration of the precipitated salt, the ethyl acetate solution is washed with an aqueous solution of 1 M sodium hydrogencarbonate, and dried over anhydrous magnesium sulfate. The solvent is distilled off under reduced pressure. The residue is purified by preparative thin-layer chromatography (solvent : ethyl acetate/hexane = 4/1, v/v) to give 210 mg of the unpolar isomer and 145 mg of the polar isomer of (2S)-2-[(2S)-2-benzyl-3-tert-butylsulfonylpropionyl]amino-3-phenylpropanol.
Unpolar isomer :
Rf : 0.49 (solvent : ethyl acetate/hexane = 4/1, v/v)
Polar isomer :
Rf : 0.33 (solvent : ethyl acetate/hexane = 4/1, v/v)
[As regards the absolute configuration of the above, see Journal of Medicinal Chemistry, 31, 1839 - 1846 (1988).]

To the afore-mentioned unpolar isomer compound (200 mg) are added acetic acid (1 ml) and 6 M hydrochloric acid (3 ml), and the mixture is heated to 90°C, and thereafter stirred for 6 hours. The reaction mixture is concentrated to about half the amount under reduced pressure and thereto is added water (15 ml), followed by extraction with chloroform (15 ml x 2 times). The combined chloroform solution is washed with 1 M hydrochloric acid, and dried over anhydrous sodium sulfate. The solvent is distilled off under reduced pressure and the residue is purified by preparative thin-layer chromatography to afford (2S)-2-benzyl-3-tert-butylsulfonylpropionic acid (43 mg) as colorless oil. The Rf value and NMR data are the same as for Compound 10.

Preparation 12

N-tert-Butoxycarbonyl-O-methyl-L-tyrosine (Compound 12)

To N-tert-butoxycarbonyl-L-tyrosine (545 mg) in an aqueous solution of 10% potassium hydroxide (3 ml) is added dimethylsulfuric acid (366 mg). After stirring at room temperature for 30 minutes, thereto is added dimethylsulfuric acid (133 mg), followed by 30 minutes' stirring at room temperature. To the reaction mixture is added water (5 ml), and the mixture is washed with diethyl ether (5 ml). To the aqueous solution is added 6N hydrochloric acid to adjust the pH to 3.5, and the mixture is extracted with chloroform (10 ml x 2 times). The chloroform solution is washed with saturated brine, and dried over anhydrous magnesium sulfate. The solvent is distilled off under reduced pressure to give 355 mg of the title Compound 12 as colorless, sticky oil.

Preparation 13

3-Benzyloxycarbonylamino-3-methyl butyric acid (Compound 13)

By the preparation method in Bullentin de la Société Chimique de France, 828 - 830 (1964), 2,2-dimethyl-3-carbomethoxypropionic acid is obtained from 2,2-dimethylsuccinic acid.

The 2,2-dimethyl-3-carbomethoxypropionic acid (4.0 g) is dissolved in toluene (30 ml), and thereto are added triethylamine (2.5 g) and diphenylphosphoryl azide (6.9 g), followed by reflux under heating for 1.5 hours. After leaving the mixture for cooling, benzyl alcohol (2.7 g) is added to the reaction mixture, and the mixture is refluxed under heating for 24 hours. Thereafter, the reaction mixture is added to 0.5 N hydrochloric acid under ice-cooling, and the toluene solution is washed with 0.5 N hydrochlioric acid, a saturated aqueous solution of sodium hydrogencarbonate and saturated brine, followed by drying over anhydrous magnesium sulfate. The solvent is distilled off under reduced pressure and the residue is purified by silica gel column chromatography (eluent : ethyl acetate/hexane = 10/90, v/v) to give 6.0 g of 3-benzyloxycarbonylamino-3-methylbutyric acid methyl ester as yellow oil.

To the 3-benzyloxycarbonylamino-3-methylbutyric acid methyl ester (6.0 g) in methanol (30 ml) is added an aqueous solution of 2N sodium hydroxide (15 ml), and the mixture is stirred at room temperature for 2 hours. Methanol is distilled off under reduced pressure, and to the residual aqueous mixture is added 6N hydrochloric acid to adjust the pH to 2 -3, followed by extraction with chloroform (40 ml x 2 times). The chloroform solution is dried over anhydrous magnesium sulfate, and the solvent is distilled off under reduced pressure to afford 5.3 g of the title Compound 13 as colorless, sticky oil.

Preparation 14

N-(4-Hydroxypiperidino)carbonyl-L-phenylalanine (Compound 14)

In the same manner as in Preparation 8, N-(4-hydroxypiperidino)carbonyl-L-phenylalanine benzyl ester (1.3 g) is obtained as colorless oil from L-phenylalanine benzyl ester (5 g) and 4-hydroxypiperidine (4.0 g). The thus-obtained ester is hydrogenated to afford 1.0 g of the title Compound 14 as white powder.

Preparation 15

3-(3-Pyridyl)propionic acid (Compound 15)

3-(3-Pyridyl)-1-propanol (4.1 g) is dissolved in a mixed solution of 95% sulfuric acid (1.13 ml) and water (48 ml), and thereto is added potassium permanganate (6.3 g), during which addition the internal temperature is maintained at 50° C. After a change of color of the reaction mixture from purple to black, the mixture is heated to 80° C, and stirred for 3 minutes. After filtration with celite, the filtrate is concentrated under reduced pressure, and thereto are added ethanol (100 ml) and activated carbon (250 mg), followed by reflux under heating for 5 minutes. After filtration of the activated carbon with celite, the solvent is distilled off under reduced pressure to afford 1.43 g of the title Compound 15 as white powder.

Preparation 16

(2S)-2-(N-tert-Butoxycarbonyl-L-prolyl)oxy-3-phenylpropionic acid (Compound 16)

N-tert-Butoxycarbonyl-L-proline (1.88 g) and (2S)-2-hydroxy-3-phenylpropionic acid benzyl ester (see Preparation 9, 2.0 g) are dissolved in dichloromethane (70 ml), and thereto are added N,N-dicyclohexylcarbodiimide (2.0 g) and 4-dimethylaminopyridine (95 mg), followed by stirring at room temperature for 4 hours. To the reaction mixture is added diethyl ether, and after filtration of salt, the filtrate is concentrated under reduced pressure. The residue is purified by silica gel column chromatography (eluent : ethyl acetate/hexane = 1/9, v/v) to give 4.29 g of (2S)-2-(N-tert-butoxycarbonyl-L-prolyl)oxy-3-phenylpropionic acid benzyl ester as colorless, sticky oil. In the same manner as in Preparation 9, the obtained ester is hydrogenated and purified to give 2.81 g of the title Compound 16 as colorless, sticky oil.

Preparation 17

N-Piperidinocarbonyl-L-phenylalanine (Compound 17)

In the same manner as in Preparation 8 using piperidine in place of morpholine, there is obtained 2.96 g of N-piperidinocarbonyl-L-phenylalanine benzyl ester as yellow, sticky oil.
Rf : 0.52 (solvent : ethyl acetate/hexane = 50/50, v/v)
NMR ($CDCl_3$) $\delta$ : 1.40 - 1.65 (m, 6H),3.11 (m, 2H), 3.27 (m, 4H), 4.85 (m, 2H), 5.03 - 5.24 (m, 2H), 6.93 - 7.45 (m, 10H)

The thus-obtained ester is hydrogenated with palladium-carbon to afford 0.92 g of the title Compound 17 as white powder.

Preparation 18

$N^{\alpha}$-(2-Benzyl-3-tert-butylsulfonylpropionyl)-L-histidinehydrazide (Compound 18, isomer A)

To 2-benzyl-3-tert-butylsulfonylpropionic acid (Compound 10, 500 mg) in N,N-dimethylformamide (8ml) are added 1-hydroxybenzotriazole monohydrate (310 mg) and N,N-dicyclohexylcarbodiimide (360 mg) while stirring under ice-cooling. After stirring for 2 hours under ice-cooling, L-histidine methyl ester (300 mg) in N,N-dimethylformamide (8 ml) is added thereto. The reaction mixture is stirred for 2 hours under ice-cooling and 16 hours at room temperature, followed by filtration. The filtrate is concentrated under reduced pressure. The residue is dissolved in chloroform (20 ml), washed with a saturated aqueous solution of sodium hydrogencarbonate, and dried over anhydrous magnesium sulfate. The solvent is distilled off under reduced pressure and the residue is purified by silica gel column chromatography (eluent : chloroform/methanol = 40/1, v/v) to give 294 mg of the unpolar isomer A and 309 mg of the polar isomer B of $N^{\alpha}$-(2-benzyl-3-tert-butylsulfonylpropionyl)-L-histidine methyl ester.
Unpolar isomer A : white powder
Rf : 0.27 (solvent : chloroform/methanol = 10/1, v/v)

Polar isomer B : colorless, sticky oil
Rf : 0.22 (solvent : chloroform/methanol = 10/1, v/v)

The thus-obtained unpolar isomer A (100 mg) of said ester is dissolved in methanol and thereto is added hydrazine monohydrate (58 mg), followed by 12 hours' stirring at room temperature. The reaction mixture is concentrated under reduced pressure. The residue is purified by preparative thin-layer chromatography (solvent : chloloform/methanol = 5/1, v/v) to give 70 mg of the title Compound 18 (isomer A) as white solid.

## Preparation 19

N-[1-(4-Benzyloxycarbonyl)piperazinylcarbonyl]-L-phenylalanine (Compound 19)

Piperazine (3 g) is dissolved in chloroform (200 ml), and thereto are added triethylamine (1.17 g) and carbobenzoxy chloride (1.98 g), followed by 1 hour's stirring at room temperature. The reaction mixture is washed with a saturated aqueous solution of sodium hydrogencarbonate and concentrated under reduced pressure. Thereto is added diethyl ether (50 ml) and the mixture is extracted with a 0.5 M citric acid aqueous solution (10 ml x 5 times). To the aqueous solution is added sodium hydrogencarbonate to adjust the pH to 7.5, and the mixture is extracted with diethyl ether (40 ml x 4 times). The diethyl ether solution is dried over anhydrous magnesium sulfate and the solvent is distilled off under reduced pressure to give 0.89 g of 4-benzyloxycarbonylpiperazine as yellow-reddish, sticky oil.

By the same reaction and purification as in Preparation 17 using 4-benzyloxycarbonylpiperazine (421 mg) in place of piperidine, there is obtained 272 mg of N-[1-(4-benzyloxycarbonyl)piperazinylcarbonyl]-L-phenylalanine benzyl ester as colorless, sticky oil.

Thereafter, the obtained ester (97 mg) is dissolved in tetrahydrofuran (5 ml), and thereto is added an aqueous solution of 1N sodium hydroxide (0.58 ml), followed by stirring at room temperature for 4 hours. After the reaction mixture is concentrated under reduced pressure, water (30 ml) is added thereto and the mixture is washed with diethyl ether. To the aqueous solution is added an aqueous solution of 0.5 M citric acid to adjust the pH to 3.5, followed by extraction with ethyl acetate (30 ml x 3 times). The ethyl acetate solution is dried over anhydrous magnesium sulfate, and the solvent is distilled off under reduced pressure to afford the title Compound 19 as white powder.

## Preparation 20

(2S)-2-(2,2-Dimethylpropionyl)oxy-3-phenylpropionic acid (Compound 20)

(2S)-2-Hydroxy-3-phenylpropionic acid benzyl ester (see Preparation 9, 500 mg), 4-dimethylaminopyridine (24 mg), and N,N-diisopropylethylamine (252 mg) are dissolved in dichloromethane (10 ml), and thereto are dropwise added pivaloyl chloride (0.36 ml) while stirring under ice-cooling. After stirring at room temperature for 1 hour, the solvent is distilled off under reduced pressure and the residue is purified by silica gel column chromatography (eluent : dichloromethane) to give 674 mg of (2S)-2-(2,2-dimethylpropionyl)oxy-3-phenylpropionic acid benzyl ester as colorless, sticky oil.

The thus-obtained ester (644 mg) is hydrogenated with palladium-carbon to afford 315 mg of the title Compound 20 as colorless, sticky oil.

## Preparation 21

N-[(Tetrahydro-4H-1,4-thiazine)-4-yl-carbonyl]-L-phenylalanine (Compound 21)

In the same manner as in Preparation 8, N-[(tetrahydro-4H-1,4-thiazine)-4-yl-carbonyl]-L-phenylalanine

benzyl ester (6.0 g) is obtained as pale-yellow solid from L-phenylalanine benzyl ester (4.0 g) and thiomorpholine (1.79 g).

The obtained ester (238 mg) is dissolved in methanol (5 ml), and thereto is added an aqueous solution of 1N sodium hydroxide (1.24 ml), followed by stirring at room temperature for 1 hour. After the methanol is distilled off under reduced pressure, water (8 ml) is added thereto and the solution is washed with diethyl ether. Thereto is added 6N hydrochloric acid to adjust the pH to 3, and the mixture is extracted with chloroform (10 ml x 2 times). The chloroform solution is dried over anhydrous magnesium sulfate, and the solvent is distilled off under reduced pressure to afford 168 mg of the title Compound 21 as white powder.

Preparation 22

N-tert-Butoxycarbonylmethoxymethylcarbonyl-L-phenylalanine (Compound 22)

N-Carboxymethoxymethylcarbonyl-L-phenylalanine benzyl ester (Unexamined Japanese Patent Publication No. 183551/1988, 200 mg) and tert-butyl alcohol (36 mg) are dissolved in dichloromethane (10 ml), and thereto are added N,N-dicyclohexylcarbodiimide (111 mg) and 4-dimethylaminopyridine (7 mg) while stirring under ice-cooling, followed by stirring under ice-cooling for 1 hour and at room temperature for 13 hours. The reaction mixture is filtrated and the filtrate is concentrated under reduced pressure. Thereto is added ethyl acetate (20 ml) and the precipitated solid is filtered off. The filtrate is washed with an aqueous solution of 0.5 M citric acid, water, an aqueous solution of 1N sodium hydrogencarbonate and saturated brine, and dried over anhydrous magnesium sulfate. The solvent is distilled off under reduced pressure. The residue is purified by preparative thin-layer chromatography (solvent : ethyl acetate/hexane = 1/1, v/v) to give 50 mg of N-tert-butoxycarbonylmethoxymethylcarbonyl-L-phenylalanine benzyl ester as colorless, sticky oil.

The thus-obtained ester (50 mg) is hydrogenated to give 38 mg of the title Compound 22 as colorless, sticky oil.

Preparation 23

N-[N-(2-Methoxyethoxymethoxyethyl)-N-methylaminocarbonyl]-L-phenylalanine (Compound 23)

N-[N-(2-Hydroxyethyl)-N-methylaminocarbonyl]-L-phenylalanine benzyl ester [Journal of Medicinal Chemistry, 31, 2277 - 2288 (1988), 500 mg] is dissolved in dichloromethane (10 ml), and N,N-diisopropylethylamine (0.77 ml) and 2-methoxyethoxymethylchloride (0.48 ml) are added thereto, followed by stirring at room temperature for 19 hours. The reaction mixture is concentrated under reduced pressure and the residue is dissolved in ethyl acetate (30 ml). The obtained solution is washed with an aqueous solution of 0.5 M citric acid, a saturated aqueous solution of sodium hydrogencarbonate and saturated brine. The ethyl acetate solution is dried over anhydrous magnesium sulfate and the solvent is distilled off under reduced pressure to give 554 mg of N-[N-(2-methoxyethoxymethoxyethyl)-N-methylaminocarbonyl]-L-phenylalanine benzyl ester as pale-yellow, sticky oil. The thus-obtained ester (420 mg) is dissolved in methanol (20 ml), and 10% palladium-carbon (42 mg) is added thereto, followed by hydrogenation under atmospheric pressure for 3 hours. The catalyst is filtered off, and the solvent is distilled off under reduced pressure to give 346 mg of the title Compound 23 as colorless, sticky oil.

Preparation 24

(4S)-4-Cyclohexylmethyl-5-[(2-isopropyl-1,3-dithiane-2-yl)methyl]-1,3-oxazolidin-2-on (Compound 24)

2-Isopropyl-1,3-dithiane (Compound 2, 1.49 g) is dissolved in dry tetrahydrofuran (40 ml) under an

argon atmosphere, and thereto is added dry N,N,N′,N′-tetramethylethylenediamine (45 ml), followed by cooling to -78 °C. To this solution is dropwise added a 1.6 M n-butyllithium hexane solution (5.75 ml) while stirring, and the solution is warmed to 0 °C over a period of 1 hour, followed by cooling again to -60 °C. To this mixture is dropwise added a solution of (3S)-3-(N-tert-butoxycarbonyl)amino-4-cyclohexyl-1,2-epoxybutane (Compound 1, 1.24 g) in dry tetrahydrofuran (10 ml), and the temperature of the mixture is gradually raised to 0 °C, followed by stirring at 0 °C for 70 hours. To this reaction mixture is added ice (10 g), and after stirring, the solution is concentrated under reduced pressure. The residue is extracted with ethyl acetate (20 ml x 4 times). The ethyl acetate solutions are combined, washed with saturated brine, and dried over anhydrous magnesium sulfate. The solvent is distilled off under reduced pressure, and the yellow, oily residue is purified by silica gel column chromatography (eluent : ethyl acetate/hexane = 25/75, v/v) to give 1.33 g of the title Compound 24 as pale-yellow, sticky oil.

Preparation 25

(4S)-4-Cyclohexylmethyl-5-(3-methyl-2-oxobutyl)-1,3-oxazolidin-2-one (Compound 25)

Compound 24 (0.37 g) as obtained in Preparation 24 is dissolved in a mixed solvent (10 ml) of acetonitrile/water = 4/1 (v/v) under a nitrogen atmosphere, and mercury (II) chloride (0.6 g) and calcium carbonate (0.21 g) are added thereto. While vigorously stirring, the mixture is refluxed under heating for 2.5 hours. After the reaction mixture is kept standing for cooling, the white precipitate is filtered off with celite. The celite and the white precipitate are washed with chloroform (70 ml), and the filtrate and the washing solution are combined. The combined solution is washed with an aqueous solution of 5 M ammonium acetate and water. The thus-obtained solution is dried over anhydrous magnesium sulfate, and the solvent is distilled off under reduced pressure to give 0.32 g of the title Compound 25 as white solid.

Preparation 26

(4S)-Cyclohexylmethyl-5-(3-methyl-2-hydroxybutyl)-1,3-oxazolidin-2-one (Compound 26)

Compound 25 (0.32 g) as obtained in Preparation 25 is dissolved in methanol (10 ml), and thereto is added sodium borohydride (89 mg), followed by stirring at room temperature for 1 hour. To this solution is added 1 M hydrochloric acid (3 ml) and the mixture is stirred at room temperature for 5 minutes. Thereafter, a saturated aqueous solution of sodium hydrogencarbonate is added to neutralize the solution. Methanol is distilled off under reduced pressure, and the obtained aqueous mixture is extracted with chloroform (10 ml x 4 times). The chloroform solutions are combined, washed with saturated brine, and dried over anhydrous magnesium sulfate. The solvent is distilled off under reduced pressure and the residue is purified by silica gel column chromatography (eluent : ethyl acetate/hexane = 25/75 and then 30/70, v/v) to give 99 mg of the unpolar isomer and 115 mg of the polar isomer of the title Compound 26.
Unpolar isomer : white solid
Polar isomer : white solid

Preparation 27

(2S)-2-Amino-1-cyclohexyl-6-methyl-3,5-heptanediol (Compound 27, isomer A)

The unpolar isomer (99 mg) of Compound 26 as obtained in Preparation 26 is dissolved in a mixed solution of dioxane/water = 1/1 (v/v) under a nitrogen atmosphere, and thereto is added barium hydroxide octahydrate (174 mg), followed by reflux under heating for 4 hours. After the mixture is left standing for cooling, an aqueous solution of 1 M phosphoric acid (c.a. 0.5 ml) is added to adjust the pH thereof to 4.5,

and dioxane is distilled off under reduced pressure. To the obtained aqueous mixture is added a saturated aqueous solution of sodium hydrogencarbonate (7 ml), and the mixture is extracted with chloroform (10 ml x 4 times). The chloroform solutions are combined, washed with saturated brine, and dried over anhydrous magnesium sulfate. The solvent is distilled off under reduced pressure. The residue is purified by preparative thin-layer chromatography (solvent : chloloform/methanol/28% ammonia water = 80/20/1, v/v) to give 69 mg of the title Compound 27 (isomer A) as white solid.

Preparation 28

(2S)-2-Amino-1-cyclohexyl-6-methyl-3,5-heptanediol (Compound 28, isomer B)

The polar isomer (106 mg) of Compound 26 as obtained in Preparation 26 is treated in the same manner as in Preparation 27 to give 85 mg of the title Compound 28 (isomer B) as pale-yellow, sticky oil.

Preparation 29

(6S)-6-(N-tert-Butoxycarbonyl)amino-7-cyclohexyl-2-methyl-5-hydroxy-3-heptanone (Compound 29)

(2S)-2-(N-tert-Butoxycarbonyl)amino-3-cyclohexylpropanal (3.88 g, see Preparation 1) and 3-methyl-2-butanone (3.9 g) are dissolved in dry tetrahydrofuran under an argon atmosphere, and thereto is dropwise added a mixture of lithium diisopropylamide in a 10% (w/w) hexane suspension (20 ml) and dry tetrahydrofuran (20 ml) while stirring under cooling in a dry ice-ethanol bath over a period of 20 minutes. The reaction mixture is allowed to become room temperature gradually while stirring over a period of 15 hours. Thereafter, water is added thereto, and the mixture is extracted with diethyl ether and then with chloroform. The diethyl ether solution and the chloroform solution are combined, and the solvent is distilled off under reduced pressure. The residue is purified by silica gel column chromatography (eluent : ethyl acetate/hexane = 1/2, v/v) to give 1.81 g of the title Compound 29 as white solid.

Preparation 30

(2S)-2-(N-tert-Butoxycarbonyl)amino-1-cyclohexyl-6-methyl-3,5-heptanediol (Compound 30)

Compound 29 (102 mg) as obtained in Preparation 29 is dissolved in methanol (2 ml), and thereto is added sodium borohydride (57 mg), followed by stirring at room temperature for 5 hours. The reaction mixture is concentrated under reduced pressure and to the residue is added water (5 ml), followed by extraction with ethyl acetate. The ethyl acetate solution is washed with saturated brine, and dried over anhydrous magnesium sulfate. The solvent is distilled off under reduced pressure. The residue is purified by preparative thin-layer chromatography to give 55 mg of the unpolar isomer C and 28 mg of the polar isomer D of the title Com pound 30.
Unpolar isomer C : pale-yellow crystalline solid
Polar isomer D : pale-yellow crystalline solid

Preparation 31

(2S)-2-Amino-1-cyclohexyl-6-methyl-3,5-heptanediol (Compound 31, isomer C)

The unpolar isomer C (27 mg) of Compound 30 as obtained in Preparation 30 is dissolved in trifluoroacetic acid (1 ml), followed by stirring at room temperature for 30 minutes. The reaction mixture is concentrated under reduced pressure and to the residue is added a saturated aqueous solution of sodium hydrogencarbonate (1 ml), followed by extraction with chloroform. The chloroform solution is washed with a saturated aqueous solution of sodium hydrogencarbonate and saturated brine, and dried over anhydrous magnesium sulfate. The solvent is distilled off under reduced pressure. The residue is purified by preparative thin-layer chromatography (solvent : chloloform/methanol = 5/1, v/v) to give 11 mg of the title Compound 31 (isomer C).

Example 1

(2S)-2-[$N^{\alpha}$-{3-Morpholinocarbonyl-2-(1-naphthylmethyl)propionyl}-L-histidyl]amino-1-cyclohexyl-6-methyl-3,5-heptanediol (Compound 1, isomer A)

$N^{\alpha}$-[3-Morpholinocarbonyl-2-(1-naphthylmethyl)propionyl]-L-histidinehydrazide (Compound 5, 47.8 mg) is dissolved in N,N-dimethylformamide (2 ml) and thereto are added a 4 M hydrogen chloride dioxane solution (82.5 µl) at -20°C and then isopentyl nitrite (14.1 mg), followed by stirring. After stirring for 30 minutes, the temperature of the reaction mixture is lowered to -30°C and the mixture is neutralized with triethylamine. To this solution is added a solution of (2S)-2-amino-1-cyclohexyl-6-methyl-3,5-heptanediol (Compound 27, isomer A, 19.5 mg) in 2 ml of N,N-dimethylformamide at -30°C, and thereafter the mixture is stirred at 0°C for 48 hours. The reaction mixture is concentrated under reduced pressure, and chloroform (20 ml) and a saturated aqueous solution of sodium hydrogencarbonate (2 ml) are added thereto. The mixture is stirred for dissolution of the residue, and partitioned after vigorous stirring. The water layer is extracted with chloroform, and the combined chloroform solution is washed with saturated brine, and dried over anhydrous magnesium sulfate. The solvent is distilled off under reduced pressure. The residue is purified by preparative thin-layer chromatography (solvent : chloloform/methanol = 5/1, v/v) to give 30 mg of the title Compound 1 (isomer A) as white powder.
SIMS : $(M + H)^+$ Measured : 690.5
Calculated : 690.4

Example 2

(2S)-2-[ $N^{\alpha}$-[3-Morpholinocarbonyl-2-(1-naphthylmethyl)propionyl-L-histidyl]amino-1-cyclohexyl-6-methyl-3,5-heptanediol (Compound 2, isomer B)

By the same reaction as in Example 1 using Compound 28 (isomer B, 19.5 mg) of Preparation 28 in place of Compound 27, there is obtained 35 mg of the title Compound 2 (isomer B) as white powder.
SIMS : $(M + H)^+$ Measured : 690.5
Calculated : 690.4

Preparation 32

2-Isopropyl-2-[(3S)-3-amino-4-cyclohexyl-2-hydroxybutyl-1,3-dithiane (Compound 32)

By the same reaction as in Preparation 27 using Compound 24 (215 mg) in place of Compound 26, there is obtained 227 mg of the title Compound 32 as colorless, sticky oil.
SIMS : $(M + H)^+$ Measured : 332.3
Calculated : 332.2

Example 3

(2S)-2-[$N^{\alpha}$-{3-Morpholinocarbonyl-2-(1-naphthylmethyl)propionyl}-L-histidyl]amino-1-cyclohexyl-3-hydroxy-6-methyl-5-heptanone (Compound 3)

By the same reaction as in Example 1 using $N^{\alpha}$-[3-morpholinocarbonyl-2-(1-naphthylmethyl)propionyl]-L-histidinehydrazide (Compound 5, 95.6 mg) and 2-isopropyl-2-[(3S)-3-amino-4-cyclohexyl-2-hydroxybutyl]-1,3-dithiane (Compound 32, 66.3 mg), there is obtained 69 mg of dithianeketal derivative of (2S)-2-[$N^{\alpha}$-{3-morpholinocarbonyl-2-(1-naphthylmethyl)propionyl}-L-histidyl]amino-1-cyclohexyl-3-hydroxy-6-methyl-5-heptanone as colorless semi-solid which is represented by the formula

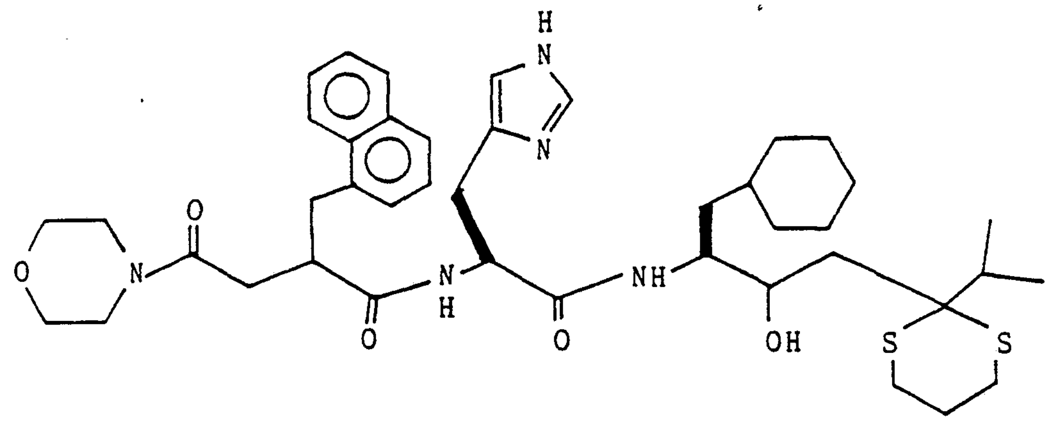

Rf : 0.54 (solvent : chloroform/methanol = 5/1, v/v)

The above-mentioned dithianeketal derivative (31.3 mg) is reacted in the same manner as in Preparation 25 to obtain 16 mg of the title Comound 3.

SIMS : $(M + H)^+$ Measured : 688.3

Calculated 688.4

Preparation 33

(2S)-2-($N^{\alpha}$-tert-Butoxycarbonyl-$N^{\alpha}$-methyl-L-histidyl)amino-1-cyclohexyl-6-methyl-3,5-heptanediol (Compound 33)

By the same reaction as in Example 1 using N-tert-butoxycarbonyl-$N^{\alpha}$-methyl-L-histidinehydrazide (Compound 6, 0.58 g) in place of Compound 5, there is obtained 0.2 g of the title Compound 33 as white powder.

Preparation 34

(2S)-2-($N^{\alpha}$-Methyl-L-histidyl)amino-1-cyclohexyl-6-methyl-3,5-heptanediol (Compound 34)

By the same procedure as in Preparation 31, there is obtained 112 mg of the title Compound 34 as white crystals from Compound 33 (200 mg).

Example 4

(2S)-2-[$N^{\alpha}$-(N-tert-Butoxycarbonyl-L-phenylalanyl)-$N^{\alpha}$-methyl-L-histidyl]amino-1-cyclohexyl-6-methyl-3,5-heptanediol (Compound 4, isomer A)

To N-tert-butoxycarbonyl-L-phenylalanine (22 mg) in N,N-dimethylformamide (0.5 ml) are added 1-hydroxybenzotriazole monohydrate (10 mg) and N,N-dicyclohexylcarbodiimide (20 mg) while stirring under ice-cooling. After stirring under ice-cooling for 1 hour, (2S)-2-($N^{\alpha}$-methyl-L-histidyl)amino-1-cyclohexyl-6-methyl-3,5-heptanediol (Compound 34, 30 mg) in N,N-dimethylformamide (0.5 ml) is added thereto. The reaction mixture is stirred at room temperature for 60 hour, followed by filtration. The filtrate is concentrated under reduced pressure. The residue is dissolved in ethyl acetate (10 ml), washed with a saturated aqueous solution of sodium hydrogencarbonate, and dried over anhydrous magnesium sulfate. The solvent is distilled off under reduced pressure. The residue is purified by preparative thin-layer chromatography (solvent : chloloform/methanol/28% ammonia water = 85/15/1, v/v) to give 19 mg of the title Compound 4 (isomer A) as white powder.
SIMS : $(M + H)^+$ Measured : 642.6
Calculated : 642.4

## Example 5

(2S)-2-[$N^{\alpha}$-(N-tert-Butoxycarbonyl-L-phenylalanyl)-$N^{\alpha}$-methyl-L-histidyl]amino-1-cyclohexyl-6-methyl-3,5-heptanediol (Compound 5, isomer C)

By the same reaction as in Preparation 33 using $N^{\alpha}$-(N-tert-butoxycarbonyl-L-phenylalanyl)-$N^{\alpha}$-methyl-L-histidinehydrazide (Compound 7, 29 mg) and (2S)-2-amino-1-cyclohexyl-6-methyl-3,5-heptanediol (Compound 31, isomer C, 11 mg), there is obtained 10 mg of the title Compound 5 (isomer C) as white powder.

## Example 6

(2S)-2-[$N^{\alpha}$-(N-Morpholinocarbonyl-L-phenylalanyl)-$N^{\alpha}$-methyl-L-histidyl]amino-1-cyclohexyl-6-methyl-3,5-heptanediol (Compound 6)

By the same reaction as in Example 4 using N-morpholinocarbonyl-L-phenylalanine (Compound 8, 16 mg) in place of N-tert-butoxycarbonyl-L-phenylalanine, there is obtained 13 mg of the title Compound 6 as white powder.
SIMS : $(M + H)^+$ Measured : 655.1
Calculated : 655.4

## Example 7

(2S)-2-[$N^{\alpha}$-{(2S)-2-Morpholinocarbonyloxy-3-phenylpropionyl}-$N^{\alpha}$-methyl-L-histidyl]amino-1-cyclohexyl-6-methyl-3,5-heptanediol (Compound 7)

In the same manner as in Example 4 using (2S)-2-morpholinocarbonyloxy-3-phenylpropionic acid (Compound 9, 16 mg) in place of N-tert-butoxycarbonyl-L-phenylalanine,there is obtained 13 mg of the title Compound 7 as white powder.
SIMS : $(M + H)^+$ Measured : 656.2

Calculated : 656.4

Example 8

(2S)-2-[$N^{\alpha}$-{(2S)-2-Benzyl-3-tert-butylsulfonylpropionyl}-$N^{\alpha}$-methyl-L-histidyl]amino-1-cyclohexyl-6-methyl-3,5-heptanediol (Compound 8)

In the same manner as in Example 4 using (2S)-2-benzyl-3-tert-butylsulfonylpropionic acid (Compound 11, 19 mg) in place of N-tert-butoxycarbonyl-L-phenylalanine, there is obtained 4 mg of the title Compound 8 as white powder.
SIMS : $(M + H)^+$ Measured : 661.4
Calculated : 661.4

Example 9

(2S)-2-[$N^{\alpha}$-{3-Morpholinocarbonyl-2-(1-naphthylmethyl)propionyl}-$N^{\alpha}$-methyl-L-histidyl]amino-1-cyclohexyl-6-methyl-3,5-heptanediol (Compound 9, isomer A and isomer B)

By the same reaction as in Example 4 using 3-morpholinocarbonyl-2-(1-naphthylmethyl)propionic acid (Compound 3, 32 mg) in place of N-tert-butoxycarbonyl-L-phenylalanine, there are obtained 0.8 mg (isomer A) and 1.3 mg (isomer B) of the title Compound 9.
Isomer A : white powder
SIMS : $(M + H)^+$ Measured : 704.2
Calculated : 704.4
Isomer B : white powder
SIMS : $(M + H)^+$ Measured : 704.2
Calculated : 704.4

Example 10

(2S)-2-($N^{\alpha}$-L-Phenylalanyl-$N^{\alpha}$-methyl-L-histidyl)amino-1-cyclohexyl-6-methyl-3,5-heptanediol (Compound 10)

By reacting (2S)-2-[$N^{\alpha}$-(N-tert-butoxycarbonyl-L-phenylalanyl)-$N^{\alpha}$-methyl-L-histidyl]amino-1-cyclohexyl-6-methyl-3,5-heptanediol (Compound 4, isomer A, 220 mg) in the same manner as in Preparation 31, there is obtained 150 mg of the title Compound 10 as white powder.
SIMS : $(M + H)^+$ Measured : 542.7
Calculated : 542.4

Example 11

(2S)-2-[$N^{\alpha}$-{N-(2-tert-Butoxycarbonylamino-2-methylpropionyl)-L-phenylalanyl}-$N^{\alpha}$-methyl-L-histidyl]amino-1-cyclohexyl-6-methyl-3,5-heptanediol (Compound 11)

By the preparation method in the literature [N. Izumiya, T. Kato, H. Aoyanagi and M. Waki, PEPUCHIDO GOSE NO KISO TO JIKKEN (Fundamentals and Experiments of Peptide Synthesis), Maruzen (1985)], 2-tert-butoxycarbonylamino-2-methyl propionic acid is obtained from 2-amino-isobutyric acid. The thus-obtained propionic acid is reacted with Compound 10 in the same manner as in Example 4 to give 36 mg of the title Compound 11 as white powder.
SIMS : $(M + H)^+$ Measured : 727.5
Calculated : 727.5

Example 12

(2S)-2-[$N^{\alpha}$-(N-tert-Butoxycarbonyl-O-methyl-L-tyrosyl)-$N^{\alpha}$-methyl-L-histidyl]amino-1-cyclohexyl-6-methyl-3,5-heptanediol (Compound 12)

By the same reaction as in Example 4 using N-tert-butoxycarbonyl-O-methyl-L-tyrosine (Compound 12, 155 mg) in place of N-tert-butoxycarbonyl-L-phenylalanine, there is obtained 244 mg of the title Compound 12 as white powder.
SIMS : $(M + H)^+$ Measured : 672.4
Calculated : 672.4

Example 13

(2S)-2-[$N^{\alpha}$-(O-methyl-L-tyrosyl)-$N^{\alpha}$-methyl-L-histidyl]amino-1-cyclohexyl-6-methyl-3,5-heptanediol (Compound 13)

By the same reaction as in Example 10 using (2S)-2-[$N^{\alpha}$-(N-tert-butoxycarbonyl-O-methyl-L-tyrosyl)-$N^{\alpha}$-methyl-L-histidyl]amino-1-cyclohexyl-6-methyl-3,5-heptanediol (Compound 12, 200 mg) in place of Compound 4, there is obtained 115 mg of the title Compound 13 as white powder.
SIMS : $(M + H)^+$ Measured : 572.3
Calculated : 572.4

Example 14

(2S)-2-[$N^{\alpha}$-{N-(3-Benzyloxycarbonylamino-3-methylbutyryl)-O-methyl-L-tyrosyl}-$N^{\alpha}$-methyl-L-histidyl]amino-1-cyclohexyl-6-methyl-3,5-heptanediol (Compound 14)

By the same reaction as in Example 11 using 3-benzyloxycarbonylamino-3-methylbutyric acid (Compound 13, 48 mg) and (2S)-2-[$N^{\alpha}$-(O-methyl-L-tyrosyl)-$N^{\alpha}$-methyl-L-histidyl]amino-1-cyclohexyl-6-methyl-3,5-heptanediol (Compound 13, 100 mg), there is obtained 91 mg of the title Compound 14 as white powder.
SIMS : $(M + H)^+$ Measured : 805.2
Calculated : 805.5

Example 15

(2S)-2-[$N^{\alpha}$-{N-(3-Amino-3-methylbutyryl)-O-methyl-L-tyrosyl}-$N^{\alpha}$-methyl-L-histidyl]amino-1-cyclohexyl-6-methyl-3,5-heptanediol (Compound 15)

Compound 14 (70 mg) as obtained in Example 14 is dissolved in acetic acid (13 ml), and 10%

palladium-carbon (15 mg) is added thereto, followed by hydrogenation under atmospheric pressure for 2 hours. After the catalyst is filtered off, the filtrate is concentrated under reduced pressure. To the residue is added a saturated aqueous solution of sodium hydrogencarbonate (10 ml) and the mixture is extracted with chloroform (15 ml x 2 times). The thus-obtained chloroform solution is dried over anhydrous magnesium sulfate, and the solvent is distilled off under reduced pressure to give 52 mg of the title Compound 15 as white powder.
SIMS : $(M + H)^+$ Measured : 671.5
Calculated : 671.5

## Example 16

(2S)-2-[$N^{\alpha}$-{N-(4-Hydroxypiperidino)carbonyl-L-phenylalanyl}-$N^{\alpha}$-methyl-L-histidyl]amino-1-cyclohexyl-6-methyl-3,5- heptanediol (Compound 16)

By the same reaction as in Example 4 using N-(4-hydroxypiperidino)carbonyl-L-phenylalanine (Compound 14, 25 mg) in place of N-tert-butoxycarbonyl-L-phenylalanine, there is obtained 33 mg of the title Compound 16 as white powder.
SIMS : $(M + H)^+$ Measured : 669.4
Calculated : 669.4

## Example 17

(2S)-2-[$N^{\alpha}$-[N-{3-(3-Pyridyl)propionyl}-L-phenylalanyl]-$N^{\alpha}$-methyl-L-histidyl]amino-1-cyclohexyl-6-methyl-3,5-heptanediol (Compound 17)

By the same reaction as in Example 11 using 3-(3-pyridyl)propionic acid (Compound 15, 15 mg) in place of 2-tert-butoxycarbonylamino-2-methylpropionic acid, there is obtained 27 mg of the title Compound 17 as white powder.
SIMS : $(M + H)^+$ Measured : 675.4
Calculated : 675.4

## Example 18

(2S)-2-[$N^{\alpha}$-{N-(2-Amino-2-methylpropionyl)-L-phenylalanyl]-$N^{\alpha}$-methyl-L-histidyl]amino-1-cyclohexyl-6-methyl-3,5-heptanediol (Compound 18)

By the same reaction as in Example 10, the protective group of Compound 11 (17 mg) is removed to afford 6 mg of the title Compound 18 as white powder.
SIMS : $(M + H)^+$ Measured : 627.5
Calculated : 627.4

## Example 19

(2S)-2-[$N^{\alpha}$-{(2S)-2-(N-tert-Butoxycarbonyl-L-proryl)oxy-3-phenylpropionyl}-$N^{\alpha}$-methyl-L-histidyl]amino-1-cyclohexyl-6-methyl-3,5-heptanediol (Compound 19)

By the same reaction as in Example 4 using (2S)-2-(N-tert-butoxycarbonyl-L-prolyl)oxy-3-phenylpropionic acid (Compound 16, 100 mg) in place of N-tert-butoxycarbonyl-L-phenylalanine, there is obtained 117 mg of the title Compound 19 as white powder.
SIMS : $(M + H)^+$ Measured : 740.3
Calculated : 740.5

Example 20

(2S)-2-[$N^\alpha$-(N-Piperidinocarbonyl-L-phenylalanyl)-$N^\alpha$-methyl-L-histidyl]amino-1-cyclohexyl-6-methyl-3,5-heptanediol (Compound 20)

By the same reaction as in Example 4 using N-piperidinocarbonyl-L-phenylalanine (Compound 17, 20 mg) in place of N-tert-butoxycarbonyl-L-phenylalanine, there is obtained 15 mg of the title Compound 20 as white powder.
SIMS : $(M + H)^+$ Measured : 653.6
Calculated : 653.4

Example 21

(2S)-2-[$N^\alpha$-(2-Benzyl-3-tert-butylsulfonylpropionyl)-L-histidyl]amino-1-cyclohexyl-6-methyl-3,5-heptanediol (Compound 21)

By the same reaction as in Example 1 using $N^\alpha$-(2-benzyl- 3-tert-butylsulfonylpropionyl)-L-histidinehydrazide (Compound 18, isomer A, 55 mg) and (2S)-2-amino-1-cyclohexyl-6-methyl-3,5-heptanediol (Compound 27, isomer A, 31 mg), there is obtained 40 mg of the title Compound 21 as white powder.
SIMS : $(M + H)^+$ Measured : 647.4
Calculated : 647.4

Example 22

(2S)-2-[$N^\alpha$-[N-{1-(4-Benzyloxycarbonyl)piperazinylcarbonyl}-L-phenylalanyl]-$N^\alpha$-methyl-L-histidyl]amino-1-cyclohexyl-6-methyl-3,5-heptanediol (Compound 22)

By the same reaction as in Example 4 using N-[1-(4-benzyloxycarbonyl)piperazinylcarbonyl]-L-phenylalanine (Compound 19, 66 mg) in place of N-tert-butoxycarbony-L-phenylalanine, there is obtained 65 mg of the title Compound 22 as white powder.
SIMS : $(M + H)^+$ Measured : 788.4
Calculated : 788.5

Example 23

(2S)-2-[$N^\alpha$-{N-(1-Piperazinylcarbonyl)-L-phenylalanyl}-$N^\alpha$-methyl-L-histidyl]amino-1-cyclohexyl-6-methyl-3,5-heptanediol (Compound 23)

Compound 22 as obtained in Example 22 (36 mg) is dissolved in methanol (4 ml) and water (1 ml), and

thereto is added 10% palladium-carbon (6 mg), followed by hydrogenation under atmospheric pressure for 15 hours. After the catalyst is filtered off, the solvent is distilled off under reduced pressure. The residue is purified by preparative thin-layer chromatography to give 17 mg of the title Compound 23 as white powder.
SIMS : $(M + H)^+$ Measured : 654.3
Calculated : 654.4

## Example 24

(2S)-2-[$N^\alpha$-{(2S)-2-(2,2-Dimethylpropionyl)oxy-3-phenylpropionyl}-$N^\alpha$-methyl-L-histidyl]amino-1-cyclohexyl-6-methyl-3,5-heptanediol (Compound 24)

By the same reaction as in Example 4 using (2S)-2-(2,2-dimethylpropionyl)oxy-3-phenylpropionic acid (Compound 20, 21 mg) in place of N-tert-butoxycarbonyl-L-phenylalanine, there is obtained 21 mg of the title Compound 24 as white powder.
SIMS : $(M + H)^+$ Measured : 627.4
Calculated : 627.4

## Example 25

(2S)-2-[$N^\alpha$-[N-{(Tetrahydro-4H-1,4-thiazine)-4-yl-carbonyl}-L-phenylalanyl]-$N^\alpha$-methyl-L-histidyl]amino-1-cyclohexyl-6-methyl-3,5-heptanediol (Compound 25)

By the same reaction as in Example 4 using N-[(tetrahydro-4H-1,4-thiazine)-4-yl-carbonyl]-L-phenylalanine (Compound 21, 25 mg) in place of N-tert-butoxycarbonyl-L-phenylalanine, there is obtained 30 mg of the title Compound 25 as white powder.
SIMS : $(M + H)^+$ Measured : 671.6
Calculated : 671.4

## Preparation 35

(2S)-2-($N^\alpha$-Benzyloxycarbonyl-$N^\alpha$-methyl-L-histidyl)amino-1-cyclohexyl-6-methyl-3,5-heptanediol (Compound 35)

By the same reaction as in Preparation 33 using $N^\alpha$-benzyloxycarbonyl-$N^\alpha$-methyl-L-histidinehydrazide (1.56 g) obtained from $N^\alpha$-benzyloxycarbonyl-$N^{im}$-tosyl-L-histidine by the method of Preparation 6, there is obtained 1.45 g of the title Compound 35 as white powder.

## Preparation 36

(2S)-2-($N^\alpha$-tert-Butoxycarbonyl-L-histidyl)amino-1-cyclohexyl-6-methyl-3,5-heptanediol (Compound 36)

To $N^\alpha$-tert-butoxycarbonyl-L-histidine (210 mg) and (2S)-2-amino-1-cyclohexyl-6-methyl-3,5-heptanediol (Compound 27, isomer A, 200 mg) in N,N-dimethylformamide (7 ml) is added a solution of triethylamine (83 mg) and diphenylphosphoryl azide (226 mg) in N,N-dimethylformamide (2 ml) while stirring under ice-cooling. After stirring under ice-cooling for 1 hour and at room temperature for 15 hours, the reaction mixture is concentrated under reduced pressure, and the residue is dissolved in chloroform (15 ml). The

chloroform solution is washed with a saturated aqueous solution of sodium hydrogencarbonate, and dried over anhydrous magnesium sulfate. The solvent is distilled off under reduced pressure and the residue is purified by silica gel column chromatography (eluent : chloroform/methanol = 20/1, v/v) to give 278 mg of the title Compound 36 as white powder.

Preparation 37

(2S)-2-(L-Histidyl)amino-1-cyclohexyl-6-methyl-3,5-heptanediol (Compound 37)

By the same reaction as in Preparation 34 using (2S)-2-($N^{\alpha}$-tert-butoxycarbonyl-L-histidyl)amino-1-cyclohexyl-6-methyl-3,5-heptanediol (Compound 36, 278 mg), there is obtained 163 mg of the title Compound 37 as white powder.

Example 26

(2S)-2-[$N^{\alpha}$-{N-(3-Benzyloxycarbonylamino-3-methylbutyryl)-O-methyl-L-tyrosyl}-L-histidyl]amino-1-cyclohexyl-6-methyl-3,5-heptanediol (Compound 26)

By reacting N-(3-benzyloxycarbonylamino-3-methylbutyryl)-O-methyl-L-tyrosine (Published Japanese Translations of PCT Patent Applications from Other Countries (Tokuhyo) No. 502514/1989, 62 mg) and (2S)-2-(L-histidyl)amino-1-cyclohexyl-6-methyl-3,5-heptanediol (Compound 37, 50 mg) by the method of Example 4, there is obtained 60 mg of the title Compound 26 as white powder.

Example 27

(2S)-2-[$N^{\alpha}$-{N-(3-Benzyloxycarbonylamino-3-methylbutyryl)-L-phenylalanyl}-$N^{\alpha}$-methyl-L-histidyl]amino-1-cyclohexyl-6-methyl-3,5-heptanediol (Compound 27)

By the same reaction as in Example 11 using 3-benzyloxycarbonylamino-3-methylbutyric acid (Compound 13, 31 mg) in place of 2-tert-butoxycarbonylamino-2-methylpropionic acid, there is obtained 57 mg of the title Compound 27 as white powder.

Example 28

(2S)-2-[$N^{\alpha}$-{N-(3-Amino-3-methylbutyryl)-O-methyl-L-tyrosyl}-L-histidyl]amino-1-cyclohexyl-6-methyl-3,5-heptanediol (Compound 28)

By the same reaction as in Example 15 using (2S)-2-[$N^{\alpha}$-{N-(3-benzyloxycarbonylamino-3-methylbutyryl)-O-methyl-L-tyrosyl}-L-histidyl]amino-1-cyclohexyl-6-methyl-3,5-heptanediol (Compound 26, 60 mg), there is obtained 40 mg of the title Compound 28 as white powder.
SIMS : $(M + H)^{+}$ Measured : 657.4
Calculated : 657.4

Example 29

(2S)-2-[$N^{\alpha}$-{N-(3-Amino-3-methylbutyryl)-L-phenylalanyl}-$N^{\alpha}$-methyl-L-histidyl]amino-1-cyclohexyl-6-methyl-3,5-heptanediol (Compound 29)

By the same reaction as in Example 15 using (2S)-2-[$N^{\alpha}$-{N-(3-benzyloxycarbonylamino-3-methylbutyryl)-L-phenylalanyl}-$N^{\alpha}$-methyl-L-histidyl]amino-1-cyclohexyl-6-methyl-3,5-heptanediol (Compound 27, 57 mg), there is obtained 38 mg of the title Compound 29 as white powder.
SIMS : $(M + H)^+$ Measured : 641.3
Calculated : 641.3

Example 30

(2S)-2-[$N^{\alpha}$-{N-(3-Amino-3-methylbutyryl)-O-methyl-L-tyrosyl}- L-histidyl]amino-1-cyclohexyl-6-methyl-3,5-heptanediol diacetate (Compound 30)

After (2S)-2-[$N^{\alpha}$-{N-(3-amino-3-methylbutyryl)-O-methyl-L-tyrosyl}-L-histidyl]amino-1-cyclohexyl-6-methyl-3,5-heptanediol (Compound 28, 21 mg) is dissolved in acetic acid (0.5 ml), the solution is concentrated under reduced pressure. The residue is dissolved in water (0.5 ml), and the obtained solution is lyophilized to afford 19 mg of the title Compound 30 as white powder.

Example 31

(2S)-2-[$N^{\alpha}$-{N-(3-Amino-3-methylbutyryl)-L-phenylalanyl}-$N^{\alpha}$-methyl-L-histidyl]amino-1-cyclohexyl-6-methyl-3,5-heptanediol diacetate (Compound 31)

By treating (2S)-2-[$N^{\alpha}$-{N-(3-amino-3-methylbutyryl)-L-phenylalanyl}-$N^{\alpha}$-methyl-L-histidyl]amino-1-cyclohexyl-6-methyl-3,5-heptanediol (Compound 29, 15 mg) by the method of Example 30, there is obtained 14 mg of the title Compound 31 as white powder.

Example 32

(2S)-2-[$N^{\alpha}$-{(2S)-2-Hydroxy-3-phenylpropionyl}-$N^{\alpha}$-methyl-L-histidyl]amino-1-cyclohexyl-6-methyl-3,5-heptanediol (Compound 32)

By the same reaction as in Example 4 using (2S)-2-hydroxy-3-phenylpropionic acid (see Preparation 9, 14 mg) in place of N-tert-butoxycarbonyl-L-phenylalanine, there is obtained 13 mg of the title Compound 32 as white powder.
SIMS : $(M + H)^+$ Measured : 543.3
Calculated : 543.4

Example 33

(2S)-2-[$N^{\alpha}$-{N-(2-Hydroxyethyl)-N-methylaminocarbonyl-L-phenylalanyl}-$N^{\alpha}$-methyl-L-histidyl]amino-1-cyclohexyl-6-methyl-3,5-heptanediol (Compound 33)

By the same reaction as in Example 4 using N-[N-(2-hydroxyethyl)-N-methylaminocarbonyl]-L-phenylalanine [Journal of Medicinal Chemistry, 31, 2277 - 2288 (1988), 22 mg] in place of N-tert-butoxycarbonyl-L-phenylalanine, there is obtained 20 mg of the title Compound 33 as white powder.
SIMS : $(M + H)^+$ Measured : 643.7
Calculated : 643.4

Example 34

(2S)-2-[$N^{\alpha}$-{(2S)-2-(3,5-Dioxomorpholino)-3-phenylpropionyl}-$N^{\alpha}$-methyl-L-histidyl]amino-1-cyclohexyl-6-methyl-3,5-heptanediol (Compound 34)

By the same reaction as in Example 4 using (2S)-2-(3,5-dioxomorpholino)-3-phenylpropionic acid [Unexamined Japanese Patent Publication (Kokai) No. 183551/1988, 44 mg] in place of N-tert-butoxycarbonyl-L-phenylalanine, there is obtained 13 mg of the title Compound 34 as white powder.
SIMS : $(M + H)^+$ Measured : 640.6
Calculated : 640.4

Example 35

(2S)-2-[$N^{\alpha}$-(N-Carboxymethoxymethylcarbonyl-L-phenylalanyl)-$N^{\alpha}$-methyl-L-histidyl]amino-1-cyclohexyl-6-methyl-3,5-heptanediol trifluoroacetate (Compound 35)

By the same reaction as in Example 4 using N-tert-butoxycarbonylmethoxymethylcarbonyl-L-phenylalanine (Compound 22, 38 mg) in place of N-tert-butoxycarbonyl-L-phenylalanine, there is obtained 43 mg of (2S)-2-[$N^{\alpha}$-(N-tert-butoxycarbonylmethoxymethylcarbonyl-L-phenylalanyl)-$N^{\alpha}$-methyl-L-histidyl]-amino-1-cyclohexyl-6-methyl-3,5-heptanediol as colorless, sticky oil. This compound (13 mg) is dissolved in trifluoroacetic acid (0.1 ml) and kept standing at room temperature for 45 minutes. The solvent is distilled off under reduced pressure to give 11 mg of the title Compound 35 as white powder.
SIMS : $(M + H)^+$ Measured : 658.6 (free base)
Calculated : 658.4 (free base)

Example 36

(2S)-2-[$N^{\alpha}$-[N-{(1,1-Dioxo-2,3,5,6-tetrahydro-4H-1,4-thiazine)-4-yl-carbonyl}-L-phenylalanyl]-$N^{\alpha}$-methyl-L-histidyl]amino-1-cyclohexyl-6-methyl-3,5-heptanediol (Compound 36)

By the same reaction as in Example 4 using N-[(1,1-dioxo-2,3,5,6-tetrahydro-4H-1,4-thiazine)-4-yl-carbonyl]-L-phenylalanine [Unexamined Japanese Patent Publication (Kokai) No. 183551/1988, 27 mg] in place of N-tert-butoxycarbonyl-L-phenylalanine, there is obtained 24 mg of the title Compound 36 as white powder.
SIMS : $(M + H)^+$ Measured : 703.5
Calculated : 703.4

Example 37

(2S)-2-[$N^{\alpha}$-[N-{N-(2-Methoxyethoxymethoxyethyl)-N-methylaminocarbonyl}-L-phenylalanyl]-$N^{\alpha}$-methyl-L-histidyl]amino-1-cyclohexyl-6-methyl-3,5-heptanediol (Compound 37)

By the same reaction as in Example 4 using N-[N-(2-methoxyethoxymethoxyethyl)-N-methylaminocarbonyl]-L-phenylalanine (Compound 23, 30 mg) in place of N-tert-butoxycarbonyl-L-phenylalanine, there is obtained 12 mg of the title Compound 37 as white powder.
SIMS : $(M + H)^+$ Measured : 731.2
Calculated : 731.5

## Example 38

(2S)-2-[$N^{\alpha}$-[N-{(1-Oxo-2,3,5,6-tetrahydro-4H-1,4-thiazine)-4-yl-carbonyl}-L-phenylalanyl]-$N^{\alpha}$-methyl-L-histidyl]amino-1-cyclohexyl-6-methyl-3,5-heptanediol (Compound 38)

Compound 25 (15 mg) as obtained in Example 25 is dissolved in dichloromethane (0.5 ml), and thereto is added m-chloroperbenzoic acid (4.6 mg) while stirring under ice-cooling. After stirring under ice-cooling for 10 minutes, ethyl acetate (2.5 ml) is added to the reaction mixture, followed by washing with an aqueous solution of 10% sodium sulfite. The ethyl acetate solution is dried over anhydrous magnesium sulfate, and the solvent is distilled off under reduced pressure. The residue is purified by preparative thin-layer chromatography (solvent : chloroform/methanol = 5/1, v/v) to give 3.5 mg of the title Compound 38 as white powder.
SIMS : $(M + H)^+$ Measured : 687.6
Calculated : 687.4

## Preparation 38

(6S)-6-(N-tert-Butoxycarbonyl)amino-7-cyclohexyl-2,2-dimethyl-5-hydroxy-3-heptanone (Compound 38)

Diisopropylamine (2.3 ml) is dissolved in dry tetrahydrofuran (30 ml) under an argon atmosphere, and thereto is dropwise added an n-butyllithium hexane solution (1.57 M, 10.5 ml) over a period of 20 minutes while stirring under cooling at -30 °C. The mixture is stirred for 1 hour, during which stirring the temperature is maintained at -30 °C. The reaction mixture is cooled to -70 °C, and thereto is dropwise added a pinacoline (1.65 g) - dry tetrahydrofuran (30 ml) solution over a period of 20 minutes while stirring. The temperature of the reaction mixture is gradually raised to 0 °C, and thereafter again lowered to -70 °C. Thereto is dropwise added a (2S)-2-(N-tert-butoxycarbonyl)amino-3-cyclohexylpropanal (2.80 g) - dry tetrahydrofuran (30 ml) solution over a period of 20 minutes while stirring. The mixture is stirred at -70 °C for 30 minutes, and added to an ice-cooled saturated aqueous solution of sodium hydrogencarbonate (100 ml), followed by extraction with diethyl ether. The diethyl ether solution is washed twice with saturated brine (150 ml), and dried over anhydrous magnesium sulfate. The solvent is distilled off and the residue is purified by silica gel column chromatography (eluent : ethyl acetate/hexane = 1/9, v/v) to give the unpolar isomer A (630 mg) and the polar isomer B (465 mg) of the title Compound 38 as crystalline solid.
Unpolar isomer A : pale-yellow crystalline solid
Polar isomer B : pale-yellow crystalline solid

## Preparation 39

(2S)-2-(N-tert-Butoxycarbonyl)amino-1-cyclohexyl-6,6-dimethyl-3,5-heptanediol (Compound 39)

By the same procedure as in Preparation 30, the unpolar isomer A (630 mg) of Compound 38 as obtained in Preparation 38 is reduced. The product is purified by preparative thin-layer chromatography to give 180 mg of the unpolar isomer C and 120 mg of the polar isomer D of the title Compound 39.
Unpolar isomer C : pale-yellow sticky oil

Polar isomer D : pale-yellow powdery solid

Preparation 40

(2S)-2-Amino-1-cyclohexyl-6,6-dimethyl-3,5-heptanediol (Compound 40, isomer C)

In the same manner as in Preparation 31, there is obtained 35 mg of the title Compound 40 as colorless, sticky oil from Compound 39 (unpolar isomer C, 65 mg).

Example 39

(2S)-2-[$N^{\alpha}$-{3-Morpholinocarbonyl-2-(1-naphthylmethyl) propionyl}-L-histidyl]amino-1-cyclohexyl-6,6-dimethyl-3,5-heptanediol (Compound 39, isomer C)

By the same reaction as in Example 1 using $N^{\alpha}$-[3-morpholinocarbonyl-2-(1-naphthylmethyl)propionyl]-L-histidinehydrazine (Compound 5, 79 mg) and (2S)-2-amino-1-cyclohexyl-6,6-dimethyl-3,5-heptanediol (Compound 40, isomer C, 35 mg), there is obtained 78 mg of the title Compound 39 as white powder.
SIMS : $(M + H)^+$ Measured : 704.3
Calculated : 704.4

Preparation 41

(2S)-2-Amino-1-cyclohexyl-6,6-dimethyl-3,5-heptanediol (Compound 41, isomer D)

By the same reaction as in Preparation 40 using Compound 39 (polar isomer D, 60 mg) as obtained in Preparation 39, there is obtained 25 mg of the title Compound 41 as white powder.

Example 40

(2S)-2-[$N^{\alpha}$-{3-Morpholinocarbonyl-2-(1-naphthyl)propionyl}-L-histidyl]amino-1-cyclohexyl-6,6-dimethyl-3,5-heptanediol (Compound 40, isomer D)

By the same reaction as in Example 39 using (2S)-2-amino-1-cyclohexyl-6,6-dimethyl-3,5-heptanediol (Compound 41, isomer D, 25 mg) in place of compound 40 (isomer C), there is obtained 45 mg of the title Compound 40 as white powder.
SIMS : $(M + H)^+$ Measured : 704.6
Calculated : 704.4

Preparation 42

(6S)-6-(N-tert-Butoxycarbonyl)amino-7-cyclohexyl-5-hydroxy-3-heptanone (Compound 42)

By the same reaction as in Preparation 38 using methyl ethyl ketone (1.77 ml) in place of pinacoline,

there is obtained 1.4 g of the unpolar isomer A as pale-yellow, sticky oil and 2.8 g of the polar isomer B as white solid, of the title Compound 42.
Unpolar isomer A : pale-yellow, sticky oil
Polar isomer B : white solid

Preparation 43

(2S)-2-(N-tert-Butoxycarbonyl)amino-1-cyclohexyl-3,5-heptanediol (Compound 43)

By the same reaction as in Preparation 39 using (6S)-6-(N-tert-butoxycarbonyl)amino-7-cyclohexyl-5-hydroxy-3-heptanone (Compound 42, unpolar isomer A, 400 mg) in place of Compound 38 (unpolar isomer A) used in Preparation 39, there is obtained 180 mg of the unpolar isomer C and 100 mg of the polar isomer D, of the title Compound 43.
Unpolar isomer C : pale-yellow oil
Polar isomer D : pale-yellow crystals

Preparation 44

(2S)-2-Amino-1-cyclohexyl-3,5-heptanediol (Compound 44, isomer C)

By the same reaction as in Preparation 40 using Compound 43 (unpolar isomer C, 60 mg), there is obtained 26 mg of the title Compound 44.
Property : pale-yellow oil

Example 41

(2S)-2-[$N^{\alpha}$-{3-Morpholinocarbonyl-2-(1-naphthylmethyl)propionyl}-L-histidyl]amino-1-cyclohexyl-3,5-heptanediol (Compound 41, isomer C)

By the same reaction as in Example 39 using (2S)-2-amino-1-cyclohexyl-3,5-heptanediol (Compound 44, isomer C, 60 mg) in place of Compound 40 (isomer C), there is obtained 26 mg of the title Compound 41.
Property : pale-yellow powdery solid
SIMS : $(M + H)^{+}$ Measured : 676.4
Calculated : 676.4

Preparation 45

(2S)-2-Amino-1-cyclohexyl-3,5-heptanediol (Compound 45, isomer D)

By the same reaction as in Preparation 40 using (2S)-2-(N-tert-butoxycarbonyl)amino-1-cyclohexyl-3,5-heptanediol (Compound 43, polar isomer D, 74 mg) in place of Compound 39 (unpolar isomer C), there is obtained 30 mg of the title Compound 45 as white powder.

Example 42

(2S)-2-[$N^{\alpha}$-{3-Morpholinocarbonyl-2-(1-naphthylmethyl)propionyl}-L-histidyl]amino-1-cyclohexyl-3,5-heptanediol (Compound 42, isomer D)

By the same reaction as in Example 39 using (2S)-2- amino-1-cyclohexyl-3,5-heptanediol (Compound 45, isomer D, 26 mg) in place of (2S)-2-amino-1-cyclohexyl-6,6-dimethyl-3,5-heptanediol (Compound 40, isomer C), there is obtained 52 mg of the title Compound 42 as white powder.
Property : white powdery solid
SIMS : $(M + H)^+$ Measured 676.3
Calculated : 676.4

## Preparation 46

N-{4-(N-tert-Butoxycarbonyl-L-alanyl)oxypiperidino}carbonyl-L-phenylalanine (Compound 46)

By the same reaction as in Preparation 16 using N-(4-hydroxypiperidino)carbonyl-L-phenylalanine benzyl ester (Preparation 14, 1.0 g) and N-tert-butoxycarbonyl-L-alanine (520 mg), there is obtained 1.49 g of N-{4-(N-tert-butoxycarbonyl-L-alanyl)oxypiperidino}carbonyl-L-phenylalanine benzyl ester as colorless oil. The obtained ester (1.25 g) is hydrogenated to afford 1.25 g of the title Compound 46 as white powder.

## Preparation 47

N-(4-Diphenylphosphonooxypiperidino)carbonyl-L-phenylalanine (Compound 47)

To N-(4-hydroxypiperidino)carbonyl-L-phenylalanine benzyl ester (Preparation 14, 438 mg) in methylene chloride (10 ml) are added triethylamine (0.32 ml) and 4-dimethylaminopiridine (280 ml) under ice-cooling, followed by dropwise addition of diphenylphosphoryl chloride (0.48 ml) over a period of 5 minutes. After stirring at 0°C for 1 hour, thereto is added an aqueous solution of 0.5 M citric acid. The mixture is extracted with diethyl ether, and the organic layer is washed with a saturated aqueous solution of sodium hydrogencarbonate and saturated brine, followed by drying over anhydrous magnesium sulfate. The solvent is distilled off under reduced pressure, and the residue is purified by preparative thin-layer chromatography (solvent : chloroform/methanol = 95/5, v/v) to give 418 mg of N-(4-diphenylphosphonooxypiperidino)-carbonyl-L-phenylalanine benzyl ester as colorless oil. The obtained ester (93 mg) is hydrogenated with 10% palladium carbon to afford 79 mg of the title Compound 47 as colorless oil.

## Preparation 48

N-[4-(3-tert-Butoxycarbonylpropionyl)oxypiperidino]carbonyl-L-phenylalanine (Compound 48)

In accordance with the method in the literature [Angewandte Chemie International Edition in English, Vol. 17, pp. 569 - 583 (1978)], there is obtained succinic acid monobenzyl ester (30.0 g) from anhydrous succinic acid (15.2 g) and benzyl alcohol (15.0 ml). By the same reaction as in Preparation 16, there is obtained 5.14 g of 3-tert-butoxycarbonylpropionic acid benzyl ester as colorless liquid from succinic acid monobenzyl ester (5.0 g) and tert-butyl alcohol (2.7 ml). The obtained ester (4.36 g) is hydrogenated to afford 3.0 g of succinic acid mono-tert-butyl ester as color less liquid. Thereafter, the same reaction as in Preparation 16 is conducted to afford 273 mg of N-{4-(3-tert-butoxycarbonylpropionyl)-oxypiperidino}carbonyl-L-phenylalanine benzyl ester as colorless oil from succinic acid mono-tert-butyl

ester (100 mg) and N-(4-hydroxypiperidino)carbonyl-L-phenylalanine benzyl ester (Preparation 14, 200 mg). This ester (240 mg) is hydrogenated to afford 193 mg of the title Compound 48 as colorless oil.

Example 43

(2S)-2-[N$^{\alpha}$-{N-(4-L-Alanyloxypiperidino)carbonyl-L-phenylalanyl}-N$^{\alpha}$-methyl-L-histidyl]amino-1-cyclohexyl-6-methyl-3,5-heptanediol ditrifluoroacetate (Compound 43)

By the same reaction as in Example 4 using Compound 46 (93 mg) obtained in Preparation 46 in place of N-tert-butoxycarbonyl-L-phenylalanine in Example 4, there is obtained 118 mg of (2S)-2-[N$^{\alpha}$-[N-{4-(N-tert-butoxycarbonyl-L-alanyl)oxypiperidino}carbonyl-L-phenylalanyl]-N$^{\alpha}$-methyl-L-histidyl]amino-1-cyclohexyl-6-methyl-3,5-heptanediol as white powder. Thereafter, the same reaction as in Example 10 is conducted using the obtained compound to give 70 mg of white powder. The obtained powder (57.8 mg) is dissolved in a small amount of water, and lyophilized to afford 55.1 mg of the title Compound 43 as white powder.
SIMS : $(M + H)^{+}$ Measured : 740.3 (free base)
Calculated : 740.5 (free base)

Example 44

(2S)-2-[N$^{\alpha}$-{N-(4-Phosphonooxypiperidino)carbonyl-L-phenylalanyl}-N$^{\alpha}$-methyl-L-histidyl]amino-1-cyclohexyl-6-methyl-3,5-heptanediol (Compound 44)

By the same reaction as in Example 4 using Compound 47 (76 mg) obtained in Preparation 47 in place of N-tert-butoxycarbonyl-L-phenylalanine in Example 4, there is obtained 80 mg of (2S)-2-[N$^{\alpha}$-{N-(4-diphenylphosphonooxypiperidino)carbonyl-L-phenylalanyl}-N$^{\alpha}$-methyl-L-histidyl]amino-1-cyclohexyl-6-methyl-3,5-heptanediol as white powder. The obtained compound (26 mg) is hydrogenated at 50°C for 14 hours using platinum (IV) oxide in methanol to afford 4 mg of the title Compound 44.

Example 45

(2S)-2-[N$^{\alpha}$-[N-{4-(3-Carboxypropionyl)oxypiperidino}carbonyl-L-phenylalanyl]-N$^{\alpha}$-methyl-L-histidyl]amino-1-cyclohexyl-6-methyl-3,5-heptanediol trifluoroacetate (Compound 45)

By the same reaction as in Example 4 using Compound 48 (112 mg) obtained in Preparation 48 in place of N-tert-butoxycarbonyl-L-phenylalanine in Example 4, there is obtained 87 mg of (2S)-2-[N$^{\alpha}$-[N-{4-(3-tert-butoxycarbonylpropionyl)oxypiperidino}carbonyl-L-phenylalanyl]-N$^{\alpha}$-methyl-L-histidyl]amino-1-cyclohexyl-6-methyl-3,5-heptanediol as white powder. The obtained compound (52 mg) is dissolved in trifluoroacetic acid (3 ml) and the mixture is stirred for 1 hour under ice-cooling. Thereafter, the solvent is distilled off under reduced pressure to afford 52 mg of the title Compound 45 as white powder.

Example 46

(6S)-6-[N$^{\alpha}$-{(2S)-3-Morpholinocarbonyl-2-(1-naphthylmethyl)propionyl}-L-histidyl]amino-5-(3-carboxypropionyl)oxy-7-cyclohexyl-2-methyl-3-heptanol trifluoroacetate (Compound 46)

By the same reaction as in Preparation 16 using succinic acid mono-tert-butyl ester (54 mg) and Compound 1 (90 mg) obtained in Example 1, there is obtained 63 mg of (6S)-6-[$N^{\alpha}$-{3-morpholinocarbonyl-2-(1-naphthylmethyl)propionyl}-L-histidyl]amino-5-(3-tert-butoxycarbonylpropionyl)oxy-7-cyclohexyl-2-methyl-3-heptanol as white powder. The obtained compound (47 mg) is dissolved in trifluoroacetic acid (3 ml) and the mixture is stirred for 1 hour under ice-cooling. Thereafter, the solvent is distilled off under reduced pressure to afford 45 mg of the title Compound 46 as white powder.

## Example 47

(2S)-2-[$N^{\alpha}$-[(2S)-2-[N-Methyl-N-[2-{N-(morpholinocarbonyl)-N-methylamino}ethyl]aminocarbonyloxy]-3-phenylpropionyl]-$N^{\alpha}$-methyl-L-histidyl]amino-1-cyclohexyl-6-methyl-3,5-heptanediol (Compound 47)

By the same reaction as in Example 4 using (2S)-2-[N-methyl-N-[2-{N-(morpholinocarbonyl)-N-methylamino}ethyl]aminocarbonyloxy]-3-phenylpropionic acid (30 mg) in place of N-tert-butoxycarbonyl-L-phenylalanine in Example 4, there is obtained 36 mg of the title Compound 47 as white powder.
SIMS : $(M + H)^+$ Measured : 770.3
Calculated : 770.5

## Example 48

(2S)-2-[$N^{\alpha}$-{N-(3,4-cis-Dihydroxypyrrolidinyl)carbonyl-L-phenylalanyl}-$N^{\alpha}$-methyl-L-histidyl]amino-1-cyclohexyl-6-methyl-3,5-heptanediol (Compound 48)

By the same reaction as in Example 4 using N-(3,4-cis-dihydroxypyrrolidinyl)carbonyl-L-phenylalanine (120 mg) obtained from L-phenylalanine methyl ester by the method in the literature [Unexamined Japanese Patent Publication (Kokai) No. 221357/1989] and Compound 34 (107 mg) obtained in Preparation 34, there is obtained 40 mg of the title Compound 48 as white powder.

The physical properties of the Compounds appeared in Preparations and Examples as detailedly illustrated in the present specification are summarized in the following Table. The abbrebiations in Table have the following meanings:
AcOEt : ethyl acetate
Hex : hexane
$C_6H_6$ : benzene
$CHCl_3$ : chloroform
MeOH : methanol
$NH_3$.aq : 28% ammonia water
Diox : dioxane
AcOH : acetic acid
Boc : tert-butoxycarbonyl
Z : benzyloxycarbonyl

| COMPOUND | STRUCTURAL FORMULA | Rf value, ( ):solvent | NMR ($CDCl_3$)δ: |
|---|---|---|---|
| Preparation 1 (1) | 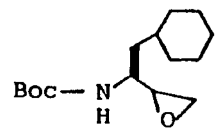 | | 0.8-1.9(m,13H),1.44(s,9H),2.60(m,1H),2.73(m,1H),2.98(m,1H),4.02(m,1H),4.3(m,1H). |
| Preparation 2 (2) | 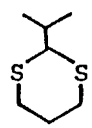 | 0.73 (AcOEt/Hex=25/75 V/V) | |
| Preparation 3 (3) | 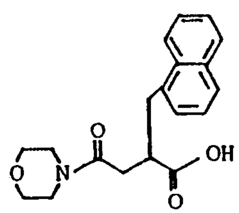 | | 2.35-2.70(m,2H),3.05-3.85(m,11H),7.25-8.15(m,7H). |
| Preparation 4 (4) | 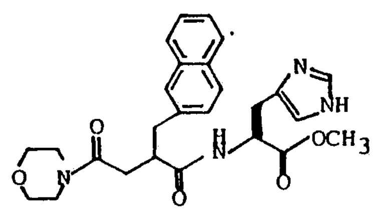 | 0.16 ($CHCl_3$/MeOH/aq.$NH_3$=95/3/0.6 V/V) | |

| COMPOUND | STRUCTURAL FORMULA | Rf value, ( ):solvent | NMR ($CDCl_3$)δ: |
|---|---|---|---|
| Preparation 5 (5) | 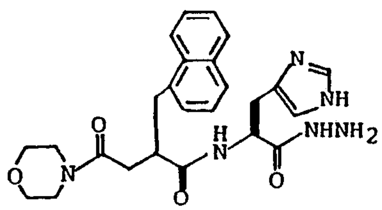 | 0.53 ($CHCl_3$/MeOH/aq.$NH_3$=80/20/1 V/V) | |
| Preparation 6 (6) | 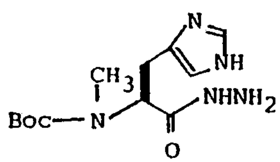 | 0.23 ($CHCl_3$/MeOH=5/1 V/V) | |
| Preparation 7 (7) | 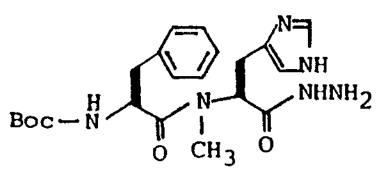 | 0.60 ($CHCl_3$/MeOH=5/1 V/V) | |
| Preparation 8 (8) | 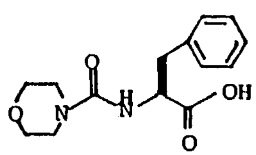 | 0.32 ($C_6H_6$/Diox/AcOH=10/5/1 V/V) | 3.05-3.40(m,6H),3.52-3.75(m,4H),4.67(m,1H),4.94(br d,1H,J=7Hz),7.12-7.4(m,5H). |

| COMPOUND | STRUCTURAL FORMULA | Rf value, ( ):solvent | NMR ($CDCl_3$)δ: |
|---|---|---|---|
| Preparation 9 (9) | 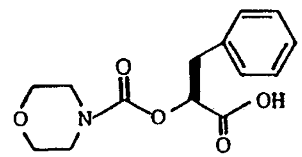 | 0.42 ($C_6H_6$/Diox/AcOH=10/5/1 V/V) | 3.02-3.80(m,10H),5.2 (dd,1H,J=4.14,9.02Hz), 7.12-7.42(m,5H). |
| Preparation 10 (10) | 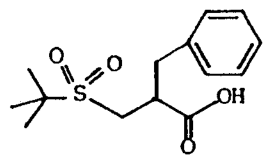 | 0.54 ($C_6H_6$/Diox/AcOH=10/3/0.5 V/V) | 1.36(s,9H),2.9-3.1(m, 2H),3.23(m,1H),3.35-3.53(m,2H),7.15-7.42 (m,5H). |
| Preparation 11 (11) | 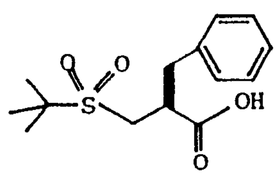 | 0.54 ($C_6H_6$/Diox/AcOH=10/3/0.5 V/V) | 1.36(s,9H),2.9-3.1(m, 2H),3.23(m,1H),3.35-3.53(m,2H),7.15-7.42 (m,5H). |
| Preparation 12 (12) | 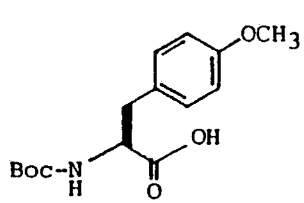 | 0.44 ($C_6H_6$/Diox/AcOH=10/3/0.5 V/V) | 1.42(9H),3.10(m,2H), 3.79(s,3H),4.56(m,1H), 4.94(br d,1H,J=9Hz), 6.75-7.16(m,4H). |

| COMPOUND | STRUCTURAL FORMULA | Rf value, ( ):solvent | NMR ($CDCl_3$)δ: |
|---|---|---|---|
| Preparation 13 (13) | 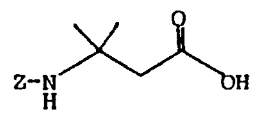 | 0.67 ($C_6H_6$/Diox/AcOH=10/5/1 V/V) | 1.43(s,6H),2.77(s,2H),5.07(s,2H),7.21-7.42 (m,5H). |
| Preparation 14 (14) | 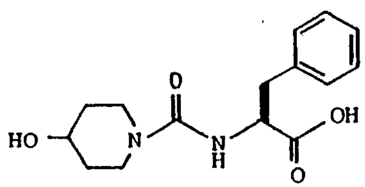 | 0.15 ($C_6H_6$/Diox/AcOH=10/5/1 V/V) | 1.34-1.54(m,2H),1.71-1.89(m,2H),2.86-3.38 (m,5H),3.51-3.68(m,2H),3.85(m,1H),4.53(m,1H),4.88(br d,1H,J=7Hz),7.12-7.38(m,5H). |
| Preparation 15 (15) | 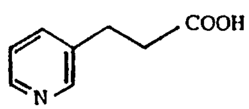 | 0.12 ($CHCl_3$/MeOH=10/1 V/V) | NMR ($D_2O$)δ: 2.62(t,2H),3.08(t,2H),7.90(m,1H),8.39(m,1H),8.61(m,2H). |
| Preparation 16 (16) | 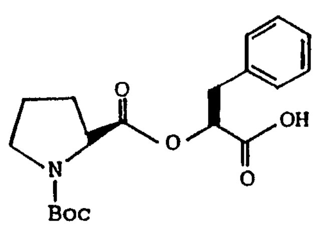 | 0.51 ($C_6H_6$/Diox/AcOH=10/5/1 V/V) | 1.25(s) and 1.42(s) total(9H),1.61-2.26 (m,4H),2.85-3.57(m,4H),4.18-4.40(m,1H),5.12-5.50(m,1H),7.10-7.35(m,5H). |

| COMPOUND | STRUCTURAL FORMULA | Rf value, ( ):solvent | NMR $(CDCl_3)\delta$: |
| --- | --- | --- | --- |
| Preparation 17 (17) | 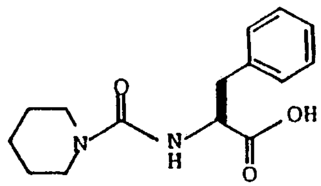 | 0.42 ($C_6H_6$/Diox/AcOH=10/5/1 V/V) | |
| Preparation 18 (18) | 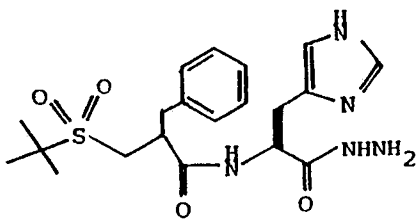 (isomer A) | 0.36 ($CHCl_3$/MeOH=5/1 V/V) | 1.50(s,9H),2.63(m,1H), 2.83-3.07(m,3H),3.31 (m,1H),3.88(m,2H),4.65 (m,1H),5.90(br s,1H), 6.08(s,1H),7.17-7.41(m, 6H),9.02(br d,1H,J= 9Hz). |
| Preparation 19 (19) | 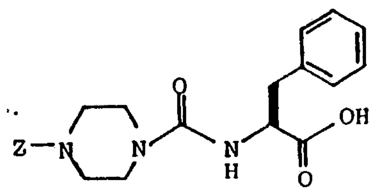 | 0.52 ($C_6H_6$/Diox/AcOH=10/5/1 V/V) | |
| Preparation 20 (20) | 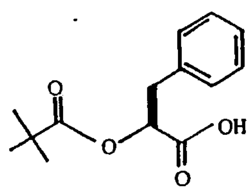 | 0.49 ($C_6H_6$/Diox/AcOH=10/3/0.5 V/V) | 1.15(s,9H),3.20(m,2H), 5.21(m,1H),7.17-7.36 (m,5H). |

| COMPOUND | STRUCTURAL FORMULA | Rf value, ( ):solvent | NMR ($CDCl_3$)δ: |
|---|---|---|---|
| Preparation 21 (21) | 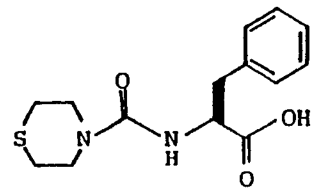 | 0.32 ($C_6H_6$/Diox/AcOH=10/5/1 V/V) | 2.49(m,4H),3.19(m,2H), 3.57(m,4H),4.63(m,1H), 4.85(br d,1H). |
| Preparation 22 (22) | 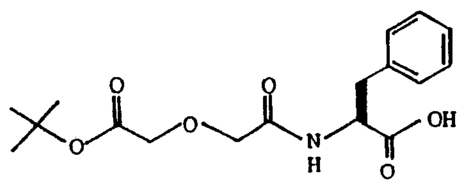 | 0.32 ($C_6H_6$/Diox/AcOH=10/3/0.5 V/V) | 1.47(s,9H),3.02-3.33 (m,2H),3.94(s,2H),4.04 (s,2H),4.85(m,1H) |
| Preparation 23 (23) | 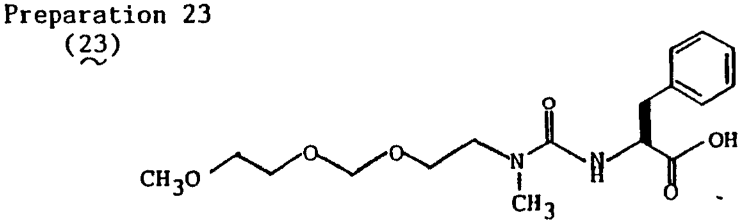 | 0.31 ($C_6H_6$/Diox/AcOH=10/5/1 V/V) | 2.88(s,3H),3.38(s,3H), 4.44(m,1H),4.59(m,2H). |
| Preparation 24 (24) | 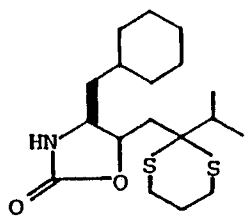 | 0.36 (AcOEt/Hex=35/65 V/V) | 0.8-2.1(m,21H),2.2-2.48 (m,3H),2.63-2.93(m,4H), 3.63(m,1H),4.6(m,1H), 5.4(br s,1H). |

| COMPOUND | STRUCTURAL FORMULA | Rf value, ( ):solvent | NMR ($CDCl_3$)δ: |
|---|---|---|---|
| Preparation 25 (25) | 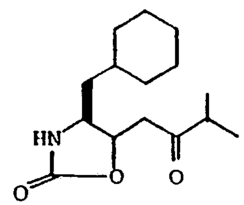 | 0.39 (AcOEt/Hex=1/1 V/V) | |
| Preparation 26 (26) | 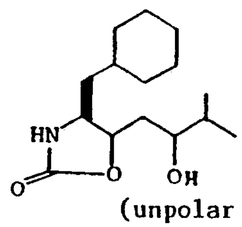 (unpolar isomer) | 0.52 (AcOEt/Hex=1/1 V/V) | 0.7-2.0(m,23H),3.57(m,1H),3.71(m,1H),4.45(m,1H),5.6(br s,1H). |
| Preparation 26 (26) | 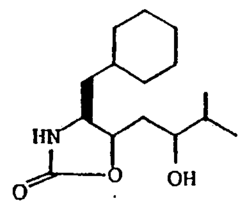 (polar isomer) | 0.48 (AcOEt/Hex=1/1 V/V) | 0.7-2.05(m,23H),3.51-3.72(m,2H),4.34(m,1H),5.9(br s,1H). |
| Preparation 27 (27) | 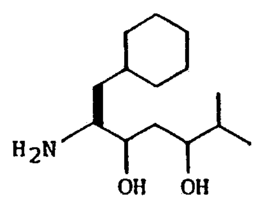 (isomer A) | 0.40 ($CHCl_3$/MeOH/aq.$NH_3$=80/20/1 V/V) | 0.68-1.9(m,22H),2.55(br s,4H),2.79(m,1H),3.45-3.70(m,2H). |

| COMPOUND | STRUCTURAL FORMULA | Rf value, ( ):solvent | NMR ($CDCl_3$)δ: |
|---|---|---|---|
| Preparation 28 (28) | 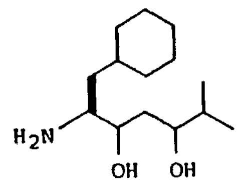 (isomer B) | 0.57 ($CHCl_3$/MeOH/aq.$NH_3$=80/20/1 V/V) | 0.72-1.86(m,22H),2.45-3.05(m,5H),3.51(m,1H),3.66(m,1H). |
| Preparation 29 (29) | 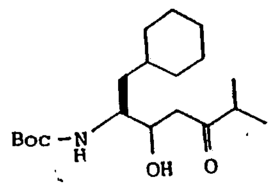 | 0.60 (AcOEt/Hex=1/2 V/V) | 0.7-1.94(m,19H),1.45(s,9H),2.5-2.7(m,2H),3.4(m,1H),3.65(m,1H),3.98(m,1H),4.57(br s,1H,J=9Hz). |
| Preparation 30 (30) | 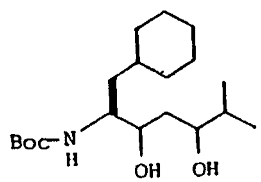 (isomer C) | 0.67 (AcOEt/Hex=1/2 V/V) | 0.65-1.97(m,22H),1.45(s,9H),3.45-3.95(m,3H),4.69(br d,1H,J=8Hz). |
| Preparation 30 (30) | 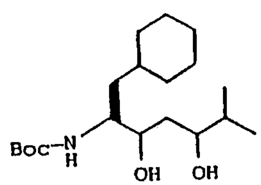 (isomer D) | 0.53 (AcOEt/Hex=1/2 V/V) | 0.65-2.0(m,22H),1.45(s,9H),3.5-3.96(m,3H),4.52(br d,1H,J=8Hz). |

| COMPOUND | STRUCTURAL FORMULA | Rf value, ( ):solvent | NMR ($CDCl_3$)δ: |
|---|---|---|---|
| Preparation 31 (31) | 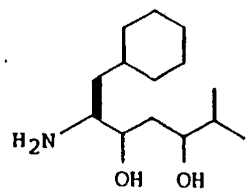 | 0.34 ($CHCl_3$/MeOH=5/1 V/V) | 0.68-1.87(m,22H),3.01(m,1H),3.36(br s,4H),3.61(m,1H),3.81(m,1H). |
| Example 1 (1) | 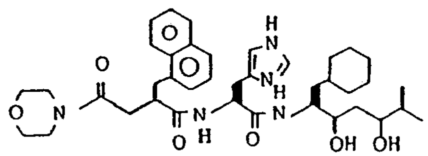 (isomer A) | 0.43 ($CHCl_3$/MeOH=5/1 V/V) | 0.6-1.9(m,22H),2.46(m,1H),2.67(m,1H),3.0-3.87(m,15H),3.94(m,1H),4.61(m,1H),6.72(br d,1H,J=9Hz),6.91(s,1H),7.2-8.3(m,9H). |
| Example 2 (2) | 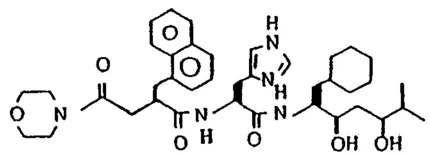 (isomer B) | 0.50 ($CHCl_3$/MeOH=5/1 V/V) | 0.6-1.9(m,22H),2.5(m,1H),2.71(m,1H),3.03-3.77(m,15H),3.84(m,1H),4.57(m,1H),6.61(br d,1H,J=9Hz),6.67(s,1H),7.22-8.18(m,9H). |
| Preparation 32 (32) | 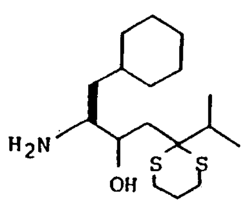 | 0.50 ($CHCl_3$/MeOH/aq.$NH_3$=90/10/1 V/V) | 0.72-2.13(m,22H),2.25-2.5(m,2H),2.63-3.08(m,5H),3.76(m,1H). |

| COMPOUND | STRUCTURAL FORMULA | Rf value, ( ):solvent | NMR ($CDCl_3$)δ: |
|---|---|---|---|
| Example 3 (3) | 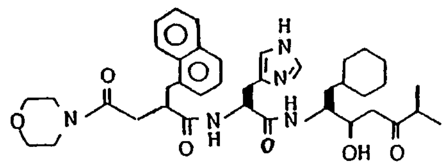 | 0.49 ($CHCl_3$/MeOH=5/1 V/V) | 0.67-1.9(m,19H),2.4-2.87(m,5H),3.0-3.8(m,13H),3.8-4.1(m,2H),4.6(m,1H),6.55(br s,1H),6.85(s,1H),7.23-8.2(m,8H). |
| Preparation 33 (33) | 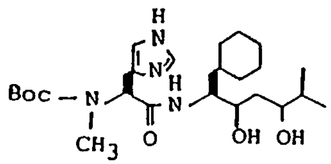 | 0.39 ($CHCl_3$/MeOH=5/1 V/V) | 0.74-1.90(m,22H),1.46(s,9H),2.76-3.37(m,2H),2.84(s,3H),3.58(m,1H),3.80(m,1H),4.03(m,1H),4.91(m,1H),6.63(br d,1H,J=10Hz),6.82(s,1H),7.45(s,1H). |
| Preparation 34 (34) | 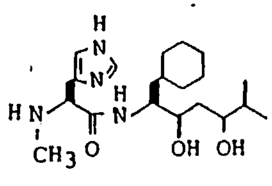 | 0.28 ($CHCl_3$/MeOH/aq.$NH_3$=85/15/1 V/V) | |

| COMPOUND | STRUCTURAL FORMULA | Rf value, ( ):solvent | NMR ($CDCl_3$)δ: |
|---|---|---|---|
| Example 4 (4) | 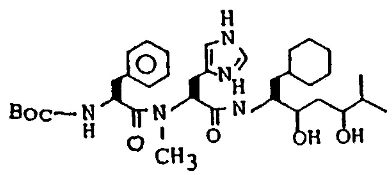 (isomer A) | 0.50 ($CHCl_3$/MeOH/aq.$NH_3$=85/15/1 V/V) | 0.72-1.97(m,22H),1.37 (s,9H),1.98(br s,1H), 2.54(br s,1H),2.63-3.34 (m,4H),2.81 and 3.03(s, 3H,rotamer),3.57(m,1H), 3.81(m,1H), 4.08(m,1H),4.59-5.03 (m,2H),5.28(br d,1H,J= 7Hz),6.68 and 6.74(s, 1H,rotamer), 7.10-7.36(m,5H),7.39 and 7.46(s,1H,rotamer), 7.61(br d,1H,J=9Hz). |
| Example 5 (5) | 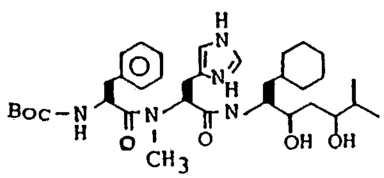 (isomer C) | 0.50 ($CHCl_3$/MeOH=5/1 V/V) | 0.68-1.88(m,22H),1.36 (s,9H),2.06(br s,1H), 2.55(br s,1H),2.62-3.37 (m,4H),2.62 and 2.93(s, 3H,rotamer), 3.57(m,1H),3.88(m,1H), 4.07(m,1H),4.54-5.0(m, 2H),5.22(br d,1H,J=6Hz), 6.70 and 6.78(s,1H, rotamer),7.1- 7.38(m,5H),7.40 and 7.50 (s,1H,rotamer), 7.69(br d,1H,J=9Hz). |

| COMPOUND | STRUCTURAL FORMULA | Rf value, ( ):solvent | NMR ($CDCl_3$)δ: |
|---|---|---|---|
| Example 6 (6) | 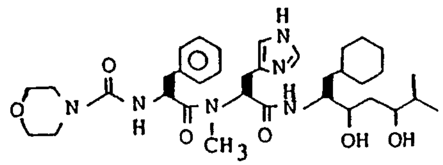 | 0.49 ($CHCl_3$/MeOH/aq.$NH_3$=85/15/1 V/V) | 0.70-1.85(m,22H),2.07 (br s,1H),2.54(br s,1H), 2.63-3.80(m,14H),2.79 and 2.99(s,3H,rotamer), 4.07(m,1H),4.51-5.05(m, 2H),5.24 and 5.40(br d, 1H,J=8Hz,rotamer), 6.70 and 6.80(s,1H, rotamer), 7.10-7.36(m,5H),7.39 and 7.49(s,1H,rotamer), 7.86(br d,1H,J=10Hz). |
| Example 7 (7) | 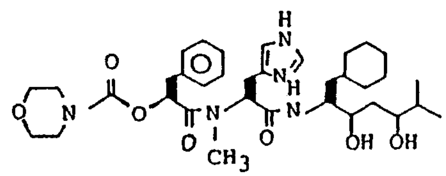 | 0.47 ($CHCl_3$/MeOH/aq.$NH_3$=85/15/1 V/V) | 0.70-1.86(m,22H),2.37 (br s,2H),2.60-3.88(m, 14H),2.83 and 3.02(s, 3H,rotamer), 4.08(m,1H),4.86-5.15(m, 1H),5.16-5.45(m,1H),6.75 and 6.81(s,1H,rotamer), 7.10-7.54(m,7H). |
| Example 8 (8) | 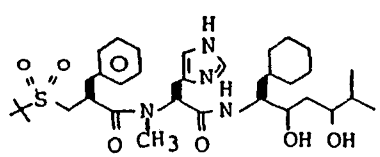 | 0.33($CHCl_3$/MeOH/aq.$NH_3$=90/10/1 V/V) | 1.36 and 1.37 (s,9H,rotamer),2.76 and 2.94(s,3H, rotamer),3.56(m,1H),4.09 (m,1H),6.66 and 6.79(s, 1H,rotamer),7.45 and 7.54(s,1H,rotamer). |

| COMPOUND | STRUCTURAL FORMULA | Rf value, ( ):solvent | NMR ($CDCl_3$)δ: |
|---|---|---|---|
| Example 9 (9) | 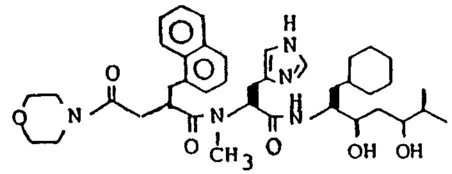 (isomer A) | 0.59 ($CHCl_3$/MeOH/aq.$NH_3$=85/15/1 V/V) | |
| Example 9 (9) | 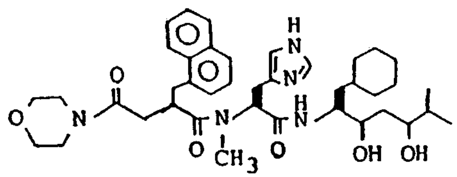 (isomer B) | 0.57 ($CHCl_3$/MeOH/aq.$NH_3$=85/15/1 V/V) | |
| Example 10 (10) | 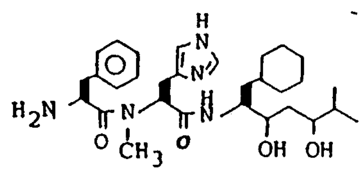 | 0.34 ($CHCl_3$/MeOH/aq.$NH_3$=85/15/1 V/V) | 0.69-1.90(m,22H),2.52-3.40(m,4H),2.84 and 3.02(s,3H,rotamer), 3.54(m,1H), 3.65-3.92(m,2H),4.00 (m,1H),5.01 and 5.33 (m,1H,rotamer),6.67 and 6.76(s,1H,rotamer), 7.04-7.39(m,5H), 7.41 and 7.43(s,1H, rotamer), 8.47(br d, 1H,J=10Hz). |

| COMPOUND | STRUCTURAL FORMULA | Rf value, ( ):solvent | NMR $(CDCl_3)\delta$: |
|---|---|---|---|
| Example 11 (11) | 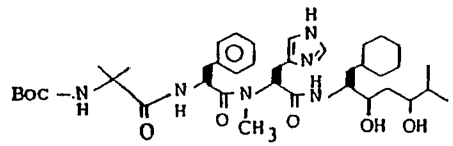 | 0.38 ($CHCl_3$/MeOH/aq.$NH_3$=85/15/1 V/V) | 0.74-1.91(m,28H),1.41 (s,9H),2.10(br s,1H), 2.60-3.38(m,4H),2.72 and 2.92(s,3H,rotamer), 3.53-4.12(m, 3H),4.89-5.25(m,2H), 6.63 and 6.76(s,1H, rotamer), 7.05-7.39(m,5H),7.45 and 7,54(s,1H,rotamer). |
| Example 12 (12) | 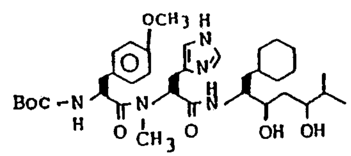 | 0.43 ($CHCl_3$/MeOH/aq.$NH_3$=85/15/1 V/V) | 0.70-1.85(m,22H),1.38 (s,9H),2.47-3.37(m,4H), 2.81 and 3.02(s,3H, rotamer),3.55 (m,1H),3.75(m,1H),3.76 (s,3H),4.04(m,1H),4.50-5.31(m,2H),5.70(br d,J=10Hz),6.70 and 6.75(s, 1H,rotamer), 6.76-6.90(m,2H),7.00-7.18(m,2H),7.41 and 7.47 (s,1H,rotamer), 7.58(br s,1H). |
| Example 13 (13) | 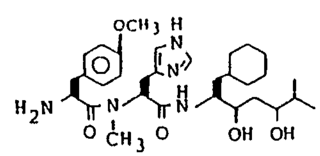 | 0.35 ($CHCl_3$/MeOH/aq.$NH_3$=85/15/1 V/V) | 0.65-1.88(m,22H),2.45-3.36(m,4H),2.84 and 2.91(s,3H,rotamer), 3.54(m,1H),3.63-3.90 (m,2H),3.78(s,3H), 3.98(m,1H),5.00 and 5.33(m,1H,rotamer),6.68 and 6.75(s,1H,rotamer), 6.78-6.90(m,2H), 7.00-7.14(m,2H),7.40 and 7.45(s,1H,rotamer), 8.46(br d,1H,J=9Hz). |

| COMPOUND | STRUCTURAL FORMULA | Rf value, ( ):solvent | NMR ($CDCl_3$)δ: |
|---|---|---|---|
| Example 14 (14) | 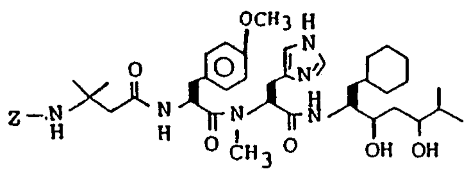 | 0.49 ($CHCl_3$/MeOH/aq.$NH_3$=85/15/1 V/V) | 0.55-1.85(m,28H),2.20-3.90(m,8H),2.76 and 3.01(s,3H,rotamer), 3.73(s,3H), 4.05(m,1H),4.58-5.33 (m,2H),5.04(s,2H),6.65 and 6.69(s,1H,rotamer), 6.68-7.49(m,10H), 7.74(br d,1H,J=9Hz). |
| Example 15 (15) | 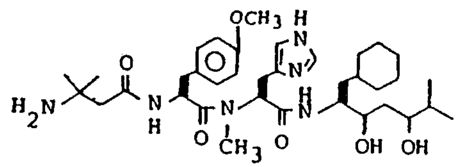 | 0.17 ($CHCl_3$/MeOH/aq.$NH_3$=85/15/1 V/V) | 0.71-1.87(m,28H),2.00-2.24(m,2H),2.54-3.36 (m,4H),2.79 and 3.02(s, 3H,rotamer), 3.55(m,1H),3.77(s,3H), 3.78(m,1H),4.06(m,1H), 4.66-5.37(m,2H),6.71 and 6.75(s,1H,rotamer), 6.77-6.93(m,2H), 7.04-7.21(m,2H),7.43(s, 1H),7.65(br d,1H,J=9Hz). |
| Example 16 (16) | 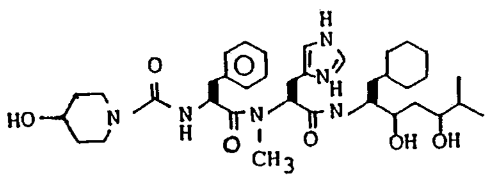 | 0.35 ($CHCl_3$/MeOH=5/1 V/V) | 0.73-1.90(m,26H),2.05 (br s,1H),2.33-3.39(m, 7H),2.77 and 3.01(s,3H, rotamer),3.45-3.96(m,5H),4.06(m,1H), 4.72(m,1H),4.80-5.10(m,1H),5.49(br d,1H, J=7Hz),6.69 and 6.76(s, 1H,rotamer), 7.1-7.48(m,6H),7.93(br d, 1H,J=9Hz). |

| COMPOUND | STRUCTURAL FORMULA | Rf value, ( ):solvent | NMR $(CDCl_3)\delta$: |
|---|---|---|---|
| Example 17 (17) | 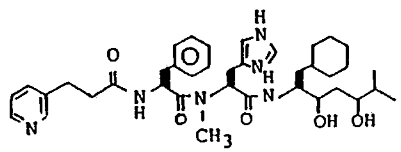 | 0.30 ($CHCl_3$/MeOH/aq.$NH_3$=85/15/1 V/V) | 0.73-1.90(m,22H),2.09 (br s,1H),2.38-3.33(m, 9H),2.80 and 2.97(s,3H, rotamer), 3.59(m,1H),3.82(m,1H), 4.08(m,1H),4.82-5.41(m, 2H),6.68 and 6.69(s,1H, rotamer),6.96-7.57(m,9H),7.62(br d,1H, J=10Hz),8.30-8.50(m,2H). |
| Example 18 (18) | 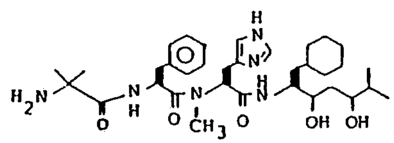 | 0.37 ($CHCl_3$/MeOH/aq.$NH_3$=85/15/1 V/V) | 0.74-1.85(m,28H),2.59-3.37(m,4H),2.77 and 3.00(s,3H,rotamer), 3.56(m,1H), 3.71-4.19(m,2H),4.75(m, 1H),5.00(m,1H),6.69 and 6.78(s,1H,rotamer), 7.14-7.42(m,5H),7.45 and 7.49(s,1H,rotamer), 7.72 (br d,1H,J=10Hz),8.35(br d,1H,J=3Hz). |
| Example 19 (19) | 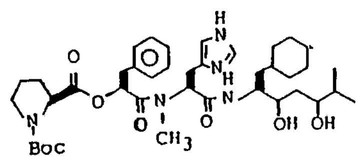 | 0.31 ($CHCl_3$/MeOH/aq.$NH_3$=90/10/1 V/V) | |
| Example 20 (20) | 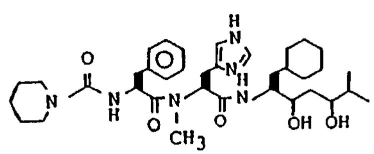 | 0.39 ($CHCl_3$/MeOH/aq.$NH_3$=90/10/1 V/V) | 0.70-1.85(m,28H),2.50-3.40(m,8H),2.78(s,3H), 3.54(m,1H),3.75(m,1H), 4.09(m,1H),4.79(m,1H), 4.96(m,1H),5.27(br d, 1H,J=8Hz),6.69(s,1H),7.15-7.40(m,5H),7.42(s,1H), 7.99(br d,1H,J=10Hz). |

| COMPOUND | STRUCTURAL FORMULA | Rf value, ( ):solvent | NMR ($CDCl_3$)δ: |
| --- | --- | --- | --- |
| Example 21 (21) | 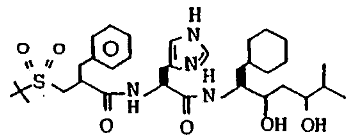 | 0.45 ($CHCl_3$/MeOH=5/1 V/V) | 0.70-1.84(m,22H),1.34 (s,9H),2.78-3.40(m,6H), 3.49-3.69(m,2H),3.81(m, 1H),3.96(m, 1H),4.56(m,1H),6.58(br d,1H,J=9Hz),6.90(s,1H), 7.10-7.34(m,5H),7.46(s, 1H). |
| Example 22 (22) | 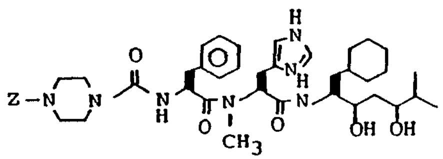 | 0.51 ($CHCl_3$/MeOH/aq.$NH_3$=90/10/1 V/V) | 0.69-1.83(m,22H),2.59-3.60(m,13H),2.80(s,3H), 3.72(m,1H),4.07(m,1H), 4.71(m,1H),4.89(m,1H), 5.12(s,2H),5.30(br d, 1H),6.69(s,1H),7.10-7.49(m,11H),7.82(br d,1H,J=9Hz). |
| Example 23 (23) | 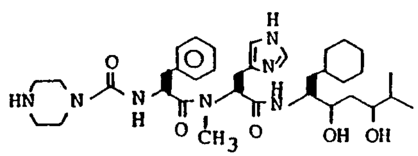 | 0.16 ($CHCl_3$/MeOH/aq.$NH_3$=90/10/1 V/V) | 0.70-1.83(m,22H),2.45-3.40(m,12H),2.78(s,3H), 3.52(m,1H),3.73(m,1H), 4.07(m,1H),4.76(m,1H), 4.91(m,1H),5.23(br d, J=6Hz),6.68(s,1H),7.12-7.52(m,6H),7.89(br d, J=9Hz). |
| Example 24 (24) | 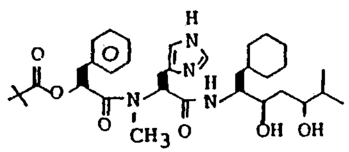 | 0.53 ($CHCl_3$/MeOH=5/1 V/V) | 1.12-1.15(s,9H,rotamer), 2.83 and 3.06(s,9H,rotamer), 3.54(m,1H), 3.81(m,1H),4.08(m,1H), 6.76 and 6.83(s,1H, rotamer), 7.74 and 7.49(s,1H, rotamer). |

| COMPOUND | STRUCTURAL FORMULA | Rf value, ( ):solvent | NMR ($CDCl_3$)δ: |
|---|---|---|---|
| Example 25 (25) | 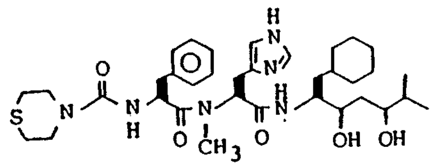 | 0.53 ($CHCl_3$/MeOH/aq.$NH_3$=85/15/1 V/V) | 4.10(m,1H),4.51-5.10(m,2H),6.71 and 6,80(s,1H,rotamer),7.84 (br d,1H). |
| Prepraration 35 (35) | 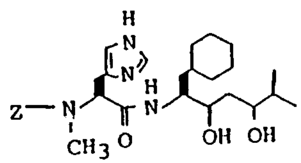 | 0.34 ($CHCl_3$/MeOH/aq.$NH_3$=90/10/1 V/V) | 2.93(s,3H),3.54(m,1H),3.78(m,1H),4.04(m,1H),4.95(m,1H),5.10(m,2H). |
| Preparation 36 (36) | 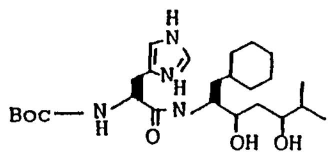 | 0.47 ($CHCl_3$/MeOH=5/1 V/V) | 1.44(s,9H),3.05(m,2H),3.58(m,1H),3.79(m,1H),4.00(m,1H),4.33(m,1H),6.84(s,1H),7.51(s,1H). |
| Preparation 37 (37) | 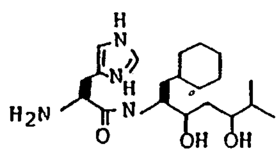 | 0.14 ($CHCl_3$/MeOH/aq.$NH_3$=85/15/1 V/V) | |

| COMPOUND | STRUCTURAL FORMULA | Rf value, ( ):solvent | NMR ($CDCl_3$)δ: |
|---|---|---|---|
| Example 26 (26) | 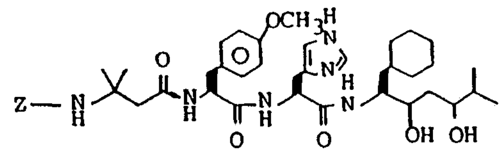 | 0.50 ($CHCl_3$/MeOH/aq.$NH_3$=85/15/1 V/V) | 3.56(m,1H),3.73(s,3H), 3.97(m,1H),4.44(m,1H), 4.57(m,1H),4.95(m,2H), 7.49(s,1H). |
| Example 27 (27) | 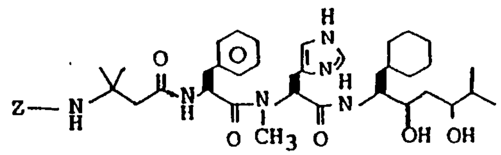 | 0.56 ($CHCl_3$/MeOH/aq.$NH_3$=85/15/1 V/V) | 2.76 and 3.00(s,3H, rotamer), 3.42-4.12(m,3H),5.05 (s,2H),6.64 and 6.69 (s,1H,rotamer). |
| Example 28 (28) | 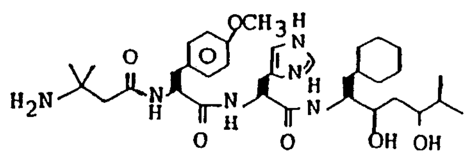 | 0.12 ($CHCl_3$/MeOH/aq.$NH_3$=85/15/1 V/V) | 2.24(m,2H),2.89(m,2H), 3.19(m,2H),3.55(m,1H), 3.77(s,3H),3.91(m,1H), 4.46(m,1H),4.59(m,1H), 7.48(s,1H). |
| Example 29 (29) | 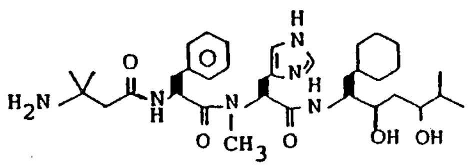 | 0.15 ($CHCl_3$/MeOH/aq.$NH_3$=85/15/1 V/V) | 3.54(m,1H),3.78(m,1H), 4.08(m,1H),7.45 and 7.47(s,1H,rotamer). |

| COMPOUND | STRUCTURAL FORMULA | Rf value, ( ):solvent | NMR ($CDCl_3$)δ: |
|---|---|---|---|
| Example 30 (30) | 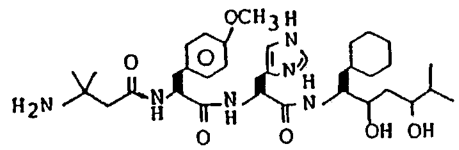 $\cdot 2CH_3COOH$ | | NMR($CD_3OD$) 1.98(s,6H),2.46(m,2H), 3.56(m,1H),3.77(s,1H), 3.95(m,1H),6.97(2,1H), 7.70(s,1H). |
| Example 31 (31) | 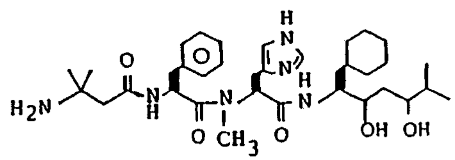 $\cdot 2CH_3COOH$ | | NMR($CD_3OD$) 1.97(s,6H),2.85 and 3.13 (s,1H,rotamer), 3.52(m,2H),3.82(m,1H), 3.98(m,1H),6.86 and 6.90 (s,1H,rotamer), |
| Example 32 (32) | 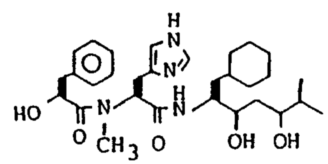 | 0.46 ($CHCl_3$/MeOH=5/1 V/V) | 3.54(m,1H),3.77(m,1H), 4.03(m,1H),4.55(m,1H), 5.12 and 5.35(m,1H, rotamer),6.64 and 6.74(s,1H,rotamer). |
| Example 33 (33) | 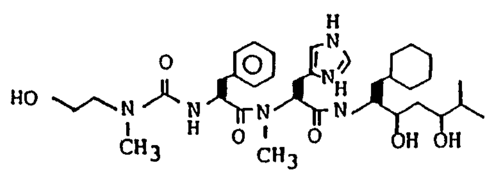 | 0.40 ($CHCl_3$/MeOH=5/1 V/V) | 4.09(m,1H),4.55-5.02(m, 2H),6.69 and 6.72(s,1H, rotamer). |

| COMPOUND | STRUCTURAL FORMULA | Rf value, ( ):solvent | NMR ($CDCl_3$)δ: |
|---|---|---|---|
| Example 34 (34) | 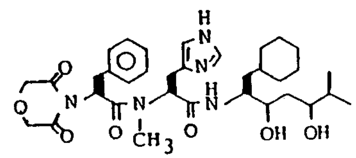 | 0.35 ($CHCl_3$/MeOH/aq.$NH_3$=85/15/1 V/V) | 2.83(s,3H),3.58(m,1H), 5.31(m,1H),5.45(m,1H), 7.62(s,1H). |
| Example 35 (35) | 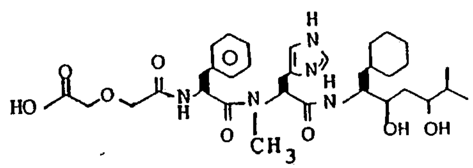 $\cdot CF_3COOH$ | 0.18 ($CHCl_3$/MeOH=5/1 V/V) | |
| Example 36 (36) | 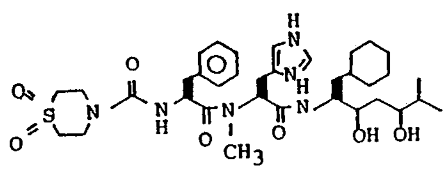 | 0.38 ($CHCl_3$/MeOH/aq.$NH_3$=85/15/1 V/V) | 3.50-3.95(m,6H),4.05 (m,1H),6.76 and 6.81(s, 1H,rotamer). |
| Example 37 (37) | 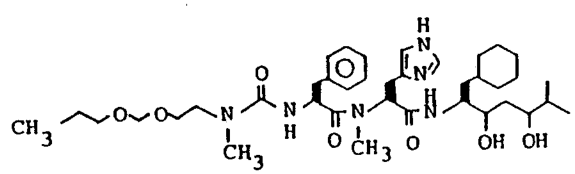 | 0.46 ($CHCl_3$/MeOH/aq.$NH_3$=85/15/1 V/V) | 3.38(s,3H),4.11(m,1H), 4.55-4.75(m,3H),4.95(m, 1H),6.69(s,1H),7.42(s,1H). |

| COMPOUND | STRUCTURAL FORMULA | Rf value, ( ):solvent | NMR ($CDCl_3$)δ: |
|---|---|---|---|
| Example 38 (38) | 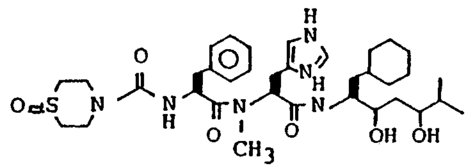 | 0.18($CHCl_3$/MeOH=5/1 V/V) | 2.80 and 3.06(s,3H,rotamer),3.53(m, 1H),4.04(m,1H),4.75(m, 1H),6.74 and 6.80(s,1H, rotamer). |
| Preparation 38 (38) | 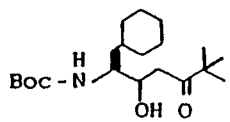 (isomer A) | | 0.77-1.9(m,32H),2.7-2.82 (m,2H),3.43(m,1H),3.65(m, 1H),3.98(m,1H),4.72(br d, 1H,J=9Hz). |
| Preparation 38 (38) | 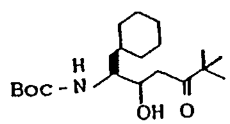 (isomer B) | | 0.75-1.96(m,32H),2.65-2.78 (m,2H),3.42(m,1H),3.63(m, 1H),3.96(m,1H),4.58(br d, 1H,J=9Hz). |
| Preparation 39 (39) | 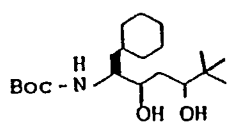 (isomer C) | 0.51 (AcOEt/Hex=30/70 V/V) | 0.7-1.92(m,32H),2.64(m,1H), 3.43-4.0(m,4H),4.72(br d, 1H,J=9Hz). |

| COMPOUND | STRUCTURAL FORMULA | Rf value, ( ):solvent | NMR ($CDCl_3$)δ: |
|---|---|---|---|
| Preparation 39 (39) | 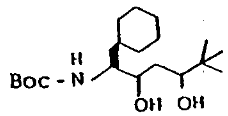 (isomer D) | 0.46 (AcOEt/Hex=30/70 V/V) | 0.75-1.9(m,32H),2.34(m,1H), 2.53(m,1H),3.49(m,1H),3.78 (m,2H),4.61(br d,1H,J=9Hz). |
| Preparation 40 (40) | 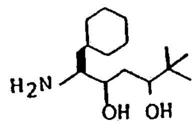 (isomer C) | 0.55 ($CHCl_3$/MeOH/aq.$NH_3$=85/15/1 V/V) | 2.98(m,1H),3.7(m,1H), 3.83(m,1H). |
| Example 39 (39) | 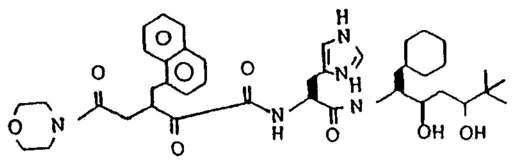 (isomer C) | 0.40 ($CHCl_3$/MeOH/aq.$NH_3$=90/10/1 V/V) | 0.67-1.85(m,24H),2.45-2.75(m,2H),3.0-3.9(m, 15H),4.56(m,1H),6.47 (br d,1H,J=9Hz),6.92 (s,1H),7.2-8.2(m,9H). |
| Preparation 41 (41) | 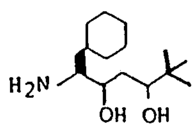 (isomer D) | 0.56 ($CHCl_3$/MeOH/aq.$NH_3$=85/15/1 V/V) | 0.68-1.87(m,24H),3.0 (m,1H),3.44(br s,4H), 3.7(m,1H),3.81(m,1H). |

| COMPOUND | STRUCTURAL FORMULA | Rf value, ( ):solvent | NMR $(CDCl_3)\delta$: |
|---|---|---|---|
| Example 40 (40) | 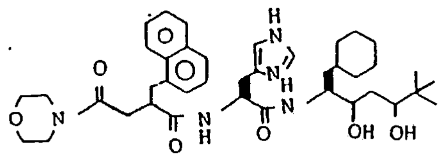 (isomer D) | 0.36 ($CHCl_3$/MeOH/aq.$NH_3$=90/10/1 V/V) | 0.67-1.9(m,24H),2.45-2.7 (m,2H),2.95-4.13 (m,1H),4.6(m,1H),6.72(br d,1H,J=9Hz),7.2-8.2(m,9H). |
| Preparation 42 (42) | 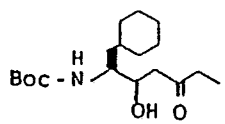 (isomer A) | 0.28 (AcOEt/Hex=1/4 V/V twice) | 0.78-1.89(m,19H),2.5(m,2H), 2.66(m,2H),3.47(m,1H),3.51-3.66(m,1H),4.02(m,1H),4.65-4.75(br d,1H , J=9Hz). |
| Preparation 42 (42) | 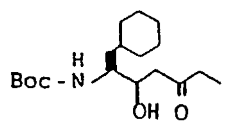 (isomer B) | 0.24 (AcOEt/Hex=1/4 V/V twice) | 0.8-1.9(m,19H),2.5(m,2H), 2.65(m,2H),3.47-3.6(m,2H), 4.0(m,1H),4.55(br d,1H, J=9Hz). |
| Preparation 43 (43) | 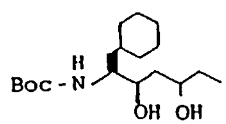 (isomer C) | 0.27 (AcOEt/Hex=3/7 V/V) | 0.7-1.62(m,20H),2.36(m, 1H),2.81-2.82(m,1H),3.66 (m,1H),3.84-3.87(m,2H), 4.63-4.67(br d,1H,J=9Hz). |

| COMPOUND | STRUCTURAL FORMULA | Rf value, ( ):solvent | NMR ($CDCl_3$)δ: |
|---|---|---|---|
| Preparation 43 (43) | 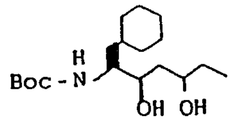 (isomer D) | 0.24 (AcOEt/Hex=3/7 V/V) | 0.7-1.6(m,20H),2.36(m,1H),2.8(m,1H),3.66(m,1H),3.81-3.9(m,2H),4.7-4.73 (br d,1H,J=9Hz). |
| Preparation 44 (44) | 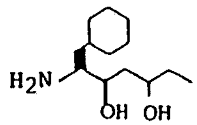 (isomer C) | 0.56 ($CHCl_3$/MeOH/aq.$NH_3$=85/15/1 V/V) | 3.0(m,1H),3.7(m,1H),3.85(m,1H). |
| Example 41 (41) | 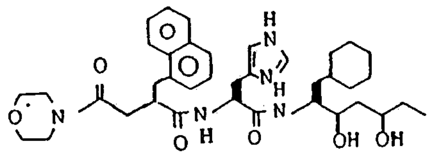 (isomer C) | 0.40 ($CHCl_3$/MeOH/aq.$NH_3$=85/15/1 V/V) | 0.7-1.82(m,20H),2.45-2.8(m,2H),3.15-3.88 (m,15H),4.58(br d,1H,J=9Hz),6.40-8.10(m,11H). |
| Preparation 45 (45) | 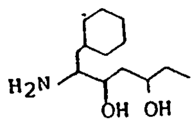 (isomer D) | 0.56 ($CHCl_3$/MeOH/aq.$NH_3$=85/15/1 V/V) | 3.0(m,1H),3.6(m,1H),3.83 (m,1H). |

| COMPOUND | STRUCTURAL FORMULA | Rf value, ( ):solvent | NMR ($CDCl_3$)δ: |
|---|---|---|---|
| Example 42 (42) | 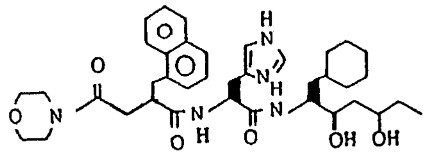 (isomer D) | 0.35 ($CHCl_3$/MeOH/aq.$NH_3$=85/15/1 V/V) | 0.68-1.75(m,20H),2.40-2.8(m,2H),3.10-3.95 (m,15H),4.60(m,1H),6.60 (br d,1H,J=9Hz),6.95-8.09(m,10H). |
| Preparation 46 (46) | 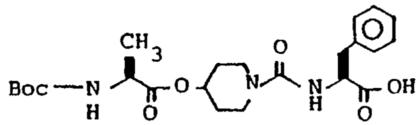 | 0.47 ($C_6H_6$/Diox/AcOH=10/5/1 V/V) | 1.37(d,3H),1.44(s,9H), 3.0-3.3(m,4H),3.4-3.7 (m,2H),4.28(m,1H),4.64 (m,1H),4.9-5.2(m,3H), 7.4-7.5(m,5H). |
| Preparation 47 (47) | 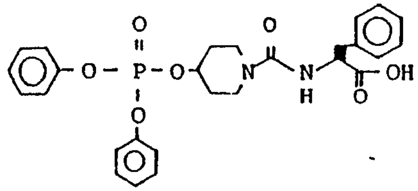 | 0.45 ($C_6H_6$/Diox/AcOH=10/5/1 V/V) | 1.4-2.0(m,4H),2.8-3.6 (m,6H),4.55(m,1H),4.74 (m,1H),5.35(d,1H),7.0-7.6(m,15H),9.6(br s,1H). |
| Preparation 48 (48) | 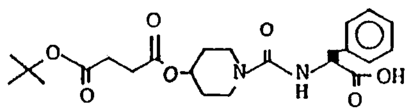 | 0.53 ($C_6H_6$/Diox/AcOH=10/5/1 V/V) | 1.48(s,9H),2.4-2.8(m,4H), 2.9-3.8(m,6H),4.64(m,1H), 4.92(m,1H),5.14(d,1H), 7.1-7.6(m,5H),9.5(br s, 1H). |

| COMPOUND | STRUCTURAL FORMULA | Rf value, ( ):solvent | NMR ($CDCl_3$)δ: |
|---|---|---|---|
| Example 43 (43) | 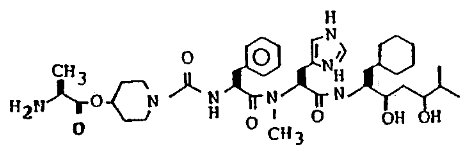 ·$2CF_3COOH$ | | NMR ($CD_3OD$)δ: 2.71 and 3.06(s,9H,rotamer), 4.08(m,1H),7.1-7.6(m,6H), 9.71 and 9.74(s,1H,rotamer). |
| Example 44 (44) | 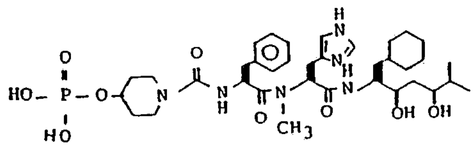 | | NMR ($CD_3OD$)δ: 2.46(m,1H),3.80(m,1H), 3.99(m,1H),4.35(m,1H), 5.40(m,1H). |
| Example 45 (45) | 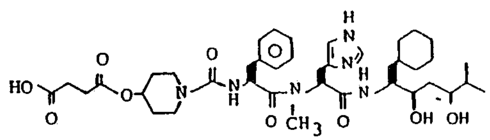 ·$CF_3COOH$ | | NMR ($CD_3OD$)δ: 2.60(m,4H),2.71 and 3.06 (s,3H,rotamer),4.75(m,1H), 5.22(m,1H),7.0-7.6(m,6H), 8.73 and 8.75(s,1H,rotamer). |
| Example 46 (46) | 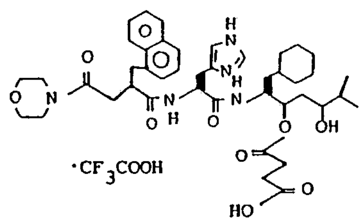 ·$CF_3COOH$ | | NMR ($CD_3OD$)δ: 2.59(m,4H),4.14(m,1H), 4.53(m,1H),5.17(m,1H),7.2-7.7(m,4H),7.35(m,1H),7.76 (m,1H),7.85(m,1H),8.15(m,1H), 8.76(m,1H). |

| COMPOUND | STRUCTURAL FORMULA | Rf value, ( ):solvent | NMR $(CDCl_3)\delta$: |
|---|---|---|---|
| Example 47 (47) | 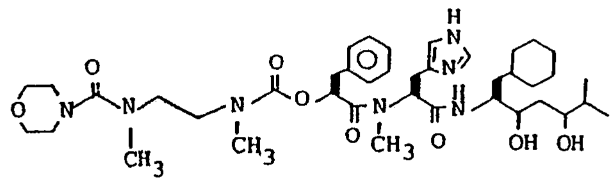 | 0.32($CHCl_3$/MeOH/aq.$NH_3$=90/10/1 V/V) | 4.08(m,1H),4.93(m,1H), 5.17-5.28(m,1H),6.74 (s,1H). |
| Example 48 (48) | 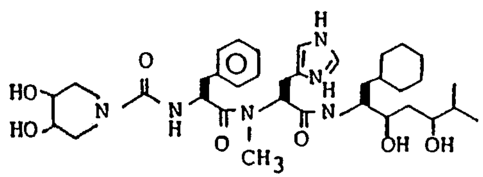 | 0.10($CHCl_3$/MeOH/aq.$NH_3$=85/15/1 V/V) | NMR $(CD_3OD)\delta$; 2.87 and 3.05 (s,3H, rotamer),3.80(m,1H), 3.99(m,1H),6.89 and 6.96(s,1H,rotamer), 7.62 and 7.78 (s,1H,rotamer). |

## Claims

(1) An amino acid derivative of the general formula:

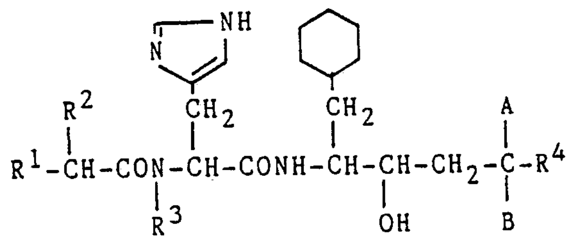

wherein $R^1$ is

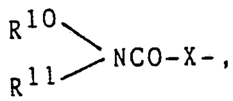 $R^{12}$-NH-, $R^{13}$-CO-Y-, $R^{13}$-$SO_2$-$CH_2$-, 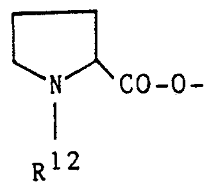 ,

HO- or 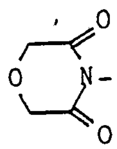

wherein, $R^{10}$ is a lower alkyl group and $R^{11}$ is

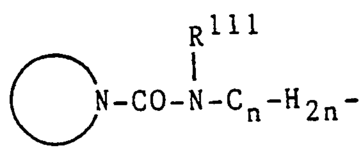

(wherein $R^{111}$ is a lower alkyl group and n is an integer of 1 to 5) or a lower alkyl group which may be substituted by hydroxy group or methoxyethoxymethoxy group, or $R^{10}$ and $R^{11}$ are

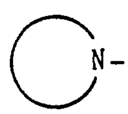

combinedly together with the adjacent nitrogen atom
$R^{12}$ is a hydrogen atom, $C_nH_{2n+1}$-O-CO- (n is as defined above) or

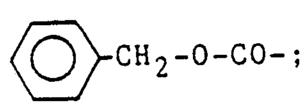

$R^{13}$ is a lower alkyl group which may be substituted by substituent(s) selected from HOOC-$(H_2C)_n$-O-, $R^{12}$-NH- (n and $R^{12}$ are as defined above) and pyridyl group;
X is -$CH_2$-, -O- or -NH- and Y is -O- or -NH-;
wherein

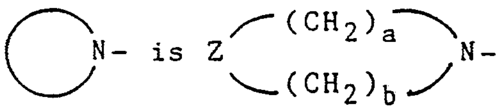

(wherein Z is -O-, -S-, -S(O)-, $-S(O)_2-$, $-CH_2-$, -CH(OH)-,
OH OH
- CH - CH -, -NH- or

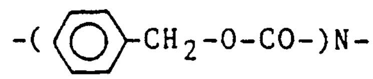

and a and b are independently an integer of 1 to 4 and the total of a and b is not more than 5) ;
$R^2$ is an aralkyl group which may be substituted by lower alkyl group(s);
$R^3$ is a hydrogen atom or a lower alkyl group;
$R^4$ is a lower alkyl group;
and A is hydroxy group and B is a hydrogen atom, or A and B are carbonyl group combinedly together with the adjacent carbon atom, or a pharmaceutically acceptable acid addition salt or an ester thereof.

(2) A compound as claimed in claim 1 wherein $R^1$ is $R^{13}-SO_2-CH_2-$ or

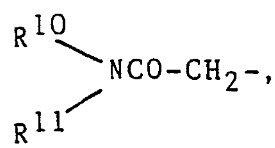

$R^2$ is naphthyl methyl group or methoxybenzyl group or $R^3$ is a lower alkyl group

(3) A compound as claimed in claim 1, which is selected from among the group consisting of (2S)-2-[N$^\alpha$-{3-morpholinocarbonyl-2-(1-naphthylmethyl)propionyl}-L-histidyl]amino-1-cyclohexyl-6-methyl-3,5-heptanediol, (2S)-2-[N$^\alpha$-{3-morpholinocarbonyl-2-(1-naphthylmethyl)propionyl}-L-histidyl]amino-1-cyclohexyl-3-hydroxy-6-methyl-5-heptanone, (2S)-2-[N$^\alpha$-(N- tert-butoxycarbonyl-L-phenylalanyl)-N$^\alpha$-methyl-L-histidyl]amino-1-cyclohexyl-6-methyl-3,5-heptanediol, (2S)-2-[N$^\alpha$-(N-morpholinocarbonyl-L-phenylalanyl)-N$^\alpha$-methyl-L-histidyl]amino-1-cyclohexyl-6-methyl-3,5-heptanediol, (2S)-2-(N$^\alpha$-{[(2S)-2-morpholinocarbonyloxy-3-phenylpropionyl}-N$^\alpha$-methyl-L-histidyl]amino-1-cyclohexyl-6-methyl-3,5-heptanediol, (2S)-2-[N$^\alpha$-{3-morpholinocarbonyl-2-(1-naphthylmethyl)propionyl}-N$^\alpha$-methyl-L-histidyl]amino-1-cyclohexyl-6-methyl-3,5-heptanediol, (2S)-2-{N$^\alpha$-{N-(2-tert-butoxycarbonylamino-2-methylpropionyl)-L-phenylalanyl}-N$^\alpha$-methyl-L-histidyl]amino-1-cyclohexyl-6-methyl-3,5-heptanediol, (2S)-2-[N$^\alpha$-{N-(3-benzyloxycarbonylamino-3-methylbutyryl)-O-methyl-L-tyrosyl}-N$^\alpha$-methyl-L-histidyl]amino-1-cyclohexyl-6-methyl-3,5-heptanediol, (2S)-2-[N$^\alpha$-{N-(4-hydroxypiperidino)carbonyl-L-phenylalanyl}-N$^\alpha$-methyl-L-histidyl]-amino-1-cyclohexyl-6-methyl-3,5-heptanediol, (2S)-2-[N$^\alpha$-[N-{3-(3-pyridyl)propionyl}-L-phenylalanyl]-N$^\alpha$-methyl-L-histidyl]amino-1-cyclohexyl-6-methyl-3,5-heptanediol, (2S)-2-[N$^\alpha$-(N-piperidinocarbonyl-L-phenylalanyl)-N$^\alpha$-methyl-L-histidyl]amino-1-cyclohexyl-6-methyl-3,5-heptanediol, (2S)-2-[N$^\alpha$-{N-(2-amino-2-methylpropionyl)-L-phenylalanyl}-N$^\alpha$-methyl-L-hisdityl]amino-1-cyclohexyl-6-methyl-3,5-heptanediol, (2S)-2-[N$^\alpha$-(2-benzyl-3-tert-butylsulfonylpropionyl)-L-histidyl]amino-1-cyclohexyl-6-methyl-3,5-heptanediol, (2S)-2-[N$^\alpha$-[N-{1-(4-benzyloxycarbonyl)piperazinylcarbonyl}-L-phenylalanyl]-N-methyl-L-histidyl]amino-1-cyclohexyl-6-methyl-3,5-heptanediol, (2S)-2-[N$^\alpha$-[N-{(tetrahydro-4H-1,4-thiazine)- 4-yl-carbonyl}-L-phenylalanyl]-N$^\alpha$-methyl-L-histidyl]amino-1-cyclohexyl-6-methyl-3,5-heptanediol, (2S)-2-[N$^\alpha$-{N-(3-amino-3-methylbutyryl)-O-methyl-L-tyrosyl}-L-histidyl]amino-1-cyclohexyl-6-methyl-3,5-heptanediol, (2S)-2-[N$^\alpha$-{N-(3-amino-3-methylbutyryl)-L-phenylalanyl}-N$^\alpha$-methyl-L-histidyl]amino-1-cyclohexyl-6-methyl-3,5-heptanediol, (2S)-2-[N$^\alpha$-{N-(2-hydroxyethyl)-N-methylaminocarbonyl-L-phenylalanyl}-N$^\alpha$-methyl-L-histidyl]-amino-1-cyclohexyl-6-methyl-3,5-heptanediol, (2S)-2-[N$^\alpha$-[(2S)-2-(3,5-dioxomorpholino)-3-phenylpropionyl}-N$^\alpha$-methyl-L-histidyl]amino-1-cyclohexyl-6-methyl-3,5-heptanediol, (2S)-2-[N$^\alpha$-[N-{(1,1-dioxo-2,3,5,6-tetrahydro-4H-1,4-thiazine)-4-yl-carbonyl}-L-phenylalanyl]-N$^\alpha$-methyl-L-histidyl]amino-1-cyclohexyl-6-methyl-3,5-heptanediol, (2S)-2-[N$^\alpha$-[N-{N-(2-methoxyethoxymethoxyethyl)-N-methylaminocarbonyl}-L-phenylalanyl]-N$^\alpha$-methyl-L-histidyl]amino-1-cyclohexyl-6-methyl-3,5-heptanediol,

(2S)-2-[$N^{\alpha}$-[N-{(1-oxo-2,3,5,6-tetrahydro-4H-1,4-thiazine)-4-yl-carbonyl}-L-phenylalanyl]-$N^{\alpha}$-methyl-L-histidyl]amino-1-cyclohexyl-6-methyl-3,5-heptanediol, (2S)-2-[$N^{\alpha}$-{3-morpholinocarbonyl-2-(1-naphthyl)-propionyl}-L-histidyl]amino-1-cyclohexyl-6,6-dimethyl-3,5-heptanediol and (2S)-2-[$N^{\alpha}$-(3-morpholinocarbonyl-2-(1-naphthylmethyl)propionyl}-L-histidyl]amino-1-cyclohexyl-6-methyl-3,5-heptanediol.

(4) An amino acid derivative of the general formula:

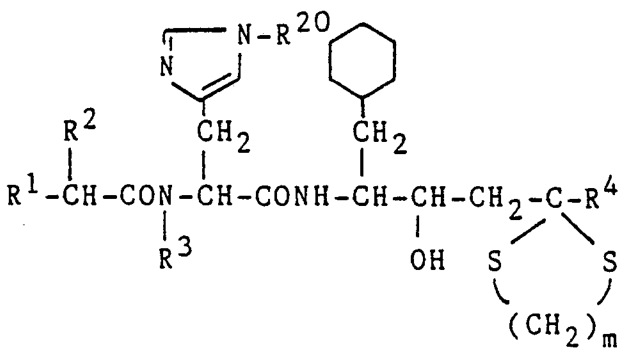

wherein $R^1$, $R^2$, $R^3$ and $R^4$ are respectively of the same meanings as defined in claim 1, $R^{20}$ is hydrogen or N-protective group and m is an integer of 2 to 4, or a pharmaceutically acceptable acid addition salt or an ester thereof.

(5) A compound of the general formula:

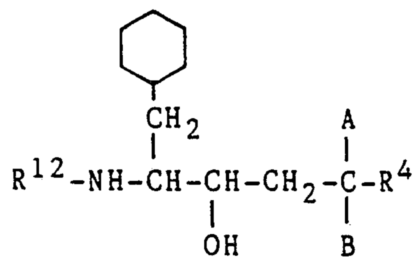

wherein $R^{12}$, A, B and $R^4$ are as defined in claim 1.

(6) A compound of the general formula:

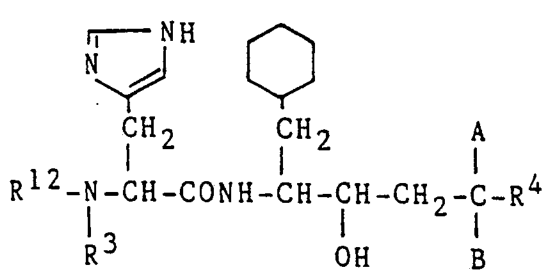

wherein $R^{12}$, $R^3$, A, B and $R^4$ are as defined in claim 1.

(7) An antihypertensive composition containing as an effective ingredient a compound as claimed in claim 1 in an amount effective for treatment and pharmaceutical carrier.

European Patent Office

# EUROPEAN SEARCH REPORT

Application Number

EP 90 10 8163

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 202 577 (G.D. SEARLE & CO.)<br>* Entire document *<br>--- | 1-7 | C 07 K 5/06<br>C 07 K 5/02<br>C 07 K 5/08<br>C 07 D 233/64<br>A 61 K 37/64<br>A 61 K 31/415 |
| Y | BIOCHEMICAL AND BIOPHYSICAL RESEARCH COMMUNICATIONS, vol. 146, no. 3, 1987, pages 959-963, Academic Press, Inc.; G.J. HANSON et al.: "Enhanced potency dipeptide glycol renin inhibitors: Studies in vitro and in the conscious rhesus"<br>* Entire article *<br>--- | 1-7 | |
| A | WO-A-8 805 050 (ABBOTT LAB.)<br>* Entire document *<br>--- | 1-7 | |
| A | EP-A-0 296 581 (E.R. SQUIBB)<br>* Page 4, line 10 - page 9, line 35; examples 1-3; claims 1,10 *<br>----- | 1-7 | |
| | | | **TECHNICAL FIELDS SEARCHED (Int. Cl.5)** |
| | | | C 07 K<br>A 61 K |

The present search report has been drawn up for all claims

| Place of search | Date of completion of the search | Examiner |
|---|---|---|
| THE HAGUE | 30-07-1990 | GROENENDIJK M.S.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document